# EUROPEAN PATENT APPLICATION

(11) **EP 4 506 449 A1**
(43) Date of publication of application: **12.02.2025**
(21) Application number: 23781446.2
(22) Date of filing: 31.03.2023
(51) Int. Cl.: C12N 5/0783, A61K 35/17, A61P 31/12, A61P 35/00, A61P 25/28

(54) **NATURAL KILLER CELLS PRODUCED FROM INDUCED PLURIPOTENT STEM CELLS, METHOD FOR PRODUCING SAME, AND USE THEREOF**

(30) Priority: 01.04.2022 KR 20220040974
(71) Applicant: Therabest Co., Ltd., Seoul 06656 (KR)
(72) Inventor: KIM, Shin-Il, Seoul 06241 (KR); HWANG, Do Won, Suwon-si Gyeonggi-do 16700 (KR); KI, Young Wook, Yongin-si Gyeonggi-do 16853 (KR)
(74) Representative: HGF
(86) International application number: PCT/KR2023/004404
(87) International publication number: WO 2023/191597

(57) **Abstract**

The present invention relates to: natural killer cells produced and mass-proliferated from induced pluripotent stem cells; and a pharmaceutical composition comprising the same as an active ingredient. The natural killer cells according to the present invention exhibited oncolytic activity at cellular and animal levels. In addition, the expression of genes and cytokines involved in tumor death and immune cell activity was increased compared to natural killer cells isolated from blood and activated. It was also confirmed that a method for inducing differentiation of natural killer cells derived from induced pluripotent stem cells, according to the present invention, is a method for producing a large number of cells within a short period of time by using a simple method compared to conventional induction methods using induced pluripotent stem cells. Therefore, it is considered that a method for differentiating natural killer cells derived from induced pluripotent stem cells, and natural killer cells produced by the method, according to the present invention, can be used in the treatment of various diseases, including cancer.

## Description

### Technical Field

The present invention relates to natural killer cells produced from induced pluripotent stem cells, a method for producing the same and a use thereof.

### Background Art

Natural killer cells (NK cells) are one of the cytotoxic lymphocytes important for innate immunity. Natural killer cells have cytotoxicity that can directly kill not only virus-infected cells but also cancer cells. In general, immune cells detect major histocompatibility complexes (MHC) presented on the surface of infected cells, induce cytokine release, and induce the death of infected cells by cell lysis or cell apoptosis.

On the other hand, natural killer cells can recognize and eliminate abnormal cells such as cancer cells without antibodies and MHC, so they can induce an immediate immune response. Natural killer cells are known to attack cancer cells, prevent the occurrence, proliferation, and metastasis of cancer cells, and effectively control cancer stem cells that play an important role in the recurrence of cancer. Accordingly, various studies are being attempted to treat cancer using natural killer cells. Therefore, the technology for mass production of allogeneic and autologous human natural killer cells has also emerged as a key technology.

The main source of human natural killer cell resources is the method of isolating and proliferating natural killer cells from blood. However, natural killer cells account for only about 10% of lymphocytes in blood. In order to apply natural killer cells to various fields including anticancer treapy, it is necessary to mass proliferate natural killer cells *in vitro* with high yield, but it is not yet at a level that can be applied clinically (Korean Patent Application Publication No. 10-2018-0117829). Recently, the technology of producing natural killer cells through differentiation-induced culture from stem cells such as human hematopoietic stem cells, embryonic stem cells, and induced pluripotent stem cells with excellent differentiation potential has also been actively studied.

### Detailed Description of Invention

### Technical Problem

The present inventors have tried to develop a method for producing human natural killer cells with high efficiency. As a result, natural killer cells were produced from isolated induced pluripotent stem cells (iPSCs), and the produced natural killer cells were mass proliferated and their characteristics were analyzed. As a result, it was confirmed that the natural killer cells exhibited properties that were differentiated from existing blood-derived natural killer cells. Based on the above, the present inventors completed the present invention.

### Solution to Problem

In order to achieve the above object, in one aspect of the present invention, there is provided a natural killer cell expressing any one protein selected from the group consisting of CD25, CD9, CD80, and a combination thereof; a pharmaceutical composition comprising the same as an active ingredient; and a method for producing the natural killer cells.

In another aspect of the present invention, there is provided a use of the natural killer cell for the prevention or treatment of a disease.

In another aspect of the present invention, there is provided a method for preventing or treating a disease, comprising: administering the natural killer cell.

### Effects of Invention

The natural killer cells according to the present invention exhibited oncolytic activity at cellular and animal levels. In addition, the expression of genes and cytokines involved in tumor death and immune cell activity was increased compared to natural killer cells isolated from blood and activated. In addition, the method for differentiating natural killer cells from iPSCs according to the present invention can produce a large number of cells within a short period of time compared to conventional methods for differentiating natural killer cells from iPSCs. In addition, it was confirmed that the activity of the natural killer cells produced in this way was excellent. Therefore, the method for differentiating natural killer cells from iPSCs of the present invention and the natural killer cells produced by the method can be used in the treatment of various diseases, including cancer.

### Brief Description of Drawings

Figure 1 is a schematic diagram of differentiating natural killer cells in the presence of autologous feeder cells from induced pluripotent stem cells (iPSCs).
Figure 2 is a diagram summarizing a method for differentiating natural killer cells in the presence of autologous feeder cells from induced pluripotent stem cells (iPSCs).
Figure 3 is a diagram showing the results obtained by confirming the morphological changes at each stage of differentiation from iPSCs into NK cells using an optical microscope.
Figure 4 is a diagram showing the results obtained by photographing cell culture vessels of the group in which autologous feeder cells were maintained and the group in which autologous feeder cells were removed after the hematopoietic stem cell differentiation stage during differentiation from iPSCs into NK cells.
Figure 5 is a diagram showing the results obtained by observing the differences between the group in which autologous feeder cells were maintained and the group in which autologous feeder cells were removed after the hematopoietic stem cell differentiation stage during differentiation from iPSCs into NK cells using an optical microscope.
Figure 6 is a graph showing the results obtained by analyzing the number of natural killer cells (suspension cells) produced at each differentiation stage in the group in which autologous feeder cells were maintained and the group in which autologous feeder cells were removed after the hematopoietic stem cell differentiation stage during differentiation from iPSCs into NK cells.
Figure 7 is a diagram showing the results obtained by analyzing the major natural killer cell markers of cells differentiating into natural killer cells by flow cytometry in the group in which autologous feeder cells were maintained and the group in which autologous feeder cells were removed after the hematopoietic stem cell differentiation stage during differentiation from iPSCs into NK cells.
Figures 8a to 8c are diagrams and graphs showing the results obtained by confirming the number of iPSC colonies (8a) adhered according to condition 1 to condition 6, respectively, the total number of cells/adhered vessel area (cm²) (8b) for each condition, and the total number of iPSC colonies per T-150 flask (8c) for each condition, upon differentiation from iPSCs into natural killer cells.
Figure 9 is a diagram showing the results obtained by confirming the morphological changes of each cell differentiated by stage according to condition 1 to condition 6 upon differentiation from iPSCs into NK cells using an optical microscope.
Figure 10 is a graph showing the results obtained by confirming the amount of natural killer cells (suspension cells) differentiated under each condition.
Figure 11 is a graph showing the results obtained by analyzing the expression rate of cell surface proteins (markers) of the differentiated natural killer cells under condition 1 to condition 6 upon differentiation from iPSCs into NK cells.
Figure 12 is a diagram and graph showing the results obtained by analyzing the number of induced pluripotent stem cell colonies adhered according to condition 1 to condition 6, respectively, upon differentiation from iPSCs into NK cells.
Figure 13 is a diagram showing the results obtained by observing the morphological changes of differentiated cells by stage for each condition (A to D) upon differentiation from iPSCs into NK cells using an optical microscope.
Figure 14 is a graph showing the results obtained by confirming the expression rate of cell surface marker proteins (markers) of the differentiated natural killer cells under each condition (A to D) upon differentiation from iPSCs into NK cells by flow cytometry.
Figure 15 shows the results of flow cytometry analysis of cells at the mesodermal cell stage (differentiation day 4) during differentiation from an induced pluripotent stem cell colony (iPSC colony) into NK cells.
Figure 16 shows the results of flow cytometry analysis of cells that were differentiated through the mesodermal cell stage into hematopoietic stem cells (differentiation day 12) during differentiation from an iPSC colony into NK cells.
Figures 17a and 17b show the results of flow cytometry analysis of cells that were differentiated from an iPSC colony through the mesodermal and hematopoietic stem cell stage into NK cells (differentiation day 26).
Figures 18a and 18b show the results of flow cytometry analysis of cells that were differentiated from an iPSC colony through the mesodermal and hematopoietic stem cell stage into NK cells (differentiation day 47).
Figure 19 is a diagram showing a method of differentiation from iPSCs into natural killer cells using Stempro34 medium.
Figure 20 shows the results of flow cytometry analysis of cells at the mesodermal cell stage (differentiation day 4) during differentiation from an iPSC colony into NK cells according to the method of Figure 19.
Figure 21 shows the results of flow cytometry analysis of cells that were differentiated through the mesodermal cell stage into hematopoietic stem cells (differentiation day 12) during differentiation from an iPSC colony into NK cells according to the method of Figure 19.
Figure 22 shows the results of flow cytometry analysis of cells that were differentiated from an iPSC colony through the mesodermal and hematopoietic stem cell stage into NK cells (differentiation day 26) according to the method of Figure 19.
Figure 23 shows the results of flow cytometry analysis of cells that were differentiated from an iPSC colony through the mesodermal and hematopoietic stem cell stage into NK cells (differentiation day 30) according to the method of Figure 19.
Figure 24 shows the results of flow cytometry analysis of cells that were differentiated from an iPSC colony through the mesodermal and hematopoietic stem cell stage into NK cells (differentiation day 34) according to the method of Figure 19.
Figure 25 shows the results of flow cytometry analysis of cells that were differentiated from an iPSC colony through the mesodermal and hematopoietic stem cell stage into NK cells (differentiation day 42) according to the method of Figure 19.
Figure 26 is a graph showing the results obtained by confirming the cytotoxicity of iPSC-derived NK cells (EiNK cells) through co-culture with chronic myeloid leukemia cell line K562 cells.

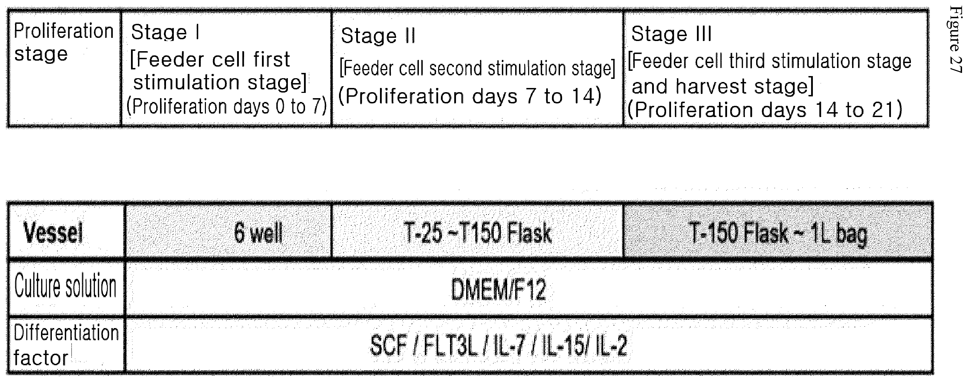

Figure 27 is a schematic diagram of the proliferation method of iPSC-derived NK cells (EiNK cells) based on feeder cells.
Figure 28 is a diagram showing the results obtained by confirming the morphological changes at each time point during the mass proliferation process of iPSC-derived NK cells (EiNK cells) using an optical microscope.
Figure 29 is a graph showing the results obtained by measuring the number of cells at each time point during the mass proliferation process of iPSC-derived natural killer cells (EiNK cells) as an increase rate and cell number.
Figures 30a and 30b are diagrams and graphs showing the results obtained by confirming the NK cell activity after mass proliferation of iPSC-derived NK cells (EiNK cells) for 20 days by flow cytometry.
Figure 31 is a diagram showing a graph and table comparing the results obtained by confirming the cytotoxicity of NK cells against tumor cells after mass proliferation of iPSC-derived NK cells (EiNK cells) for 21 days or 28 days.
Figure 32 is a diagram showing the results obtained by confirming the expression of marker proteins (TRA-1-60, SSEA4) of induced pluripotent stem cells after mass proliferation of iPSC-derived NK cells (EiNK cells) for 21 days by flow cytometry.
Figure 33 is a graph showing the results obtained by confirming the cell proliferation rate during long-term culture of iPSC-derived NK cells (EiNK cells).
Figures 34a and 34b are diagrams and graphs showing the results obtained by confirming the expression of marker proteins (Figure 35a, cultured for 63 days) of NK cells (CD3, CD45) or induced pluripotent stem cells (TRA-1-60, SSEA4); and the tumor cell-killing ability (Figure 35b) after culturing iPSC-derived NK cells (EiNK cells) for different periods (20 days, 56 days, and 63 days).
Figures 35a and 35b are diagrams and graphs showing the results obtained by confirming the expression of cell surface proteins in iPSC-derived NK cells (EiNK cells, cultured for 21 days) and blood-derived NK cells (PBNK cells, cultured for 22 days) by flow cytometry.
Figure 36 is a graph showing the results obtained by confirming the expression of genes related to tumor killing and activation of NK cells in iPSC-derived NK cells (EiNK cells, cultured for 28 days) and blood-derived NK cells (PBNK cells, cultured for 22 days).
Figures 37a and 37b are graphs showing cytokines selected to have a concentration 2 times or more higher (37a) or 1.3 to 1.9 times or more higher (37b) in the culture solution of EiNK cells by measuring cytokines in the culture solution of NK cells in iPSC-derived NK cells (EiNK cells, cultured for 21 days) and blood-derived NK cells (PBNK cells, cultured for 21 days).
Figure 38 is a diagram showing the results obtained by confirming the expression of the natural killer cell inhibitory receptor in iPSC-derived NK cells (EiNK cells, cultured for 21 days) and blood-derived NK cells (PBNK cells, cultured for 21 days) by flow cytometry.
Figure 39 is a graph showing the results obtained by confirming the tumor cell-killing ability of iPSC-derived NK cells (EiNK cells, cultured for 21 days or 28 days) and blood-derived NK cells (PBNK cells, cultured for 16 days) by flow cytometry.
Figures 40a to 40e are diagrams (40a to 40d) showing the results obtained by confirming the activity of luciferase in mice for different periods after administering an ovarian cancer (OVCAR3) cell line expressing luciferase to NSG mice and then treating with iPSC-derived NK cells (EiNK cells, cultured for 21 days), and a graph (40e) showing the results obtained by quantifying the above activity.
Figure 41 is a graph showing the results obtained by confirming the survival rate of mice after administering an ovarian cancer (OVCAR3) cell line expressing luciferase to NSG mice and then treating with iPSC-derived NK cells (EiNK cells, cultured for 21 days).
Figures 42a to 42c are diagrams showing the results obtained by confirming the yield of cells obtained after CD34 sorting (Figure 42a), cell death (Figure 42a), the expression of NK cell markers (/activation markers) (Figure 42b), and the cell proliferation rate (Figure 42c), and Figure 42d is a diagram showing the results obtained by confirming the expression of hematopoietic stem cell marker proteins (CD34/CD45) without a isolating process with CD34 markers during EiNK cell differentiation as one embodiment of the EiNK cell differentiation method of the present invention by flow cytometry.
Figure 43 is a graph showing the results obtained by measuring the size of embryoid bodies (EB) formed for each culture period during the third culture of iPSCs by a conventional EiNK cell production method.
Figure 44 is a graph showing the results obtained by measuring the survival rate of isolated cells by batch after isolating cells from embryoid bodies (EBs) formed through the third culture of iPSCs by a conventional EiNK cell production method.
Figure 45 is a graph showing the results obtained by comparing the period required for EiNK cell production according to a conventional EiNK cell production method and the EiNK cell production method according to one embodiment of the present invention.
Figure 46 is a diagram showing the results obtained by confirming the expression of marker proteins of natural killer cells (CD56, CD45, NKp30, NKp44) and induced pluripotent stem cells (TRA-1-60, SSEA4) in EiNK cells produced according to the EiNK cell production method according to one embodiment of the present invention by flow cytometry.

### Best Mode for Carrying out the Invention

### Natural killer cell

In one aspect of the present invention, there is provided a natural killer cell expressing any one protein selected from the group consisting of CD25, CD9, CD80, and a combination thereof. The natural killer cell may additionally express any one protein selected from the group consisting of CD1d, CD30, CD87, CDw327, CD33, CD49a, CD49c, CD49e, CD49f, CD66a, CD66c, CD66d, CD66e, CD71, CD83, CDw93, CD106, CD108, CD114, CD123, CD124, CD146, CD150, CD164, CD196, CD210, and a combination thereof.

As used herein, the term "natural killer cell (NK cell)" is a cytotoxic lymphocyte that constitutes a major component of the innate immune system, and is defined as a large granular lymphocyte (LGL), and constitutes a third cell differentiated from B and T lymphocytes that produce from common lymphoid progenitor (CLP). It is a lymphocyte cell that plays a part in the immune response, and exists in about 10-15% of normal human blood, and has a high killing ability when reacting with non-self. Natural killer cells may non-specifically and immediately react to cells infected with various viruses, bacterial invasion, or the generation of abnormal cells to remove foreign substances. The natural killer cells are activated in response to interferon or macrophage-derived cytokines, and the natural killer cells comprise two types of extracellular receptors that control the cytotoxic activity of cells, represented by an "activation receptor" and an "inhibitory receptor."

As used herein, the term "CD25" is an alpha subunit of the interleukin-2 receptor, and is known to regulate the activity of regulatory T cells (Tregs) and inhibit the activity and proliferation of activated T cells, thereby regulating the immune response.

As used herein, the term "CD80 (cluster of differentiation 80)" is also known as B7-1. It is a cell surface protein belonging to the B7 family of the immunoglobulin superfamily. CD80 consists of an Ig-like extracellular domain (one variable and one constant), a transmembrane region, and a cytoplasmic domain. The costimulatory ligand CD80 is expressed on antigen presenting cells (APCs) such as monocytes, dendritic cells, and activated B cells, and on T cells. CD80 binds to CD28 or CTLA-4 (cytotoxic T lymphocyte antigen-4) to regulate cell proliferation and IL-2 production. When CD80 binds to CD28, it activates T cells to promote an immune response, whereas when it binds to the immune checkpoint protein CTLA-4, it reduces the activity of T cells to inhibit an immune response.

As used herein, the term "CD9" is a protein belonging to the transmembrane protein 4 superfamily, also known as the tetraspanin family, which is involved in sperm-egg fusion associated with integrins, platelet activation and aggregation, cell adhesion, and cell migration.

As used herein, the term "CD1d" is an antigen presenting protein that binds to self and non-self glycolipids and presents them to the T cell receptor of natural killer T cells (NKT cells).

As used herein, the term "CD30" is also known as TNFRSF8 and is a protein expressed in activated T cells and B cells. It is known to induce apoptosis by binding to TRAF2 or TRAF5 and activating the NF-xB signaling pathway. In addition, it inhibits autoimmunity by inhibiting the proliferation and activity of self-activated cytotoxic T cells.

As used herein, the term "CD87" is also known as urokinase receptor or uPAR (urokinase plasminogen activator surface receptor), and plays a role in promoting plasmin formation and localization. Plasmin is a serine protease that acts to dissolve fibrin clots, and cell surface-bound plasmin is known to promote ECM degradation by activating MMPs that may degrade collagen and other proteins in the blood vessel wall, and is involved in the migration of neutrophils, monocytes, and macrophages.

As used herein, the term "CDw327" is known as SIGLEC6 (sialic acid-binding Ig-like lectin 6) or CD33LSIGLEC6, and is a cell adhesion protein involved in sialic acid-dependent cell binding.

As used herein, the term "CD33" is also known as siglec-3 and is a receptor expressed on cells of the myeloid lineage. CD33 binds to any molecule containing a sialic acid residue, such as a glycoprotein or glycolipid, and upon activation, induces a signaling pathway that inhibits phagocytosis.

As used herein, the term "CD49a" is integrin alpha-1 (integrin α-1) and is a subunit of the integrin α1β1 receptor.

As used herein, the term "CD49c" is integrin alpha-3 and is a subunit of the integrin α3β1 receptor involved in neural migration and cortical formation.

As used herein, the term "CD49e" is integrin alpha-5 and is a subunit of the integrin α5β1 receptor. α5 is cleaved posttranslationally in the extracellular domain and binds to β1 to form the fibronectin receptor.

As used herein, the term "CD49f" is integrin alpha-6, which binds to integrin beta 4 (β4), known as TSP180, or to β1 of integrin VLA-6, thereby mediating cell adhesion and cell signaling.

As used herein, the term "CD66a" is also known as CEACAM1 (carcinoembryonic antigen-related cell adhesion molecule 1) and belongs to the CEA (carcinoembryonic antigen) superfamily. The protein acts as both an inhibitory receptor and an activation receptor. For example, it acts as an inhibitory receptor in immune responses and insulin action, and has a function of promoting angiogenesis. CEACAM1 is known to suppress immune responses by inhibiting T cell proliferation and cytokine production, inhibiting the activity of natural killer cells, and inhibiting the secretion of IL-1β from neutrophils, and the like.

As used herein, the term "CD66c" is also known as CEACAM6 (carcinoembryonic antigen-related cell adhesion molecule 6) and belongs to the CEA (carcinoembryonic antigen) superfamily. It is known to be involved in cell adhesion and tumor formation/progression.

As used herein, the term "CD66d" is also known as CEACAM3 (carcinoembryonic antigen-related cell adhesion molecule 3) and belongs to the CEA (carcinoembryonic antigen) superfamily. It is expressed on granulocytes and is known to regulate phagocytosis against pathogens in innate immunity.

As used herein, the term "CD66e" is also known as CEACAM5 (carcinoembryonic antigen-related cell adhesion molecule 5) and belongs to the CEA (carcinoembryonic antigen) superfamily. It regulates cell adhesion, cell signaling, and tumor formation (/progression).

As used herein, the term "CD71" is also known as TFR1 (transferrin receptor protein 1) and is a protein involved in the influx of ions into cells via transferrin. It is known to be expressed only in normal hematopoietic bone marrow and spleen erythroid precursors.

As used herein, the term "CD83" is used as a marker protein for mature dendritic cells. It is known to stabilize the expression of MHC II, costimulatory molecules, and CD28 on the dendritic cell membrane by inhibiting the activity of E3 ubiquitin ligase.

As used herein, the term "CDw93" is also known as C1q binding protein (C1qRp). CDw93 is expressed in monocytes (macrophages) and neutrophils, but is not expressed in T cells and B cells. CDw93 binds to C1q, the recognition subunit of the first component (C1) of the complement pathway, and promotes C1q-mediated phagocytosis. CDw93 is known to be highly expressed in stem cells (precursors) that will be differentiated into hematopoietic cells and liver cells.

As used herein, the term "CD106" is also referred to as VCAM-1 (vascular cell adhesion molecule 1), and mediates the adhesion of lymphocytes, monocytes, eosinophils, and basophils to vascular endothelium. In addition, it mediates the interaction between leukocytes and endothelial cells through signaling. CD106 acts as an endothelial ligand for VLA-4 (very late antigen-4 or integrin α4β1).

As used herein, the term "CD108" is SEMA7A (semaphorin 7A) and is also known as JMH (John-Milton-Hagen) blood group antigen. It is expressed on activated lymphocytes and erythrocytes, and is also found in the esophagus, heart, brain, spleen, testis, lung, ovary, and uterus. It plays an important role in integrin-mediated signaling, and regulates cell migration and immune responses.

As used herein, the term "CD114" is known as GCSFR (granulocyte colony-stimulating factor receptor), and is a cell surface receptor for GCSF. It is a type 1 cytokine receptor, belongs to the hematopoietin receptor superfamily, and is expressed on progenitor cells present in the bone marrow, mediating cell proliferation of progenitor cells and differentiation into mature neutrophils and macrophages.

As used herein, the term "CD123" is an interleukin-3 receptor, is expressed on basophils and dendritic cells, and mediates an immune response by IL-3.

As used herein, the term "CD124" is an interleukin-4 receptor and belongs to the type 1 cytokine receptor. It regulates an immune response by binding to IL-4 and IL-13 and mediating IgE antibody production, Th2 cell differentiation promotion, and the like.

As used herein, the term "CD146" is known as MCAM (melanoma cell adhesion molecule) and is used as a marker protein of the endothelial cell lineage. It is a receptor for laminin alpha 4 (laminin α4) and is expressed in large quantities in cells that constitute the blood vessel wall, such as vascular endothelial cells, smooth muscle cells, and perivascular cells. It is also known to be expressed in some subgroups of T cells and B cells.

As used herein, the term "CD150" is known as SLAMF1 (signaling lymphocytic activation molecule 1), which mediates the interactions between homotypic or heterotypic cells, thereby regulating various immune cell activities and differentiation.

As used herein, the term "CD164" is also known as endolyn or sialomucin core protein 24. The sialomucin is a protein involved in both cell adhesion inhibition and cell adhesion, and the CD164 is a protein involved in cell adhesion.

As used herein, the term "CD196" is also known as chemokine receptor 6 (CCR6) and belongs to the G protein-coupled receptor superfamily. It is expressed in B cells, T cells (Th1, Th2, Th17, Treg), natural killer T cells (NKT cells), immature dendritic cells, and neutrophils. It regulates an immune response by binding to CCL20 (MIP3α) and mediating the maturation and differentiation of B cells, migration of dendritic cells and T cells, and the like.

As used herein, the term "CD210" is a receptor for interleukin-10 and belongs to the type 2 cytokine receptor. It is composed of α and β subunits, and the α subunit is expressed in hematopoietic cells such as T cells, B cells, natural killer cells, mast cells, and dendritic cells. On the other hand, the expression of the β subunit is mostly regulated by ubiquitin. The α subunit is the ligand binding site of the IL-10 receptor and is present only in the IL-10 receptor, but the β subunit also binds to subunits of other type 2 cytokine receptors to form a receptor. IL-10 receptor is known to mediate gastrointestinal immune response mainly in the mucosal layer.

In the present invention, the expression of the above CD1d, CD30, CD87, CDw327, CD33, CD49a, CD49c, CD49e, CD49f, CD66a, CD66c, CD66d, CD66e, CD71, CD83, CDw93, CD106, CD108, CD114, CD123, CD124, CD146, CD150, CD164, CD196 and CD210 proteins may be increased compared to a natural killer cell isolated from blood. Specifically, the expression of the above CD25, CD9, CD80, CD1d, CD30, CD87, CDw327, CD33, CD49a, CD49c, CD49e, CD49f, CD66a, CD66c, CD66d, CD66e, CD71, CD83, CDw93, CD106, CD108, CD114, CD123, CD124, CD146, CD150, CD164, CD196, CD210 proteins may be increased by about at least two times compared to a natural killer cell isolated from blood.

In this case, the natural killer cell isolated from blood may be derived from a mammal such as a rat, a mouse, a livestock, and a human. The natural killer cell isolated from blood may be a natural killer cell isolated from blood without additional modification or a natural killer cell that has been induced to be activated. Preferably, it may be a natural killer cells that have been induced to be activated.

As used herein, the term "activated natural killer cell" may refer to a natural killer cell with increased cytotoxicity or enhanced natural killer cell's inherent immune-regulating ability compared to a natural killer cell isolated from blood without additional modification. The activated natural killer cell may have an overexpression of a receptor that may target a cell or tissue to be treated.

A method for activating natural killer cells may be culturing natural killer cells isolated from blood under specific conditions, genetically modifying them, or treating them with a substance that increases the activity of natural killer cells. In this case, the natural killer cell culture may use a conventional animal cell culture medium known in the art without limitation, and for example, CellGro medium (Cellgenix), AIM-V medium, XVIVO 20, RPMI1640, and the like may be used.

As used herein, the term "substance that increases the activity of natural killer cells" refers to any substance that may increase the activity of natural killer cells, for example, the cytotoxicity of natural killer cells, or produce, increase, or proliferate activated natural killer cells. The substance that increases the activity of natural killer cells may include an immune cytokine, but is not limited thereto as long as it is a substance that may increase the activity of natural killer cells.

As used herein, the term "cytokine" refers to a signal substance that controls the body's defense system and stimulates the body, and may mean a physiological activity regulating protein. The cytokine regulates various biological activities such as cell activation, differentiation, cell migration, aging, and apoptosis induction in various cells through autocrine and paracrine actions.

The cytokine may be an interferon (IFN) family, an interleukin family, a tumor necrosis factor (TNF) family, or a combination thereof.

Interferon may be any one selected from the group consisting of type 1 interferon (e.g., interferon-α, interferon-β, interferon-x, interferon-ω), type 2 interferon (e.g., interferon-γ), type 3 interferon, and a combination thereof. Interleukin may be any one selected from the group consisting of IL-2, IL-5, IL-8, IL-12, IL-15, IL-18, IL-21, and a combination thereof. In this case, TNF may include TNF-α, TNF-β, or TNF-γ, etc. The immune cytokine may include a cytokine-antibody complex. For example, it may include an interleukin-anti-interleukin antibody complex.

Specifically, the activated natural killer cell of the present invention may be obtained by culturing blood-derived natural killer cells for 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, or 22 days.

The natural killer cell of the present invention may express any one natural killer cell marker protein selected from the group consisting of CD56, CD45, CD16, NKp30, NKp44, NKG2D, DNAM1, and a combination thereof in addition to the above protein, and may not express CD3, a marker protein of T cells, or may express it at a lower level compared to T cells. Here, the natural killer cell activation is the same as described above, and whether the natural killer cell is activated may be evaluated by detecting one or more functionally related markers, such as CD56, CD45, CD16, NKp30, NKp44, NKG2D, DNAM1, and the like.

In addition, the natural killer cell according to the present invention may have increased expression of genes related to the tumor killing ability of a natural killer cell and natural killer cell activation compared to a natural killer cell isolated from blood. Specifically, the natural killer cell according to the present invention may be characterized by additionally increased expression of any one gene selected from the group consisting of GZMB, CD226, RASGRP1, CD107a, CCR9, CD40LG, CD300c, and a combination thereof compared to a natural killer cell isolated from blood. In this case, the natural killer cell obtained from blood is the same as described above.

As used herein, the term "GZMB (granzyme B)" is one of the serine proteases expressed in cytotoxic T cells and natural killer cells. The T cells and natural killer cells induce apoptosis of target cells through granzyme B.

As used herein, the term "CD226" is an immunoglobulin-like transmembrane glycoprotein known as PTA1 (platelet and T cell activation antigen 1) or DNAM-1 (DNAX accessory molecule-1). The CD226 is expressed on natural killer cells, natural killer T cells, B cells, dendritic cells, hematopoietic stem cells, platelets, monocytes, and T cells. It binds to CD112 or CD155 and is involved in cell adhesion. It mediates signal transduction of lymphocytes and regulates lymphokine secretion and cytotoxic action of natural killer cells and cytotoxic T cells. In addition, it regulates platelet activation and aggregation, and trans-endothelial migration of natural killer cells.

As used herein, the term "RASGRP1 (RAS guanyl-releasing protein 1)" is a protein belonging to the Ras superfamily comprising a guanine nucleotide exchange factor (GEF) domain, which regulates development of T cells and B cells, homeostasis, and differentiation.

As used herein, the term "CD107a" is known as LAMP-1 (lysosomal-associated membrane protein 1 or lysosome-associated membrane glycoprotein 1), LGP120, and LAMPA, and is a degranulation marker of cytotoxic T cells and natural killer cells. It mediates the migration of cytotoxic granules to the cell surface and the adhesion of perforin to the cytotoxic granules, thereby regulating the cytolytic action of target cells. In addition, it acts to protect natural killer cells from damage caused by the degranulation process.

As used herein, the term "CCR9" is a receptor for CCL25 and is also known as CDw199. The CCR9 is known to be involved in cancer proliferation and inflammatory diseases by binding to CCL25. The interaction of CCR9/CCL25 promotes the proliferation and metastasis of tumor cells and inhibits cell death, thereby promoting the progression of cancer. In addition, it is known to promote the onset of inflammatory diseases (inflammatory bowel disease, cardiovascular disease, rheumatoid arthritis, asthma, and the like).

As used herein, the term "CD40LG (or CD40L)" is also known as CD154 and is a protein expressed on T cells. CD40LG binds to CD40 expressed on antigen presenting cells and is a costimulatory molecule that activates immune cells.

As used herein, the term "CD300c" is also known as CMRF-35 or CMRF-35A. The B7 family is a member of the Ig superfamily and is known to be expressed on the surface of antigen presenting cells such as B cells, macrophages, and dendritic cells. Expression or inhibition of the activity of CD300c protein induces activation of T cells and/or promotion of differentiation of M1 macrophages.

The natural killer cell according to the present invention may be characterized by increased secretion of any one protein selected from the group consisting of FLT3L, MIP 1 alpha/beta, PF4, IGFBP2, IGFBP3, MMP9, and a combination thereof compared to a natural killer cell isolated from blood. In this case, the protein is a secretion protein secreted outside the cell, and the secretion thereof may be increased by at least two times in the natural killer cell according to the present invention compared to a natural killer cell isolated from blood. In this case, the natural killer cell obtained from blood is the same as described above.

As used herein, the term "FLT3L (FMS-like tyrosine kinase 3 ligand)" is a protein encoded by the FLT3LG gene in humans. It is structurally homologous to stem cell factor (SCF) and colony stimulating factor 1 (CSF-1), and promotes the proliferation and differentiation of hematopoietic stem cells together with various regulatory factors.

As used herein, the term "MIP1 (macrophage inflammatory protein 1)" is a c-c motif chemokine, which exists in two forms, MIP-1α and MIP-1β, in humans. The MIP-1α and MIP-1β are also known as CCL3 and CCL4, respectively, and are secreted from activated macrophages and monocytes. CCL3 acts as a ligand for CCR1, and CCL4 acts as a ligand for CCR1 and CCR5, thereby activating monocytes, T cells, dendritic cells, NK cells, and platelets, etc.

As used herein, the term "PF4 (platelet factor 4)" is also known as CXC4 (chemokine CXC ligand 4) and is a ligand for CXCR3-B. PF4 is released from alpha granules of activated platelets during platelet aggregation and promotes blood clotting.

As used herein, the term "IGFBP2 (insulin-like growth factor-binding protein 2)" is a protein that binds to the IGF protein with high affinity. It inhibits the binding of IGF to IGF receptor (IGFR) through binding of IGFBP2 and IGF, thereby inhibiting cell proliferation and growth by IGF/IGFR interaction.

As used herein, the term "IGFBP3" is a protein that binds to IGF-1 or IGF-2 with high affinity. IGFBP3 is known to bind to IGF in the blood and play a role in prolonging its transport and half-life.

As used herein, the term "MMP9 (matrix metallopeptidase 9)" is also known as type 4 collagenase or gelatinase B (GELB) with 92 kDa. MMP9 is induced to be expressed by inflammatory stimuli and promotes cell migration by degrading the matrix.

The natural killer cell of the present invention may be a natural killer cell that does not express any one protein selected from the group consisting of CD205, CD158b, CD243, γδTCR, NKB1, D158a, CDw328, TIM3, TIGIT, and a combination thereof, or expresses it at a lower level compared to a natural killer cell obtained from blood. Specifically, the natural killer cell may be a natural killer cell that expresses any one protein selected from the group consisting of CD205, CD158b, CD243, γδTCR, NKB1, CD158a, CDw328, TIM3, TIGIT, and a combination thereof at a level at least two times lower than a natural killer cell obtained from blood. In this case, the natural killer cell obtained from blood is the same as described above.

As used herein, the term "CD205" is also known as lymphocyte antigen 75 or DEC-205 and is a receptor involved in the endocytosis of antigens captured outside the cell. It is known to reduce the proliferation of B cells.

As used herein, the term "CD158b" is known as KIR2DL2/DL3 (killer cell immunoglobulin-like receptor 2DL2/DL3) or two Ig domains and long cytoplasmic tail 2 and is a receptor for HLA-C (HLA-Cw1, HLA-Cw3, HLA-Cw7) present in natural killer cells. The protein is known to inhibit the activity of natural killer cells and inhibit the lysis of cancer cells by natural killer cells.

As used herein, the term "CD243 (cluster of differentiation 243)" is also known as P-gp (permeability glycoprotein, P-glycoprotein 1, Pgp), MDR1 (multidrug resistance protein 1), or ABCB1 (ATP-binding cassette sub-family B member 1) and is a cell membrane protein that exports foreign substances (toxins or drugs) from inside the cell to outside the cell in an ATP-dependent manner. It is widely distributed in the intestinal epithelium and is also known to be highly expressed in some cancer cells and cause anticancer agent resistance.

As used herein, the term "γδTCR" is a T cell antigen receptor (TCR) expressed on γδT cells. While the antigen receptor (αβ TCR) of αβ T cells recognizes peptide antigens presented by major histocompatibility complex molecules (pMHC) on antigen presenting cells (APCs), γδ TCRs are known to partially recognize stress-induced self-antigens, lipids, or pyrophosphates that are secreted in a somewhat restricted manner by some microorganisms or are overproduced by tumor cells.

As used herein, the term "NKB1" is also known as KIR3DL1 (killer cell immunoglobulin-like receptor 3DL1), NKAT3, or MHC class I NK cell receptor. It is expressed on natural killer cells and acts as a receptor for HLA-Bw4. It is known to inhibit the activity of natural killer cells and inhibit the lysis of tumor cells by natural killer cells.

As used herein, the term "CD158a" is also known as KIR2DS1 (killer cell immunoglobulin-like receptor 2DS1) and is a receptor for HLA-C (HLA-Cw6) present in natural killer cells. Unlike CD158b, which inhibits the activity of natural killer cells, this protein does not inhibit the activity of natural killer cells.

As used herein, the term "CDw328" is also known as SIGLEC7 (sialic acid binding Ig like lectin 7) and is a cell adhesion protein involved in sialic acid-dependent cell binding.

As used herein, the term "TIM-3 (T-cell immunoglobulin and mucin-domain containing-3)" is also known as HAVCR2 (hepatitis A virus cellular receptor 2) and is an immune checkpoint protein that inhibits immune responses. It is expressed in various tumor cells including T cells, monocytes, macrophages, dendritic cells, mast cells, and natural killer cells. The ligands of TIM-3 are known to be galectin-9, PtdSer (phosphatidylserine), HMGB1 (high mobility group box 1), and CEACAM1.

As used herein, the term "TIGIT (T-cell immunoreceptor with Ig and ITIM domains)" is also known as WUCAM and Vstm2 and is an immune receptor present on some T cells and natural killer cells. It binds to CD155 present on dendritic cells, macrophages, and the like, and inhibits the activity of T cells and natural killer cells in the body, thereby reducing the immune response.

In the present invention, the natural killer cells may be induced to differentiate from induced pluripotent stem cells. Specifically, the natural killer cells of the present invention may be produced a method for producing natural killer cells, comprising: inoculating induced pluripotent stem cells (iPSCs) into a cell culture vessel to 0.5 to 500 cells per 1 cm² of cell culture vessel area and performing adhesion culture to produce iPSC colonies; differentiating the iPSC colonies into mesodermal cells to produce mesodermal cells; differentiating the mesodermal cells into hematopoietic stem cells to produce hematopoietic stem cells; and differentiating the hematopoietic stem cells into natural killer cells to produce natural killer cells.

In addition, in the present invention, the natural killer cells may be produced by additionally performing a mass culture proliferation on the induced pluripotent stem cells.

Specifically, the natural killer cells of the present invention may be induced to differentiate from induced pluripotent stem cells through the following steps.

First, the method comprises inoculating induced pluripotent stem cells (iPSCs) as iPSCs or iPSC spheroids into a cell culture vessel so that 0.5 to 20 colonies are formed per 1 cm² of cell culture vessel area and performing adhesion culture to produce iPSC colonies.

As used herein, the term "stem cell" is a relatively undifferentiated cell that has not yet developed and refers to a cell that has the ability to differentiate into specific tissue cells under suitable conditions. The stem cell may self-renew and produce daughter cells that have the ability to maintain their own undifferentiated characteristics, and at the same time produce daughter cells that differentiate into specific types of cells. The marker proteins of stem cells include Lgr5, Lrp4, Msi-1, Sca-1, SCF, SSEA-4, Tra-1-60, Tra-1-81, and the like. The stem cells include embryonic stem cells, adult stem cells, and induced pluripotent stem cells.

As used herein, the term "induced pluripotent stem cell (iPSC)" refers to a cell generated by inducing a somatic cell that does not have pluripotency to be dedifferentiated into a cell that have pluripotency, such as an embryonic stem cell. The induced pluripotent stem cell may also be referred to as a dedifferentiated stem cell. The induced pluripotent stem cells may be generated using somatic cells from a fetus, newborn, child, or adult. The induced pluripotent stem cells may be derived from fibroblasts, keratinocytes, blood cells, or renal epidermal cells. The induced pluripotent stem cells may be produced by reprogramming somatic cells. The method of reprogramming somatic cells to produce induced pluripotent stem cells may be performed using a known method, such as the method described in Takahashi K, Yamanaka S (2006), Cell. 126:663-676.

As used herein, the term "differentiation" refers to a phenomenon in which the structure or function of a cell becomes specialized while the cell divides, proliferates, and the entire organism grows. In other words, it refers to a process in which cells, tissues, and the like of a living organism change to have a form and function suitable for performing their respective roles. In the present invention, the above term includes the process in which induced pluripotent stem cells transform into natural killer cells, and may also include the process in which progenitor cells express specific differentiation traits.

The number of cells or spheroids inoculated above may be 0.5 to 20 per 1 cm² of area, and specifically, may be 1, 2, 3, 4, 5, 6, 7, 8, 9, 10, 11, 12, 13, 14, 15, 16, 17, 18, 19, or 20 iPSCs or iPSC spheroids per 1 cm² of area.

In this case, the iPSC spheroid may comprise about 30 to about 600 iPSCs. In addition, the iPSC spheroid may comprise about 50 to about 300 iPSCs. In addition, the iPSC spheroid may comprise about 70 to about 200 iPSCs. In addition, the iPSC spheroid may comprise about 80, about 90, about 100, or about 120 iPSCs.

In addition, the number of inoculated cells may be calculated in another way. The number of cells present per ml of culture solution may be determined as the number of cells per cm² by using a method for calculating the number of inoculated cells.

Specifically, the number of inoculated induced pluripotent stem cells may be from about 0.5 to about 2,500 per 1 mL of culture solution. In addition, the number of cells may be about 1 to about 2,500, about 10 to about 2,500, about 20 to about 2,500, about 30 to about 2,500, about 40 to about 2,500, about 50 to about 2,500, about 60 to about 2,500, about 70 to about 2,500, about 80 to about 2,500, about 90 to about 2,500, about 100 to about 2,500, about 200 to about 2,500, about 300 to about 2,500, about 400 to about 2,500, about 500 to about 2,500, about 600 to about 2,500, about 700 to about 2,500, about 800 to about 2,500, about 900 to about 2,500, or about 1,000 per 1 ml. Preferably, the number of cells may be about 250 to about 2,500.

The total number of induced pluripotent stem cells inoculated by the above method on the start date of differentiation may be about 0.5 to about 1×10⁷ per 1 cm² of cell culture vessel area. Specifically, the total number of induced pluripotent stem cells inoculated by the above method on the start date of differentiation may be about 1 to about 5×10⁶, about 10 to about 2×10⁶, about 1×10² to about 1×10⁶, about 1×10³ to about 5×10⁵, or about 1×10⁴ to about 1×10⁵ per 1 cm² of cell culture vessel area, but is not limited thereto. Preferably, the total number of induced pluripotent stem cells inoculated by the above method on the start date of differentiation may be about 1×10³ to about 1×10⁶ per 1 cm² of cell culture vessel area.

As used herein, the term "culture" means growing cells under appropriately controlled environmental conditions, and the culture process of the present invention may be performed in an appropriate medium and under appropriate culture conditions known in the art. This culture process may be easily adjusted and used by those of ordinary skill in the art depending on the selected cells. The above "medium" means a known medium used in culturing cells, and includes all known cell culture media or modified media thereof.

The iPSCs may be cultured in a medium comprising a ROCK inhibitor. The ROCK inhibitor may be selected from the group consisting of Y-27632, thiazovivin, fasudil, GSK429286A, RKI-1447, H-1152, and azaindole 1. The iPSCs may be cultured in a medium comprising a ROCK inhibitor for about 10 minutes to about 1 week, about 30 minutes to about 6 days, about 1 hour to about 5 days, about 2 hours to about 4 days, about 3 hours to about 3 days, about 6 hours to about 2 days, about 12 hours to about 2 days, or about 18 hours to about 1 day.

The iPSCs may be cultured by an adhesion culture method. The single cell iPSCs may be cultured for about 1 day to about 2 weeks, about 2 days to about 13 days, about 3 days to about 12 days, about 4 days to about 11 days, about 5 days to about 10 days, about 6 days to about 9 days, or about 7 days to about 8 days after adhesion to a cell culture vessel.

The iPSC colony may be a mass of cells that have been proliferated by adhesion culture of single cell iPSCs. The iPSC colony may be cells having the same genetic information.

Second, the method comprises differentiating the iPSC colonies into mesodermal cells to produce mesodermal cells.

In this case, the number of colonies on the start date of differentiation from the iPSC colonies into mesodermal cells may be 1 to 20. In addition, the total number of iPSC cells on the start date of differentiation from the iPSC colonies into mesodermal cells may be 0.5 to 1×10⁶ cells per 1 cm² of cell culture vessel area. In this case, the total number of stem cells on the start date of differentiation is the same as described above. Preferably, the total number of iPSC cells on the start date of differentiation from the iPSC colonies into mesodermal cells may be 1×10³ to 1×10⁶ cells per 1 cm² of cell culture vessel area.

As used herein, the term "mesodermal cell" refers to a layered population of cells that exist between the ectoderm and endoderm that are produced during gastrulation, and may be seen in animals higher than flatworms. The mesodermal cells may be differentiated into blood cells; vascular endothelial cells; muscle, including smooth muscle and cardiac muscle; and connective tissue, such as bone, cartilage, and fat.

In this case, the iPSCs may have a size per colony of about 10 µm to about 3,000 µm .

Specifically, the iPSCs may have a size per colony of about 10 µm to about 3,000 µm , about 50 µm to about 2,500 µm, about 100 µm to about 2,000 µm, about 300 µm to about 1,200 µm, about 400 µm to about 800 µm, or about 500 µm to about 700 µm. Preferably, the iPSCs may have a size per colony of about 300 µm to about 1,200 µm, but is not limited thereto.

In addition, the iPSC colony may be cultured in a serum-free medium. The iPSC colony may be cultured in a medium capable of culturing stem cells.

The iPSC colony may be cultured in a medium comprising at least one selected from the group consisting of a glycogen synthase kinase-3 (GSK3) inhibitor, an activin-like receptor kinase 5 (ALK5) inhibitor, a bone morphogenetic protein 4 (BMP4), a stem cell factor (SCF), and a vascular endothelial growth factor (VEGF). The GSK3 inhibitor may be selected from the group consisting of CHIR-99021, SB216763, AT7519, CHIR-98014, TWS119, tideglusib, SB415286, and BIO (6-bromoindirubin-3-oxime, 6-bromoindirubin-3'-oxime). The ALK5 inhibitor may be selected from the group consisting of SB431542, galunisertib, LY2109761, SB525334, SB505124, GW788388, and LY364947.

The iPSC colony may be cultured in a medium comprising CHIR-99021, BMP4 and VEGF, and then cultured in a medium comprising SB431542, SCF and VEGF. The iPSC colony may be cultured in a medium comprising CHIR-99021, SB431542, BMP4, SCF and VEGF.

The iPSC colony may be cultured in the above medium for about 1 hour to about 2 weeks, about 12 hours to about 2 weeks, about 1 day to about 2 weeks, about 2 days to about 13 days, about 3 days to about 12 days, about 4 days to about 11 days, about 5 days to about 10 days, about 6 days to about 9 days, or about 7 days to about 8 days. Preferably, the iPSC colony may be cultured for about 2 days to about 4 days, but is not limited thereto.

The mesodermal cell may be a mesodermal stem cell.

Third, the method comprises differentiating the mesodermal cells into hematopoietic stem cells to produce hematopoietic stem cells.

As used herein, the term "hematopoietic stem cell (HSC)" may be used interchangeably with "hematopoietic precursor cell" or "hematopoietic progenitor cell" and refers to a progenitor cell capable of differentiating into a hematopoietic cell. That is, a hematopoietic stem cell (HSC) refers to a multipotent stem cell that produces all blood cell types, including myeloid (monocytes and macrophages), granulocytes (neutrophils, basophils, eosinophils, and mast cells), erythrocytes, megakaryocytes/platelets, and dendritic cells), and lymphoid (T cells, B cells, natural killer cells) (see [Doulatov et al., 2012; Notta et al., 2015]). The hematopoietic stem cell may express CD34 and CD133, and may be negative for CD38 expression. The hematopoietic progenitor cell may include a CD34+/CD45+ hematopoietic progenitor cell and a CD34+/CD45+/CD43+ hematopoietic progenitor cell.

In this case, the mesodermal cells may be cultured in a serum-free medium. In addition, the mesodermal cells may be cultured in a medium comprising STF (stem cell factor), FLT3L (FMS-like tyrosine kinase 3 ligand), or a combination thereof.

The mesodermal cells may be cultured for about 1 day to about 2 weeks, about 2 days to about 13 days, about 3 days to about 12 days, about 4 days to about 11 days, about 5 days to about 10 days, about 6 days to about 9 days or about 7 days to about 8 days. Preferably, the mesodermal cells may be cultured for about 2 days to about 8 days, but is not limited thereto.

The cells differentiated from the mesodermal cells into hematopoietic stem cells may express CD34. In this case, the CD34 expression rate may be about 80% or more of the total cells induced to differentiate without a separate sorting process for selecting only CD34 expressing cells. Specifically, the CD34 expression rate of the cells differentiated from the mesodermal cells into hematopoietic stem cells may be about 80% or more, about 81% or more, about 82% or more, about 83% or more, about 84% or more, about 85% or more, about 86% or more, about 87% or more, about 88% or more, about 89% or more, about 90% or more, about 91% or more, about 92% or more, about 93% or more, about 94% or more, about 95% or more, about 96% or more, about 97% or more, about 98% or more, or about 99% or more. Preferably, the CD34 expression rate of the cells differentiated from the mesodermal cells into hematopoietic stem cells may be about 85% or more.

The above "CD34" is a marker protein of hematopoietic stem cells and is involved in cell-cell adhesion. The sorting process for selecting only CD34 expressing cells may be performed by flow cytometry or a kit.

Fourth, the method comprises differentiating the hematopoietic stem cells into natural killer cells to produce natural killer cells.

In this case, the hematopoietic stem cells may be cultured in a serum-free medium. In addition, the hematopoietic stem cells may be cultured in a medium comprising STF (stem cell factor), FLT3L (FMS-like tyrosine kinase 3 ligand), or a combination thereof.

The hematopoietic stem cells may be cultured in a medium comprising IL-7, IL-15, or a combination thereof. The hematopoietic stem cells may be cultured in a medium comprising STF, FLT3L, IL-7, IL-15, or a combination thereof.

The hematopoietic stem cells may be cultured for about 1 day to about 3 months, about 5 days to about 2 months, about 1 week to about 2 months, about 2 weeks to about 2 months, about 3 weeks to about 2 months, about 1 month to about 2 months, about 1 month to about 7 weeks, about 1 month to about 6 weeks, about 1 month to about 5 weeks, or about 5 weeks to about 6 weeks (e.g., about 36 days). Preferably, the hematopoietic stem cells may be cultured for about 1 day to about 26 days, or about 1 day to about 48 days, but is not limited thereto.

In the present invention, the hematopoietic stem cells may be co-cultured with autologous feeder cells.

As used herein, the term "feeder cell" refers to a cell that is co-cultured with a second type of cell and provides growth factors and nutrients to enable the second type of cell to grow. The above feeder cells may be optionally derived from a species homologous or heterologous to the cells they support. For example, a specific type of human cell including stem cells may be supported by a culture of mouse embryonic fibroblasts and non-apoptotic mouse embryonic fibroblasts. The feeder cell may be a human-derived feeder cell such as a human skin fibroblast or a human embryonic stem cell. In this case, the feeder cells may be inactivated by treatment with an anti-cell division agent such as mitomycin or irradiation. Through the above inactivation, when co-cultured with a second type of cell, the feeder cells produce and secrete cellular metabolites in a state where cell division is stopped, thereby regulating the differentiation and growth of the second type of cell.

In this case, in the present invention, the feeder cell may be an autologous feeder cell.

In one embodiment, the autologous feeder cell of the present invention may be a cell that plays a role in regulating the differentiation and growth from hematopoietic stem cells to natural killer cells. In the present invention, the feeder cell may be a cell that plays a role in regulating the proliferation and activity of differentiated natural killer cells.

The above "autologous feeder cell" of the present invention includes a cell induced to differentiate from iPSCs into mesodermal cells. Preferably, it may include all cells that are not differentiated into hematopoietic stem cells and are adhered to the vessel among the cells induced to differentiate into hematopoietic stem cells together in the iPSC colony, but is not limited thereto.

In this case, the differentiation induction from iPSCs into natural killer cells may be performed *in vitro.*

The differentiation from induced pluripotent stem cells into natural killer cells may be performed by culturing for about 26 days. Specifically, the differentiation from iPSCs into natural killer cells may be performed by culturing for 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, or 26 days.

In addition, in the present invention, the natural killer cells derived from the induced pluripotent stem cells may be additionally cultured for 21 days after differentiation is completed. Specifically, the natural killer cells may be additionally cultured for 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, or 21 days.

In addition, in the present invention, a mass culture proliferation on the natural killer cells induced to differentiate from the induced pluripotent stem cells may be additionally performed.

In this case, upon mass culture proliferation of the natural killer cells induced to differentiate from the induced pluripotent stem cells, the natural killer cells may be cultured in a medium comprising STF, FLT3L, IL-7, IL-15, IL-2, or a combination thereof. In addition, the natural killer cells induced to differentiate from the induced pluripotent stem cells may be cultured in a medium further comprising β-mercaptoethanol, sodium selenite, ethanolamine, L-ascorbic acid, or a combination thereof. In this case, the natural killer cells induced to differentiate from the induced pluripotent stem cells may be cultured for about 1 day to about 9 weeks, about 1 day to about 8 weeks, about 1 day to about 7 weeks, about 1 day to about 6 weeks, about 1 day to about 5 weeks, about 1 day to about 4 weeks, or about 1 day to about 3 weeks for mass culture proliferation.

In the present invention, upon mass proliferation culture of the natural killer cells induced to differentiate from the induced pluripotent stem cells, the natural killer cells may be co-cultured with feeder cells. In this case, the feeder cell is the same as described above.

In this case, the mass culture proliferation on the natural killer cells derived from induced pluripotent stem cells may be performed *in vitro.*

In one embodiment of the present invention, the natural killer cells derived from induced pluripotent stem cells may not express the marker proteins of induced pluripotent stem cells, TRA-1-60 and SSEA4. TRA-1-60 and SSEA4 are marker proteins of stem cells, and the stem cells are the same as described above.

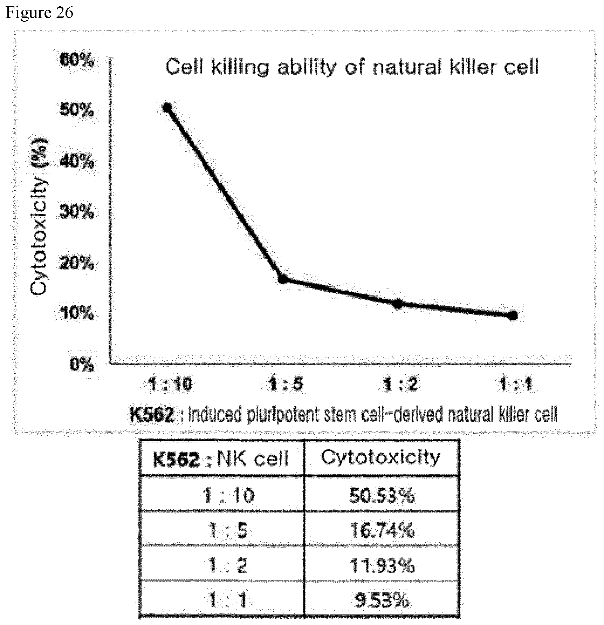

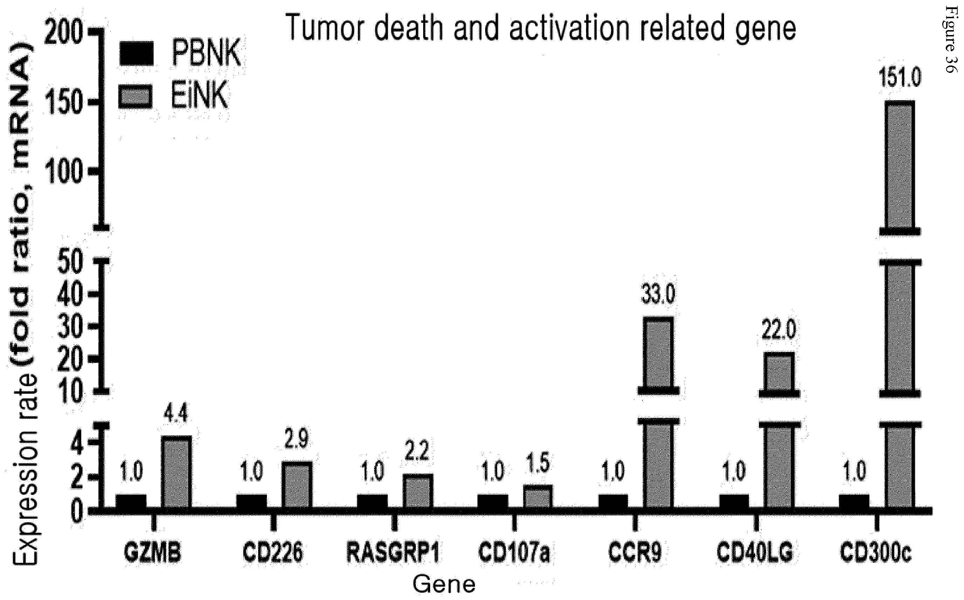

In one embodiment of the present invention, the natural killer cells exhibited the killing ability against blood cancer cell lines (Figure 26, Figure 31, Figure 34b, Figure 39e, and Table 10). In addition, it was confirmed that the natural killer cells activated immune cells more than the natural killer cells obtained from blood, and the expression of the receptor for the natural killer cell activation inhibitory protein expressed in tumor cells was reduced (Figure 36, Figure 37, Figure 38, Table 13, and Table 14). In addition, through this, it may be seen that the natural killer cells of the present invention are newly isolated natural killer cells having a high degree of natural killer cell activation and excellent killing ability against tumor cells compared to natural killer cells cultured by a conventionally known method or natural killer cells isolated from humans.

### Cell therapeutic agent comprising natural killer cells and pharmaceutical composition comprising the same

In another aspect of the present invention, there is provided a cell therapeutic agent comprising the natural killer cell of the present invention as an active ingredient.

In another aspect of the present invention, there is provided a pharmaceutical composition for preventing or treating a disease, comprising the natural killer cell of the present invention as an active ingredient.

As used herein, the term "cell therapeutic agent" is a drug used for the purpose of treatment, diagnosis, and prevention with cells and tissues prepared through isolation, culture, and special fabrication from a subject (U.S. FDA regulations). It refers to a drug used for the purpose of treatment, diagnosis, and prevention through a series of actions such as proliferating or sorting living autologous, allogeneic, or xenogeneic cells *ex vivo,* or changing the biological properties of cells in other ways to restore the function of cells or tissues. The cell therapeutic agents are largely classified into a somatic cell therapeutic agent and a stem cell therapeutic agent depending on the degree of cell differentiation. In the present invention, the cell therapeutic agent may be a somatic cell therapeutic agent.

The pharmaceutical composition of the present invention may be used for the purpose of the prevention and treatment of an infectious disease, cancer, a neurological disease, an autoimmune disease, and the like.

The infectious disease may be an infectious disease caused by hepatitis B, hepatitis C, human papilloma virus (HPV) infection, cytomegalovirus infection, viral respiratory disease, and influenza, etc.

The cancer may be liver cancer, lung cancer, colorectal cancer, breast cancer, prostate cancer, ovarian cancer, pancreatic cancer, colorectal cancer, cervical cancer, thyroid cancer, laryngeal cancer, leukemia, brain tumor, neuroblastoma, retinoblastoma, head and neck cancer, salivary gland cancer, lymphoma, and the like.

The above "neurological disease" may be a neurological disease caused by nerve damage or abnormal nerve, or a degenerative brain disease. Here, the nerve damage may be neuropraxia, axonotmesis, or neurotmesis.

The neurological disease caused by nerve damage or abnormal nerve may be a neurological disease caused by nerve damage or abnormal nerve in the central nervous system, or a neurological disease caused by nerve damage or abnormal nerve in the peripheral nervous system.

The neurological disease caused by nerve damage or abnormal nerve in the central nervous system may be an organic disease and dysfunction in the central nervous system, epilepsy, multiple sclerosis, amyotrophic lateral sclerosis, Alzheimer's disease, Lewy body dementia, Huntington's disease, Parkinson's disease, schizophrenia, traumatic brain injury, stroke, Pick's disease, Creutzfeldt-Jakob disease, progressive supranuclear palsy, multiple system atrophy, corticobasal degeneration, spinocerebellar degeneration, cerebellar atrophy, posttraumatic stress disorder, amnesia, vascular dementia, and cerebral infarction, etc.

The neurological disease caused by nerve damage or abnormal nerve in the peripheral nervous system may be peripheral neuropathy, diabetic neuropathy, peripheral neuropathic pain, peripheral neuropathy due to anticancer chemotherapy, complex regional pain syndrome, optic neuropathy, mononeuropathy, multiple mononeuropathy (mononeuritis multiplex), polyneuropathy, Guillain-Barré syndrome (acute inflammatory demyelinating polyneuropathy), chronic inflammatory demyelinating polyneuropathy, hereditary neuropathy, plexus disorder, glaucoma, macular degeneration, amyotrophic lateral sclerosis, progressive muscular atrophy, progressive bulbar palsy, poliomyelitis, post-polio syndrome, stiff person syndrome, Isaacs' syndrome, myasthenia gravis, neonatal myasthenia, botulism, Eaton-Lambert syndrome, thoracic outlet syndrome, Charcot-Marie-Tooth disease, and spinal muscular atrophy, etc.

The above "degenerative brain disease" refers to a disease that occurs in the brain among degenerative diseases that occur with age. The degenerative brain disease may include all diseases caused by death of brain nerve cells, problems in the formation or function of synapses that transmit information between brain nerve cells, or abnormal increases or decreases in the electrical activity of brain nerves. The degenerative brain disease may include Alzheimer's disease, Parkinson's disease, Huntington's disease, mild cognitive impairment, cerebral amyloid angiopathy, Down syndrome, amyloid stroke, systemic amyloid disease, Dutch type amyloidosis, Niemann-Pick disease, senile dementia, amyotrophic lateral sclerosis, spinocerebellar atrophy, Tourette's syndrome, Friedrich's ataxia, Machado-Joseph's disease, Lewy body dementia, dystonia, progressive supranuclear palsy, and frontotemporal dementia, etc., but is not limited thereto.

As used herein, the term "autoimmune disease" refers to a general term for diseases caused by an antigen-antibody reaction due to antibodies produced against one's own biological substances or body tissues. The autoimmune disease may include Crohn's disease, erythema, atopy, rheumatoid arthritis, Hashimoto's thyroiditis, pernicious anemia, Addison's disease, type 1 diabetes mellitus, lupus, chronic fatigue syndrome, fibromyalgia, hypothyroidism and hyperthyroidism, scleroderma, Behcet's disease, inflammatory bowel disease, multiple sclerosis, myasthenia gravis, Meniere's syndrome, Guillain-Barré syndrome, Sjogren's syndrome, vitiligo, endometriosis, psoriasis, vitiligo, systemic scleroderma, asthma, ulcerative colitis, and the like, but is not limited thereto.

As used herein, the term "prevention" refers to any action of inhibiting or delaying the onset of a senile disease by administration of the pharmaceutical composition. The term "treatment" refers to any action in which symptoms of a disease are improved or beneficially changed by administration of the pharmaceutical composition.

In the pharmaceutical composition of the present invention, the natural killer cells may be included in any amount (effective amount) depending on the purpose, formulation, mixing purpose, and the like, as long as they may exhibit activity. The pharmaceutical composition of the present invention may comprise 1×10² to 1×10¹⁶, 1×10² to 1×10¹⁵, 1×10² to 1×10¹⁴, or 1×10² to 1×10¹³ activated natural killer cells. Preferably, the pharmaceutical composition may comprise 2×10² to 2×10¹¹ activated natural killer cells.

Here, the "effective amount" refers to an amount of an active ingredient capable of inducing an anti-aging effect. Such an effective amount may be experimentally determined within the normal capabilities of those of ordinary skill in the art.

The composition of the present invention may be used unfrozen or may be frozen for later use. If frozen, standard cryopreservatives (e.g., DMSO, glycerol, polyvinylpyrrolidone, polyethylene glycol, albumin, dextran, sucrose, ethylene glycol, i-d erythritol, D-ribitol, D-mannitol, D-sorbitol, i-inositol, D-lactose, choline chloride, and Epilife^{®} cell freezing medium (Ccascade Biologics)) may be added to the cells prior to freezing.

The pharmaceutical composition comprising the natural killer cell of the present invention as an active ingredient may be formulated by further comprising a pharmaceutically acceptable carrier.

As used herein, the term "pharmaceutically acceptable carrier" refers to a carrier or diluent that does not significantly stimulate an organism and does not inhibit the biological activity and properties of the administered ingredient. The pharmaceutical composition of the present invention may be applied as a cell therapeutic agent, and the pharmaceutically acceptable carrier that may be included in a cell therapeutic agent may be used without limitation as long as it is known in the art, such as a buffering agent, a preservative, an analgesic agent, a solubilizer, an isotonic agent, a stabilizer, a base, an excipient, a lubricant, and the like. The pharmaceutical composition of the present invention may be prepared in the form of various formulations according to commonly used techniques.

The pharmaceutical composition of the present invention may be administered in a pharmaceutically effective amount, and may be administered through any route as long as it may induce migration to the diseased site. Here, "administration" means introducing a predetermined substance to a subject by an appropriate method. In some cases, it may be considered that the pharmaceutical composition of the present invention is loaded into a vehicle comprising a means for directing natural killer cells to a lesion.

Therefore, the pharmaceutical composition of the present invention may be administered through several routes including topical (including buccal, sublingual, dermal and intraocular administration), parenteral (including subcutaneous, intradermal, intramuscular, instillation, intravenous, intraarterial, intraarticular and intracerebrospinal fluid) or transdermal administration, and preferably, may be administered directly to the site of disease. In one embodiment, the natural killer cells may be suspended in a suitable diluent and administered to a subject, and the diluent is used to protect and maintain the cells and to facilitate their use when injected into the target tissue. The diluent may include a buffer solution such as physiological saline, a phosphate buffer solution, and HBSS, cerebrospinal fluid, and the like. In addition, the composition may be administered by any device to allow the active substance to migrate to the target cell.

The preferred mode of administration and formulation is an injection. The injection may be prepared using aqueous solutions such as physiological saline, Ringer's solution, Hank's solution or sterilized aqueous solution, vegetable oils such as olive oil, higher fatty acid ester such as ethyl oleic acid, and non-aqueous solvents such as ethanol, benzyl alcohol, propylene glycol, polyethylene glycol or glycerin, and the like. For mucosal penetration, a non-penetrating agent known in the art suitable for a barrier to pass through may be used, and may further include a pharmaceutical carrier such as ascorbic acid, sodium hydrogen sulfite, BHA, tocopherol, EDTA, and the like as a stabilizer for preventing deterioration, and an emulsifier, a buffering agent for pH adjustment, a preservative for inhibiting the growth of microorganisms such as phenylmercuric nitrate, thimerosal, benzalkonium chloride, phenol, cresol, and benzyl alcohol. Methods for formulating pharmaceutical compositions are known in the art, and specific references may be made to literature [Remington's Pharmaceutical Sciences (19th ed., 1995)], etc. The above literature is considered a part of the present specification.

As used herein, the term "pharmaceutically effective amount" refers to an amount sufficient to treat a disease at a reasonable benefit/risk ratio applicable to medical treatment and not causing side effects. The effective dose level may be readily determined by those of ordinary skill in the art based on factors including the patient's sex, age, body weight, health condition, type and severity of the disease, activity of the drug, sensitivity to the drug, method of administration, time of administration, route of administration and excretion rate, duration of treatment, drugs used in combination or simultaneously, and other factors well known in the medical field.

The preferred dosage of the pharmaceutical composition of the present invention may be about 1×10/kg to about 1×10¹²/kg, about 1×10/kg to about 1×10¹¹/kg, or about 1×10/kg to about 1×10 ¹⁰/kg per day based on cells, depending on the patient's condition, body weight, sex, and age, severity of the patient, and route of administration.

However, the dosage may be prescribed in various ways depending on factors such as the formulation method, mode of administration, patient's age, body weight, sex, pathological condition, food, administration time, route of administration, excretion rate, and response sensitivity, and those of ordinary skill in the art may appropriately adjust the dosage by considering these factors. The frequency of administration may be once or twice or more within the range of clinically acceptable side effects. The pharmaceutical composition of the present invention may be administered 1 to 3 times/day, 1 to 3 times/week, or 1 to 10 times/month. For example, the pharmaceutical composition of the present invention may be administered 1 to 9 times/month, 1 to 8 times/month, 1 to 7 times/month, 1 to 6 times/month, 1 to 5 times/month, 1 to 4 times/month, 1 to 3 times/month, or 1 to 2 times/month. Regarding the administration site, it may be administered to 1 site or 2 or more sites. For animals other than humans, it may be administered in the same dosage as humans per kg or per subject, or it may be administered in an amount converted from the above dosage by, for example, the volume ratio (for example, the average value) of the organ (heart, etc.) between the target animal and the human. Such dosage should not be construed as limiting the scope of the present invention in any way.

As used herein, the term "subject" refers to a subject that has developed or may develop a disease to which the composition of the present invention may be applied (prescribed), and may be a mammal such as a rat, mouse, or livestock, including a human. Preferably, it may be a human, but is not limited thereto.

The pharmaceutical composition of the present invention may further comprise a known substance that exhibits a preventive or therapeutic effect on the disease, if necessary.

The pharmaceutical composition of the present invention may further comprise the aforementioned substance that increases the activity of natural killer cells. The pharmaceutical composition of the present invention may be administered in combination with a conventionally known substance for preventing or treating the disease. In this case, the natural killer cells, the substance exhibiting a preventive or therapeutic effect on the disease, and/or the substance that increases the activity of natural killer cells may be administered simultaneously or sequentially.

In another aspect of the present invention, there is provided a use of the natural killer cell or a pharmaceutical composition comprising the same as an active ingredient for the prevention and treatment of a disease. Here, the natural killer cell, the pharmaceutical composition, the disease, the prevention and the treatment are the same as described above.

In another aspect of the present invention, there is provided a method for treating a disease, comprising: administering the natural killer cell or a pharmaceutical composition comprising the same as an active ingredient to a subject. In this case, the natural killer cell, the pharmaceutical composition, the disease, the administration and the treatment are the same as described above.

The subject may be any animal, such as a rat, mouse, or livestock, including a human who has developed or may develop the disease. Preferably, it may be a human.

The dosage may be about 1×10/kg to about 1×10¹²/kg, about 1×10/kg to about 1×10¹¹/kg, or about 1×10/kg to about 1×10¹⁰/kg per day based on cells depending on the patient's condition, body weight, sex, and age, severity of the patient, and route of administration. However, the dosage may be prescribed in various ways depending on factors such as the formulation method, mode of administration, patient's age, body weight, sex, pathological condition, food, administration time, route of administration, excretion rate, and response sensitivity, and those of ordinary skill in the art may appropriately adjust the dosage by considering these factors.

### Method for producing natural killer cells

In another aspect of the present invention, there is provided a method for producing natural killer cells, comprising: inoculating induced pluripotent stem cells as iPSCs or iPSC spheroids into a cell culture vessel so that 0.5 to 20 colonies are formed per 1 cm² of cell culture vessel area and performing adhesion culture to produce iPSC colonies; differentiating the iPSC colonies into mesodermal cells to produce mesodermal cells; differentiating the mesodermal cells into hematopoietic stem cells to produce hematopoietic stem cells; and differentiating the hematopoietic stem cells into natural killer cells to produce natural killer cells.

In this case, the induced pluripotent stem cells may be cultured by an adhesion culture method, and may be inoculated as a single cell or spheroid and subjected to adhesion culture. The number of cells included in the spheroid is the same as described above.

In addition, the number of the inoculated iPSCs or iPSC spheroid may be 0.5 to 20 per 1 cm² of area. The inoculated iPSCs or iPSC spheroids are the same as described above.

In addition, the number of inoculated cells may be calculated in another way. The number of cells present per ml of culture solution may be determined as the number of cells per cm² by using a method for calculating the number of inoculated cells.

In addition, the method for producing natural killer cells of the present invention may further comprise a mass culture proliferation on the natural killer cells differentiated from the induced pluripotent stem cells.

In this case, the induced pluripotent stem cells, differentiation, natural killer cells, and culture are the same as described above. In addition, the method for calculating the number of the inoculated iPSCs and the number of inoculated cells per 1 cm² of cell culture vessel area in a different way is the same as described above.

The iPSCs may be cultured in a medium comprising a ROCK inhibitor. In this case, the ROCK inhibitor is the same as described above. The iPSCs may be cultured in a medium comprising a ROCK inhibitor for about 10 minutes to about 1 week, about 30 minutes to about 6 days, about 1 hour to about 5 days, about 2 hours to about 4 days, about 3 hours to about 3 days, about 6 hours to about 2 days, about 12 hours to about 2 days, or about 18 hours to about 1 day. In addition, the iPSCs may be cultured by an adhesion culture method. The single cell iPSCs may be cultured for about 1 day to about 2 weeks, about 2 days to about 13 days, about 3 days to about 12 days, about 4 days to about 11 days, about 5 days to about 10 days, about 6 days to about 9 days, or about 7 days to about 8 days after adhesion to a cell culture vessel.

The iPSC colony may be a mass of cells that have been proliferated by adhesion culture of single cell iPSCs. The iPSC colony may be cells having the same genetic information.

The iPSC colonies may be differentiated into mesodermal cells to produce mesodermal cells. The total number of induced pluripotent stem cells on the start date of differentiation and the mesodermal cell are the same as described above. In this case, the total number of iPSC stem cells on the start date of differentiation from the iPSC colonies into mesodermal cells may be 0.5 to 1×10⁶ cells per 1 cm² of cell culture vessel area. In this case, the iPSCs may have a size per colony of about 10 µm to about 3,000 µm. Specifically, the iPSCs may have a size per colony of about 10 µm to about 3,000 µm, about 50 µm to about 2,500 µm, about 100 µm to about 2,000 µm, about 300 µm to about 1,200 µm, about 400 µm to about 800 µm, or about 500 µm to about 700 µm. Preferably, the iPSCs may have a size per colony of about 300 µm to about 1,200 µm, but is not limited thereto.

In addition, the iPSC colony may be cultured in a serum-free medium. The iPSC colony may be cultured in a medium capable of culturing stem cells. In addition, the iPSC colony may be cultured in a medium comprising at least one selected from the group consisting of a GSK3 inhibitor, an ALK5 inhibitor, BMP4, SCF, and VEGF. The GSK3 inhibitor and the ALK5 inhibitor are the same as described above.

The iPSC colony may be cultured in a medium comprising CHIR-99021, BMP4 and VEGF, and then cultured in a medium comprising SB431542, SCF and VEGF. The iPSC colony may be cultured in a medium comprising CHIR-99021, SB431542, BMP4, SCF and VEGF. In this case, the iPSC colony may be cultured in the above medium for about 1 hour to about 2 weeks, about 12 hours to about 2 weeks, about 1 day to about 2 weeks, about 2 days to about 13 days, about 3 days to about 12 days, about 4 days to about 11 days, about 5 days to about 10 days, about 6 days to about 9 days, or about 7 days to about 8 days. Preferably, the iPSC colony may be cultured for about 2 days to about 4 days, but is not limited thereto.

The mesodermal cell may be a mesodermal stem cell.

The mesodermal cells may be differentiated into hematopoietic stem cells to produce hematopoietic stem cells. In this case, the hematopoietic stem cell is the same as described above.

The mesodermal cells may be cultured in a serum-free medium and may be cultured in a medium comprising STF, FLT3L, or a combination thereof. In this case, the mesodermal cells may be cultured for about 1 day to about 2 weeks, about 2 days to about 13 days, about 3 days to about 12 days, about 4 days to about 11 days, about 5 days to about 10 days, about 6 days to about 9 days, or about 7 days to about 8 days. Preferably, the mesodermal cells may be cultured for about 2 days to about 8 days, but is not limited thereto.

The cells differentiated from the mesodermal cells into hematopoietic stem cells may express CD34. In this case, the CD34 expression rate may be about 80% or more of the total cells induced to differentiate without a separate sorting process for selecting only CD34 expressing cells. Specifically, the CD34 expression rate of the total cells differentiated from the mesodermal cells into hematopoietic stem cells may be about 80% or more, about 81% or more, about 82% or more, about 83% or more, about 84% or more, about 85% or more, about 86% or more, about 87% or more, about 88% or more, about 89% or more, about 90% or more, about 91% or more, about 92% or more, about 93% or more, about 94% or more, about 95% or more, about 96% or more, about 97% or more, about 98% or more, or about 99% or more. Preferably, the CD34 expression rate of the total cells differentiated from the mesodermal cells into hematopoietic stem cells may be about 85% or more.

The hematopoietic stem cells may be cultured in a medium comprising IL-7, IL-15, or a combination thereof. The hematopoietic stem cells may be cultured in a medium comprising STF, FLT3L, IL-7, IL-15, or a combination thereof.

The hematopoietic stem cells may be cultured for about 1 day to about 3 months, about 5 days to about 2 months, about 1 week to about 2 months, about 2 weeks to about 2 months, about 3 weeks to about 2 months, about 1 month to about 2 months, about 1 month to about 7 weeks, about 1 month to about 6 weeks, about 1 month to about 5 weeks, or about 5 weeks to about 6 weeks (e.g., about 36 days). Preferably, the hematopoietic stem cells may be cultured for about 1 day to about 26 days, or about 1 day to about 48 days, but is not limited thereto.

In the present invention, the hematopoietic stem cells may be co-cultured with autologous feeder cells.

The feeder cell is the same as described above. In this case, in the present invention, the feeder cell may be an autologous feeder cell.

In one embodiment, the autologous feeder cell of the present invention may be a cell that plays a role in regulating the differentiation and growth from hematopoietic stem cells to natural killer cells. In the present invention, the feeder cell may be a cell that plays a role in regulating the proliferation and activity of differentiated natural killer cells.

The above "autologous feeder cell" of the present invention includes a cell induced to differentiate from iPSCs into mesodermal cells. Preferably, it may include all cells that are not differentiated into hematopoietic stem cells and are adhered to the vessel among the cells induced to differentiate into hematopoietic stem cells together in the iPSC colony, but is not limited thereto.

The method may be performed *in vitro.*

The above medium may be a known medium used in cell culture or a modified medium thereof.

In the method for producing natural killer cells of the present invention, a mass culture proliferation on the natural killer cells induced to differentiate may be additionally performed.

In this case, upon the mass culture proliferation, the natural killer cells induced to differentiate from the induced pluripotent stem cells may be cultured in a medium comprising STF, FLT3L, IL-7, IL-15, IL-2, or a combination thereof. In addition, the natural killer cells induced to differentiate from the induced pluripotent stem cells may be cultured in a medium further comprising β-mercaptoethanol, sodium selenite, ethanolamine, L-ascorbic acid, or a combination thereof. In this case, the mass culture proliferation may be performed for about 1 day to about 4 weeks, or for about 1 day to about 3 weeks.

In the present invention, upon mass proliferation culture of the natural killer cells induced to differentiate from the induced pluripotent stem cells, the natural killer cells may be co-cultured with feeder cells. In this case, the feeder cell is the same as described above.

In this case, the mass culture proliferation on the natural killer cells derived from induced pluripotent stem cells may be performed *in vitro.*

The natural killer cells produced by the above method may express any one protein selected from the group consisting of CD25, CD9, CD80, and a combination thereof. In addition, the natural killer cells may additionally express any one protein selected from the group consisting of CD1d, CD30, CD87, CDw327, CD33, CD49a, CD49c, CD49e, CD49f, CD66a, CD66c, CD66d, CD66e, CD71, CD83, CDw93, CD106, CD108, CD114, CD123, CD124, CD146, CD150, CD164, CD196, CD210, and a combination thereof.

Specifically, about 50% or more, about 60% or more, about 70% or more, about 80% or more, about 90% or more, about 91% or more, about 92% or more, about 93% or more, about 94% or more, or about 95% or more of the natural killer cell population of produced by the method of the present invention may express CD25.

Specifically, about 50% or more, about 60% or more, about 70% or more, about 80% or more, about 90% or more, about 91% or more, about 92% or more, about 93% or more, about 94% or more, about 95% or more, about 96% or more, or about 97% or more of the natural killer cell population of produced by the method of the present invention may express CD80.

Specifically, about 10% or more, about 20% or more, about 30% or more, about 31% or more, about 32% or more, about 33% or more, or about 34% or more of the natural killer cell population of produced by the method of the present invention may express CD9.

Specifically, about 5% or more, about 6% or more, or about 7% or more of the natural killer cell population of produced by the method of the present invention may express CD1d.

Specifically, about 20% or more, about 30% or more, about 40% or more, about 41% or more, about 42% or more, about 43% or more, about 44% or more, or about 45% or more of the natural killer cell population of produced by the method of the present invention may express CD30.

Specifically, about 20% or more, about 30% or more, about 40% or more, about 41% or more, about 42% or more, about 43% or more, about 44% or more, about 45% or more, or about 46% or more of the natural killer cell population of produced by the method of the present invention may express CD87.

Specifically, about 10% or more, about 20% or more, about 21% or more, about 22% or more, about 23% or more, about 24% or more, about 25% or more, or about 26% or more of the natural killer cell population of produced by the method of the present invention may express CDw327.

Specifically, about 10% or more, about 11% or more, about 12% or more, about 13% or more, or about 14% or more of the natural killer cell population of produced by the method of the present invention may express CD33.

Specifically, about 50% or more, about 60% or more, about 70% or more, about 80% or more, about 90% or more, about 91% or more, about 92% or more, about 93% or more, about 94% or more, about 95% or more, about 96% or more, about 97% or more, about 98% or more, or about 99% or more of the natural killer cell population of produced by the method of the present invention may express CD49a.

Specifically, about 50% or more, about 60% or more, about 70% or more, about 80% or more, about 90% or more, about 91% or more, about 92% or more, about 93% or more, or about 94% or more of the natural killer cell population of produced by the method of the present invention may express CD49c.

Specifically, about 50% or more, about 60% or more, about 70% or more, about 80% or more, about 90% or more, about 91% or more, about 92% or more, about 93% or more, about 94% or more, about 95% or more, or about 96% or more of the natural killer cell population of produced by the method of the present invention may express CD49e.

Specifically, about 5% or more, about 6% or more, about 7% or more, about 8% or more, about 9% or more, about 10% or more, about 11% or more, about 12% or more, about 13% or more, about 14% or more, or about 15% or more of the natural killer cell population of produced by the method of the present invention may express CD49f.

Specifically, about 50% or more, about 60% or more, about 70% or more, about 74% or more, about 75% or more, about 76% or more, about 77% or more, about 78% or more, about 79% or more, about 80% or more, about 81% or more, about 82% or more, about 83% or more, or about 84% or more of the natural killer cell population of produced by the method of the present invention may express CD66 (a/b/c/d/e).

Specifically, about 50% or more, about 60% or more, about 70% or more, about 80% or more, about 90% or more, about 91% or more, about 92% or more, about 93% or more, about 94% or more, about 95% or more, or about 96% or more of the natural killer cell population of produced by the method of the present invention may express CD71.

Specifically, about 30% or more, about 31% or more, about 32% or more, about 33% or more, about 34% or more, about 35% or more, about 36% or more, about 37% or more, about 38% or more, about 39% or more, or about 40% or more of the natural killer cell population of produced by the method of the present invention may express CD83.

Specifically, about 50% or more, about 60% or more, about 70% or more, about 80% or more, about 90% or more, about 91% or more, about 92% or more, about 93% or more, about 94% or more, or about 95% or more of the natural killer cell population of produced by the method of the present invention may express CDw93.

Specifically, about 20% or more, about 21% or more, about 22% or more, about 23% or more, about 24% or more, about 25% or more, about 26% or more, about 27% or more, about 28% or more, about 29% or more, about 30% or more, about 31% or more, or about 32% or more of the natural killer cell population of produced by the method of the present invention may express CD106.

Specifically, about 50% or more, about 60% or more, about 70% or more, about 80% or more, about 90% or more, about 91% or more, about 92% or more, about 93% or more, or about 94% or more of the natural killer cell population of produced by the method of the present invention may express CD108.

Specifically, about 20% or more, about 30% or more, about 35% or more, about 36% or more, about 37% or more, about 38% or more, about 39% or more, about 40% or more, about 41% or more, or about 42% or more of the natural killer cell population of produced by the method of the present invention may express CD114.

Specifically, about 10% or more, about 20% or more, about 30% or more, about 31% or more, about 32% or more, about 33% or more, about 34% or more, or about 35% or more of the natural killer cell population of produced by the method of the present invention may express CD123.

Specifically, about 5% or more, about 6% or more, about 7% or more, about 8% or more, or about 9% or more of the natural killer cell population of produced by the method of the present invention may express CD124.

Specifically, about 50% or more, about 60% or more, about 61% or more, about 62% or more, about 63% or more, about 64% or more, or about 65% or more of the natural killer cell population of produced by the method of the present invention may express CD146.

Specifically, about 10% or more, about 20% or more, about 25% or more, about 26% or more, about 27% or more, about 28% or more, about 29% or more, about 30% or more, about 31% or more, or about 32% or more of the natural killer cell population of produced by the method of the present invention may express CD 150.

Specifically, about 70% or more, about 80% or more, about 90% or more, about 91% or more, about 92% or more, about 93% or more, about 94% or more, about 95% or more, about 96% or more, or about 97% or more of the natural killer cell population of produced by the method of the present invention may express CD 164.

Specifically, about 50% or more, about 60% or more, about 70% or more, about 80% or more, about 90% or more, about 91% or more, about 92% or more, about 93% or more, about 94% or more, or about 95% or more of the natural killer cell population of produced by the method of the present invention may express CD 196.

Specifically, about 5% or more, about 6% or more, about 7% or more, about 8% or more, about 9% or more, or about 10% or more of the natural killer cell population of produced by the method of the present invention may express CD210.

The expression level of the above CD25, CD9, CD1d, CD30, CD87, CDw327, CD33, CD49a, CD49c, CD49e, CD49f, CD66a, CD66c, CD66d, CD66e, CD71, CD83, CDw93, CD106, CD108, CD114, CD123, CD124, CD146, CD150, CD164, CD196 and CD210 proteins may be reduced by at least 5 times compared to a natural killer cell isolated from blood. Specifically, the expression level may be increased by about at least two times. The natural killer cell obtained from blood, CD25, CD9, CD1d, CD30, CD87, CDw327, CD33, CD49a, CD49c, CD49e, CD49f, CD66a, CD66c, CD66d, CD66e, CD71, CD83, CDw93, CD106, CD108, CD114, CD123, CD124, CD146, CD150, CD164, CD196 and CD210 are the same as described above.

The natural killer cell may express any one natural killer cell marker protein selected from the group consisting of CD56, CD45, CD16, NKp30, NKp44, NKG2D, DNAM1, and a combination thereof, and may not express CD3, a marker protein of T cells, or may express it at a lower level compared to T cells.

Specifically, about 50% or more, about 60% or more, about 70% or more, about 80% or more, about 90% or more, about 91% or more, about 92% or more, about 93% or more, about 94% or more, about 95% or more, or about 96% or more of the natural killer cell population of produced by the method of the present invention may express CD56, CD45, NKp30 or DNAM1.

Specifically, about 40% or more, about 50% or more, about 51% or more, about 52% or more, about 53% or more, about 54% or more, about 55% or more, about 56% or more, about 57% or more, or about 58% or more of the natural killer cell population of produced by the method of the present invention may express CD 16.

Specifically, about 50% or more, about 60% or more, about 61% or more, about 62% or more, about 63% or more, about 64% or more, about 65% or more, about 66% or more, about 67% or more, about 68% or more, about 69% or more, about 70% or more, about 71% or more, or about 72% or more of the natural killer cell population of produced by the method of the present invention may express NKp44.

Specifically, about 70% or more, about 81% or more, about 82% or more, about 83% or more, about 84% or more, about 85% or more, about 86% or more, about 87% or more, about 88% or more, or about 89% or more of the natural killer cell population of produced by the method of the present invention may express NKD2D.

In addition, about 90% or more, about 9% or more, about 96% or more, about 97% or more, about 98% or more, about 99% or more, about 99.1% or more, about 99.2% or more, about 99.3% or more, or about 99.4% or more of the natural killer cell population of produced by the method of the present invention may not express CD3.

In addition, the natural killer cell according to the method of the present invention may have an additional increase in the expression of any one gene selected from the group consisting of GZMB, CD226, RASGRP1, CD107a, CCR9, CD40LG, CD300c, and a combination thereof compared to a natural killer cell isolated from blood. The above GZMB, CD226, RASGRP1, CD107a, CCR9, CD40LG and CD300c are the same as described above.

The natural killer cell according to the present invention may be characterized by increased secretion of any one protein selected from the group consisting of FLT3L, MIP 1 alpha/beta, PF4, IGFBP2, IGFBP3, MMP9, and a combination thereof compared to a natural killer cell isolated from blood. In this case, the secretion of the above protein may be increased by at least two times compared to a natural killer cell isolated from blood. The natural killer cell obtained from blood, FLT3L, MIP1alpha/beta, PF4, IGFBP2, IGFBP3 and MMP9 are the same as described above.

The natural killer cell according to the present invention may not additionally express any one protein selected from the group consisting of CD205, CD158b, CD243, γδTCR, NKB1, CD158a, CDw328, TIM3, TIGIT, and a combination thereof, or may express it at a lower level compared to a natural killer cell obtained from blood. The natural killer cell obtained from blood, CD205, CD158b, CD243, γδTCR, NKB1, CD158a, CDw328, TIM3 and TIGIT are the same as described above.

Specifically, about 90% or more, about 95% or more, about 96% or more, or about 97% or more, about 97.5% or more, about 97.6% or more, about 97.7% or more, about 97.8% or more, or about 97.9% or more of the natural killer cell population of produced by the method of the present invention may not express CD205.

In addition, about 90% or more, about 95% or more, about 96% or more, about 97% or more, about 97.1% or more, about 97.2% or more, about 97.3% or more, about 97.4% or more of the natural killer cell population of produced by the method of the present invention may not express CD158b.

In addition, about 90% or more, about 95% or more, about 96% or more, about 97% or more, about 98% or more, about 99% or more, about 99.1% or more, about 99.2% or more, about 99.3% or more, about 99.4% or more, about 99.5% or more, about 99.6% or more, or about 99.7% or more of the natural killer cell population of produced by the method of the present invention may not express CD243.

In addition, about 90% or more, about 95% or more, about 96% or more, about 97% or more, about 98% or more, about 99% or more, about 99.1% or more, about 99.2% or more, about 99.3% or more, about 99.4% or more, about 99.5% or more, about 99.6% or more, or about 99.7% or more of the natural killer cell population of produced by the method of the present invention may not express γδTCR.

In addition, about 90% or more, about 95% or more, about 96% or more, about 97% or more, about 98% or more, about 99% or more, about 99.1% or more, about 99.2% or more, about 99.3% or more, about 99.4% or more, or about 99.5% or more of the natural killer cell population of produced by the method of the present invention may not express NKB1.

In addition, about 90% or more, about 95% or more, about 96% or more, about 97% or more, about 98% or more, about 99% or more, about 99.1% or more, about 99.2% or more, about 99.3% or more, about 99.4% or more, about 99.5% or more, or about 99.6% or more of the natural killer cell population of produced by the method of the present invention may not express CD158a.

In addition, about 90% or more, about 95% or more, about 96% or more, about 97% or more, about 98% or more, about 98.1% or more, about 98.2% or more, about 98.3% or more, about 98.4% or more, about 98.5% or more, about 98.6% or more, or about 98.7% or more of the natural killer cell population of produced by the method of the present invention may not express CDw328.

In addition, about 60% or more, about 65% or more, about 70% or more, about 75% or more, about 80% or more, about 85% or more, about 90% or more, about 95% or more, about 96% or more, about 97% or more, about 98% or more, or about 99% or more of the natural killer cell population of produced by the method of the present invention may not express TIM-3.

In addition, about 80% or more, about 85% or more, about 90% or more, about 93% or more, about 95% or more, about 96% or more, about 97% or more, about 98% or more, or about 99% or more of the natural killer cell population of produced by the method of the present invention may not express TIGIT.

The expression level of the above CD205, CD158b, CD243, γδTCR, NKB1, CD158a, CDw328, TIM-3, and TIGIT proteins may be reduced by about at least two times compared to a natural killer cell obtained from blood. The natural killer cell obtained from blood is the same as described above.

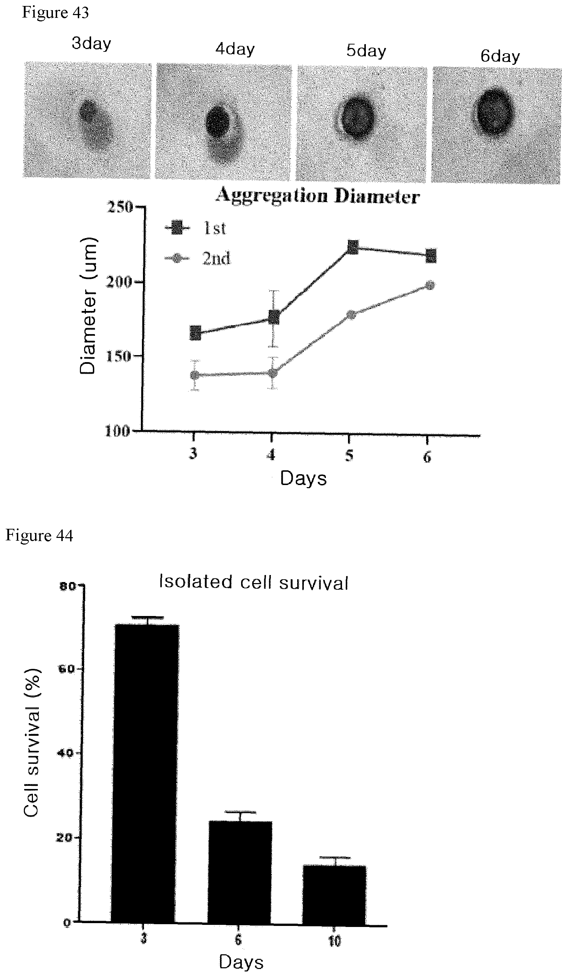

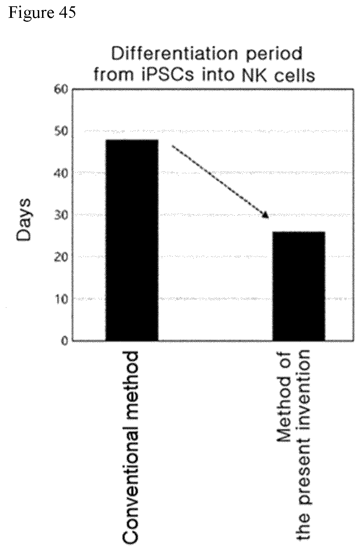

Typically, it was confirmed that when iPSCs were subjected to the third culture using Aggrewell, the size of the embryoid body was larger compared to the second culture (adhesion culture) (Figure 44). In addition, it was confirmed that there was a difference in the size of the embryoid body by batch, and that the cell survival rate was reduced when obtaining a single cell by treatment with a degrading enzyme (Figure 45). On the other hand, the method for producing natural killer cells according to the present invention may be simpler and more efficient than the third culture since iPSCs are subjected to the second culture (adhesion culture) and are induced to differentiate.

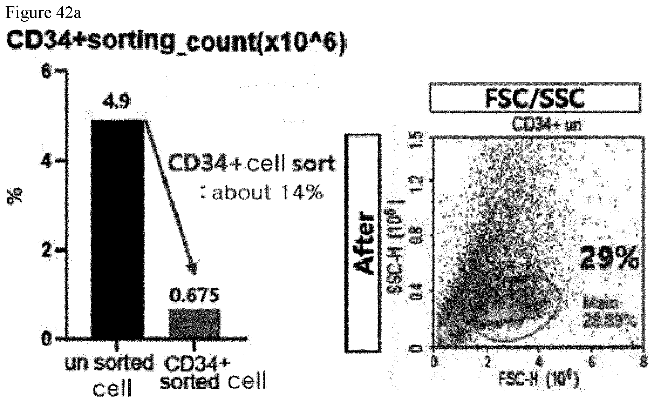

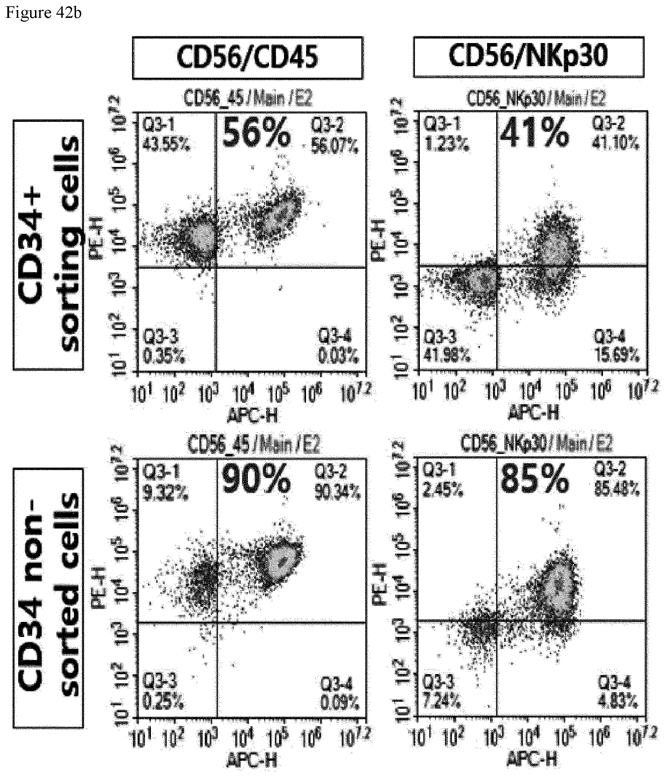

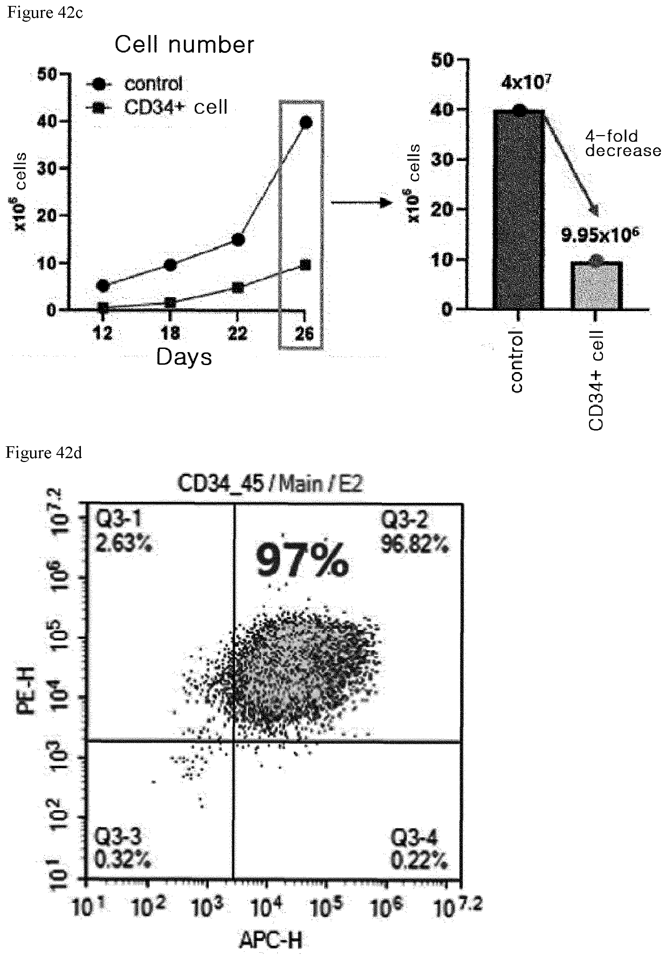

In addition, when producing natural killer cells from induced pluripotent stem cells by a conventional method, differentiation is induced from mesodermal cells to hematopoietic stem cells to obtain only CD34 positive cells (hematopoietic stem cells), which are then induced to differentiate into natural killer cells. In this case, the process of sorting only CD34 positive cells may use flow cytometry or a commercially available sorting kit. In the case of cells that have been subjected to the process of sorting CD34 positive cells in this way, it was confirmed that the cell yield was significantly reduced, cell death was increased, and the expression rate of the marker protein (CD56) and the activation marker protein (NKp30) of natural killer cells in the sorted CD34 positive cells was reduced (Figures 42a and 42b). In addition, it was confirmed that the cell proliferation rate was also significantly reduced (Figure 42c). On the other hand, in one embodiment of the present invention, the method for producing natural killer cells of the present invention may induce differentiation into natural killer cells without performing a CD34 positive cell sorting process because the expression rate of CD34, a hematopoietic stem cell differentiation marker in the intermediate stage of NK differentiation, is high at 95% or higher, thereby increasing cell yield and activity (Figure 42d).

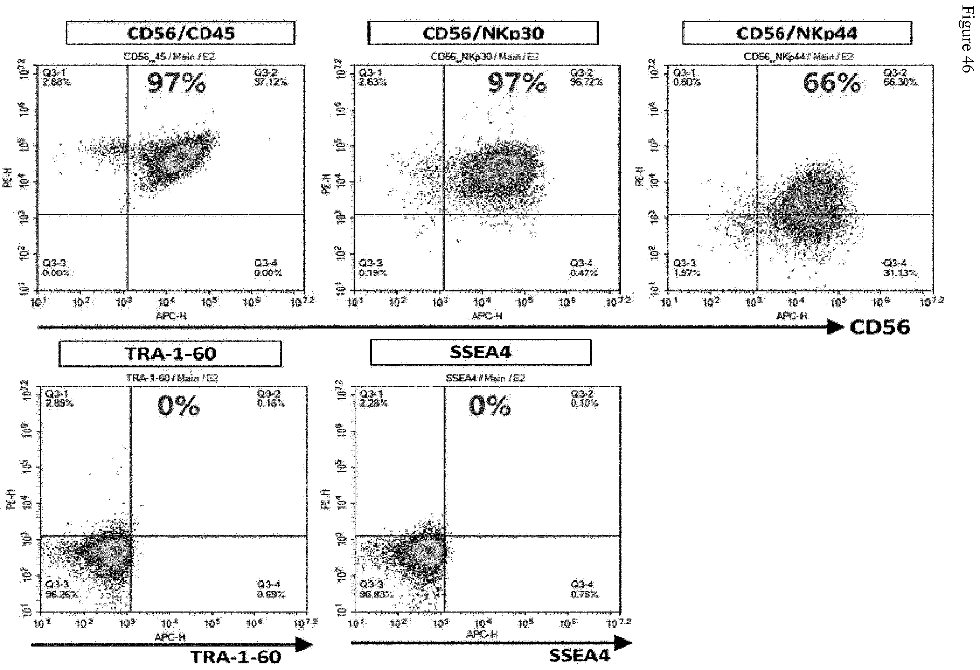

In addition, while the conventional method for producing natural killer cells requires a total differentiation induction period of 48 days, the method for producing natural killer cells according to the present invention in one embodiment of the present invention requires a differentiation induction period of 26 days. As a result of confirming the natural killer cell marker proteins (CD56, CD45, NKp44) and activation proteins (CD56, NKp30) in the EiNK cells induced to differentiate for 26 days, an expression rate of NK marker proteins and activation proteins of 66% or higher was confirmed. On the other hand, it was confirmed that the expression rate of the iPSC marker proteins (TRA-1-60, SSEA4) was 0%, indicating that the method for producing natural killer cells according to the present invention is an efficient method for producing natural killer cells in a short period of time compared to existing methods (Figures 45 and 46).

### Method for culturing stem cells

In one aspect of the present invention, there is provided a method for culturing stem cells, comprising: inoculating stem cells as iPSCs or iPSC spheroids into a cell culture vessel so that 1 to 20 iPSC colonies are formed per 1 cm² of cell culture vessel area.

In this case, the stem cells may be induced pluripotent stem cells (iPSCs). In addition, in this case, the stem cells may be cultured by an adhesion culture method. In this case, the stem cells may be inoculated as a spheroid and subjected to adhesion culture. The number of cells included in the spheroid is the same as described above.

In addition, the number of the inoculated iPSCs or iPSC spheroid may be 0.5 to 20 per 1 cm² of area. The inoculated iPSCs or iPSC spheroids are the same as described above.

In addition, the number of inoculated cells may be calculated in another way. The number of cells present per ml of culture solution may be determined as the number of cells per cm² by using a method for calculating the number of inoculated cells.

The specific culture method is the same as described above.

### Method for differentiating stem cells

In another aspect of the present invention, there is provided a method for differentiating stem cells. Specifically, the method and the total number of stem cells on the start date of differentiation of stem cells are the same as described above. Preferably, the total number of stem cells on the start date of differentiation of stem cells may be 1×10³ to 1×10⁶ cells per 1 cm² of cell culture vessel area. The above stem cells may be differentiated in various ways depending on the medium added. In one embodiment, the stem cells may be differentiated into mesodermal cells. The specific culture method is the same as described above.

### Method for producing natural killer cells from iPSCs

In another aspect of the present invention, there is provided a method for producing natural killer cells from iPSCs, comprising: inoculating induced pluripotent stem cells as iPSCs or iPSC spheroids into a cell culture vessel so that 0.5 to 20 colonies are formed per 1 cm² of cell culture vessel area and performing adhesion culture to produce iPSC colonies; differentiating the iPSC colonies into mesodermal cells to produce mesodermal cells; differentiating the mesodermal cells into hematopoietic stem cells to produce hematopoietic stem cells; and differentiating the hematopoietic stem cells into natural killer cells to produce natural killer cells.

First, the method comprises inoculating induced pluripotent stem cells (iPSCs) as iPSCs or iPSC spheroids into a cell culture vessel so that 0.5 to 20 colonies are formed per 1 cm² of cell culture vessel area and performing adhesion culture to produce iPSC colonies. The number of inoculated cells is the same as described above.

Second, the method comprises differentiating the iPSC colonies into mesodermal cells to produce mesodermal cells.

Third, the method comprises differentiating the mesodermal cells into hematopoietic stem cells to produce hematopoietic stem cells.

Fourth, the method comprises differentiating the hematopoietic stem cells into natural killer cells to produce natural killer cells.

The specific method and condition are the same as described above.

### Method for culturing hematopoietic stem cells by co-culturing with autologous feeder cells

In the present invention, the hematopoietic stem cells may be co-cultured with autologous feeder cells.

As used herein, the term "feeder cell" refers to a cell that is co-cultured with a second type of cell and provides growth factors and nutrients to enable the second type of cell to grow. The above feeder cells may be optionally derived from a species homologous or heterologous to the cells they support. For example, a specific type of human cell including stem cells may be supported by a culture of mouse embryonic fibroblasts and non-apoptotic mouse embryonic fibroblasts. The feeder cell may be a human-derived feeder cell such as a human skin fibroblast or a human embryonic stem cell. In this case, the feeder cells may be inactivated by treatment with an anti-cell division agent such as mitomycin or irradiation. Through the above inactivation, when co-cultured with a second type of cell, the feeder cells produce and secrete cellular metabolites in a state where cell division is stopped, thereby regulating the differentiation and growth of the second type of cell.

In this case, in the present invention, the feeder cell may be an autologous feeder cell.

The specific culture method and condition are the same as described above.

### Mode for Carrying out the Invention

Hereinafter, the present invention will be described in more detail by way of the following examples. However, these examples are intended to exemplify one or more embodiments, and the scope of the present invention is not limited to these examples.

### I. Production of natural killer cells

### Example 1. Method for producing natural killer cells from induced pluripotent stem cells

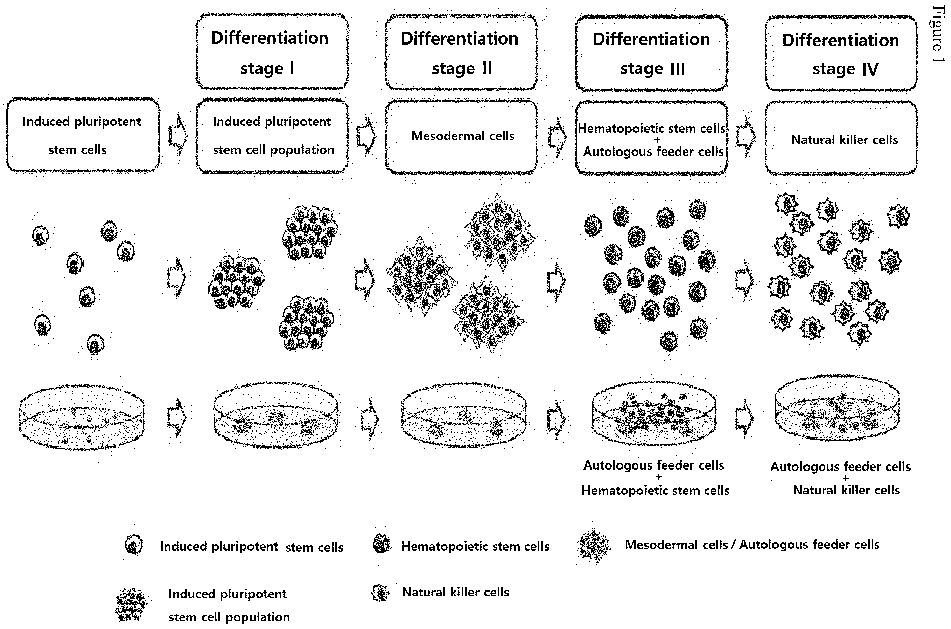

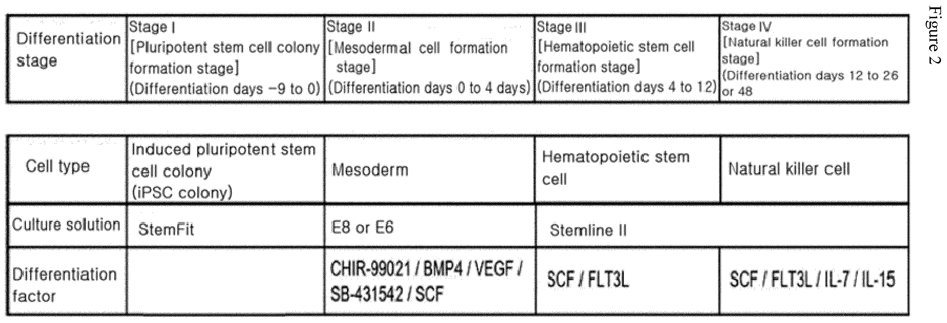

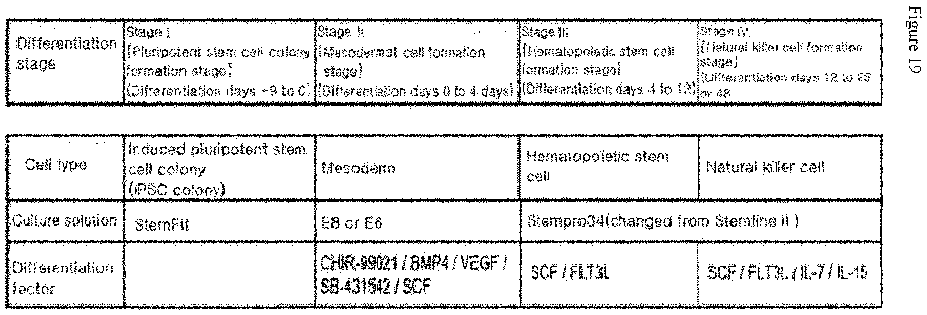

A schematic diagram of a method for obtaining natural killer cells from induced pluripotent stem cells is as shown in Figure 1. In addition, the specific experimental method is as shown in Figures 2 and 19. The method can be broadly divided into the following steps: 1) producing induced pluripotent stem cell colonies (adherent cells) from induced pluripotent stem cells (adherent cells), 2) differentiating from induced pluripotent stem cells (adherent cells) into mesodermal cells (adherent cells), 3) differentiating from mesodermal cells (adherent cells) into hematopoietic stem cells (suspension cells) and autologous feeder cells (adherent cells), and 4) differentiating from hematopoietic stem cells (suspension cells) into natural killer cells while maintaining or removing autologous feeder cells (adherent cells).

### Example 1.1. Culturing induced pluripotent stem cells

The CMC-009 induced pluripotent stem cell (iPSC) cell line used in this example was provided by the National Stem Cell Bank of the National Center for Stem Cell and Regenerative Medicine. The cell line was cultured in a cell culture vessel applied with the extracellular matrix iMatrix-511 (Nippi, 892012) under StemFit Basic04 culture medium (Ajinomoto, AJBASIC04).

### Example 1.2. Formation of homogeneous induced pluripotent stem cell colonies from induced pluripotent stem cells

### Example 1.2.1. Formation of homogeneous induced pluripotent stem cell colonies from single induced pluripotent stem cells

As a preliminary step for differentiation from human induced pluripotent stem cells into natural killer cells, the formation of induced pluripotent stem cell colonies (iPSC colonies) derived from single cell induced pluripotent stem cells was induced.

Specifically, 1 µl of iMatrix-511 per 1 cm² of the cell culture vessel was diluted in 150 µl of DPBS (Cytiva, SH3002802) and applied to the cell culture vessel for at least 1 hour, and then replaced with the StemFit Basic 04 culture medium containing 10 µM Y-27632 to prepare a cell culture vessel to which single cell iPSCs will adhere. The iPSCs were treated with TypLE (Gibco, 12604013) to separate them into single cells, and then 0.5 to 20 single cell iPSCs were inoculated per 1 cm² of the culture vessel area into the prepared cell culture vessel.

The next day, the cell culture solution was replaced with fresh StemFit Basic 04 and cultured for an additional 9 to 12 days. When the diameter of the induced pluripotent stem cell colony (iPSC colony) was between 300 and 1,600 µm (average diameter of 300 to 700 µm), the stem cell culture solution was removed and differentiation of natural killer cells was initiated. During the process of producing induced pluripotent stem cell colonies, the cell culture medium was replaced daily.

### Example 1.2.2. Formation of homogeneous induced pluripotent stem cell colonies from induced pluripotent stem cells in form of spheroid

As a preliminary step for differentiation from human induced pluripotent stem cells into natural killer cells, the formation of induced pluripotent stem cell colonies (iPSC colonies) was induced by inoculating induced pluripotent stem cells in the form of spheroid.

Specifically, 1 µl of iMatrix-511 per 1 cm² of the cell culture vessel was diluted in 150 µl of DPBS (Cytiva, SH3002802) and applied to the cell culture vessel for at least 1 hour, and then replaced with the StemFit Basic 04 culture solution containing 10 µm Y-27632 to prepare a cell culture vessel to which iPSC spheroids will adhere. The iPSCs were prepared in the form of spheroids by culturing single iPSCs. At this time, one spheroid contained about 1 to about 600 iPSCs. Thereafter, 0.5 to 20 iPSC spheroids were inoculated per 1 cm² of the culture vessel area into the prepared cell culture vessel.

The next day, the cell culture solution was replaced with fresh StemFit Basic 04 and cultured for an additional 2 to 5 days. When the diameter of the induced pluripotent stem cell colony (iPSC colony) was between 300 and 1,600 µm (average diameter of 300 to 700 µm), the stem cell culture solution was removed and differentiation of natural killer cells was initiated. During the process of producing induced pluripotent stem cell colonies, the cell culture solution was replaced daily.

### Example 1.3. Production of natural killer cells from induced pluripotent stem cell colonies

Specifically, when the diameter of the induced pluripotent stem cell colony (iPSC colony) produced in Example 1.2 above was between 300 and 1,600 µm (average diameter of 300 to 700 µm), the stem cell culture medium was removed, and the cells were cultured for 2 days with serum-free stem cell culture medium Essential 8^{™} (Gibco, 1517001) additionally containing 2 µm CHIR-99021 (Peprotech, 2520691), 80 ng/ml BMP4 (R&D Systems, 314-BP/CF) and 80 ng/ml VEGF (R&D Systems, 293-VE/CF). After 2 days, the above culture medium was removed, and the cells were cultured in serum-free cell culture medium Essential 6^{™} (Gibco, 1516401) additionally containing 2 µm SB-431542 (Peprotech, 3014193), 50 ng/ml SCF (R&D Systems, 255-SC/CF) and 80 ng/ml VEGF to induce the differentiation into mesodermal cells.

After 2 days, the above culture medium was removed, and the cells were cultured in serum-free hematopoietic stem cell culture medium Stemline II (Sigma, S0192) additionally containing 50 ng/ml SCF and 50 ng/ml FLT3L (R&D Systems, 308-FK/CF) to induce the differentiation into hematopoietic stem cells. After 8 days, the above culture medium was removed and replaced with Stemline II culture medium additionally containing 50 ng/ml SCF, 50 ng/ml FLT3L, IL-7 (R&D Systems, 207-IL-200/CF) and IL-15 (R&D Systems, 247-ILB-500/CF), and the cells were cultured for 36 days to finally induce the differentiation into natural killer cells. At this time, all culture mediums used for differentiation were replaced by half every 4 days.

### Example 1.4. Production of natural killer cells from iPSC colonies under conditions where autologous feeder cells are excluded

Specifically, when the diameter of the induced pluripotent stem cell colony (iPSC colony) produced in Example 1.2 above was between 700 and 1,200 µm, the stem cell culture medium was removed, and the cells were cultured for 2 days in serum-free stem cell culture medium Essential 8^{™} additionally containing 2 µm CHIR-99021, 80 ng/ml BMP4 and 80 ng/ml VEGF. After 2 days, the above culture medium was removed, and the cells were cultured in serum-free cell culture medium Essential 6^{™} additionally containing 2 µm SB-431542, 50 ng/ml SCF and 80 ng/ml VEGF to induce differentiation into mesodermal cells.

After 2 days, the above culture medium was removed, and the cells were cultured in serum-free hematopoietic stem cell culture medium Stemline II additionally containing 50 ng/ml SCF and 50 ng/ml FLT3L to induce the differentiation into hematopoietic stem cells. On the 12th day of differentiation, only the suspension cells excluding the autologous feeder cells in the cell culture vessel were collected, transferred to a new cell culture vessel, replaced with Stemline II culture medium additionally containing 50 ng/ml SCF, 50 ng/ml FLT3L, IL-7 and IL-15, and cultured for 36 days to finally induce the differentiation into natural killer cells. At this time, all culture mediums used for differentiation were replaced by half every 4 days.

### Example 1.5. Morphological verification of natural killer cells differentiated from iPSC colonies by differentiation day

During the differentiation from iPSCs into NK cells by the methods of Examples 1.1 to 1.4 above, the morphological changes of the cells undergoing differentiation were observed using an optical microscope. Specifically, it was confirmed that at each differentiation point, the morphology of the cells changed from pluripotent stem cells to mesodermal cells, hematopoietic stem cells, and then natural killer cells, depending on the differentiation stage, using an optical microscope (Olympus, Olympus-CKX53).

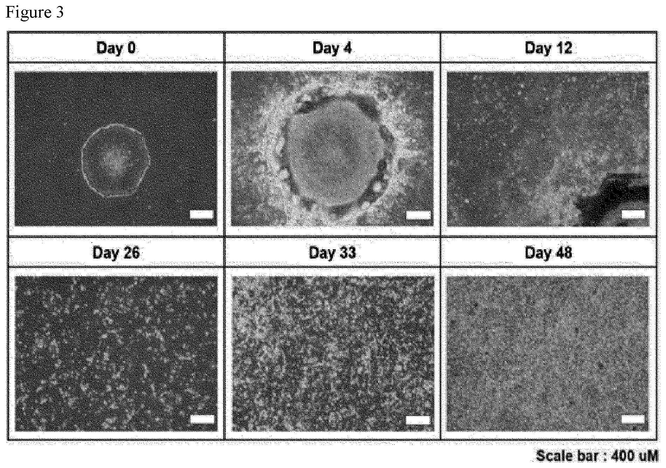

The morphological changes in cells at each differentiation stage were confirmed using an optical microscope (Figure 3). In Figure 3, the photograph on Day 0 was taken of an induced pluripotent stem cell colony (adherent cell) produced from induced pluripotent stem cells (adherent cells). At this time, the size of the colony in the photograph was between 700 µm and 1,200 µm, and the colony of that size was differentiated into mesodermal cells. In addition, the photograph on Day 4 was taken of mesodermal cells (adherent cells) differentiated from an induced pluripotent stem cell colony. In addition, the photograph on Day 12 was taken of the feeder cells (the part where cells are adhered and clumped together) and the hematopoietic stem cells (suspension cells that exist as single cells) differentiated from mesodermal cells (adherent cells). In addition, the photographs on Day 26 to Day 33 were taken of the natural killer cells that were differentiating from hematopoietic stem cells (autologous supporting cells are excluded in the photograph). Finally, the photograph on Day 48 was taken of natural killer cells (suspension cells) derived from induced pluripotent stem cells.

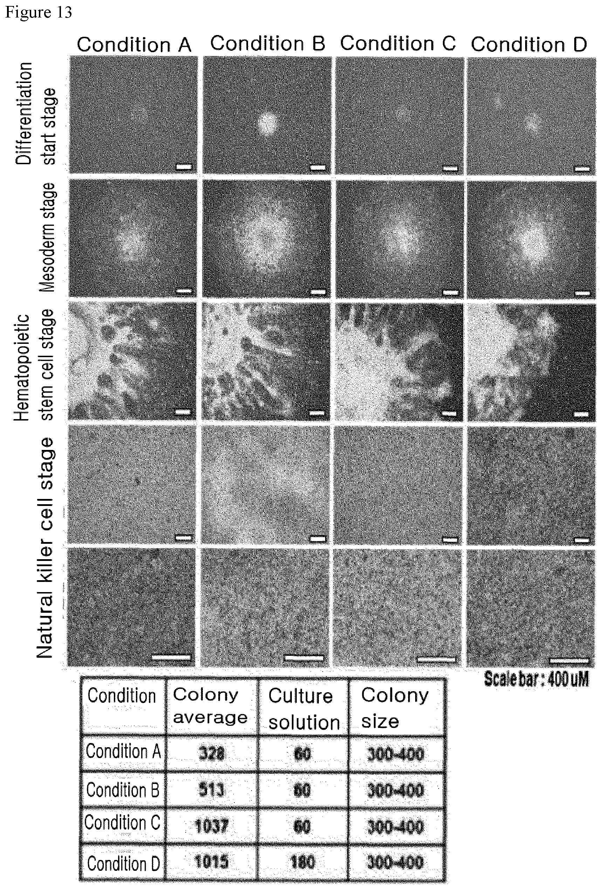

In addition, after changing the culture conditions, the results obtained by observing the morphological changes of the differentiated cells at each differentiation stage for each condition (A to D) using an optical microscope were confirmed (Figure 13). At this time, the photographs were taken under a microscope at each differentiation stage, and the photographs were taken from differentiation day 0 to the final differentiation day into the natural killer cells.

### Example 1.6. Confirmation of production of natural killer cells according to the presence or absence of feeder cells derived from iPSCs and confirmation of properties of produced natural killer cells

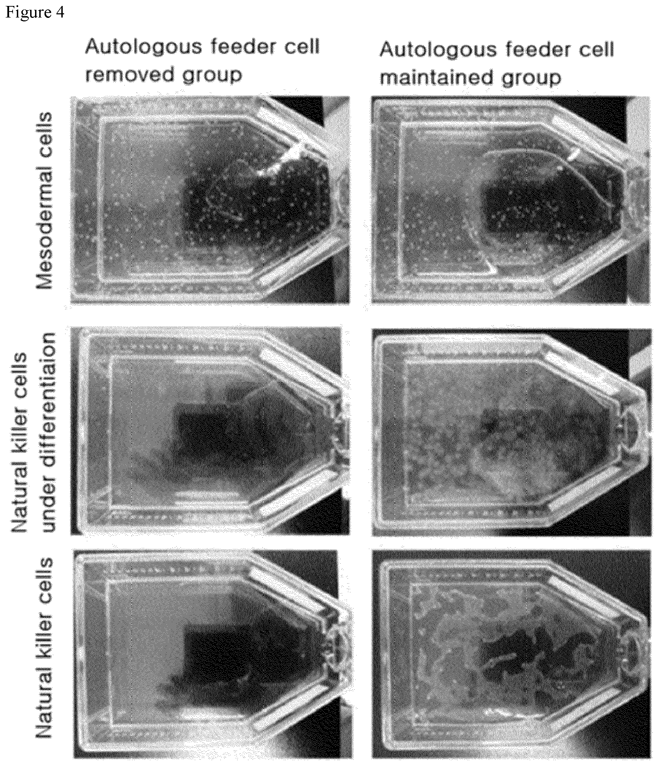

During the methods of Examples 1.1 to 1.4 above, the production of mesodermal cells and natural killer cells was confirmed depending on the presence or absence of feeder cells derived from iPSCs (Figure 4).

The top diagram shows a photograph of the entire culture flask of mesodermal cells derived from induced pluripotent stem cell colonies. At this time, since the size of the induced pluripotent stem cell colonies at the start of differentiation was impossible to capture with a general camera, the entire culture vessel could only be captured at the mesodermal cell differentiation stage that had been differentiated for 3 to 4 days.

In addition, the middle diagram is a photograph of the entire flask after the differentiation into natural killer cells was performed (differentiation day 25) in the group in which only autologous feeder cells were removed and in the group in which autologous feeder cells were maintained after the differentiation into hematopoietic stem cells was completed (differentiation day 12). Finally, the bottom diagram shows a photograph of the completion stage of autologous feeder cell differentiation.

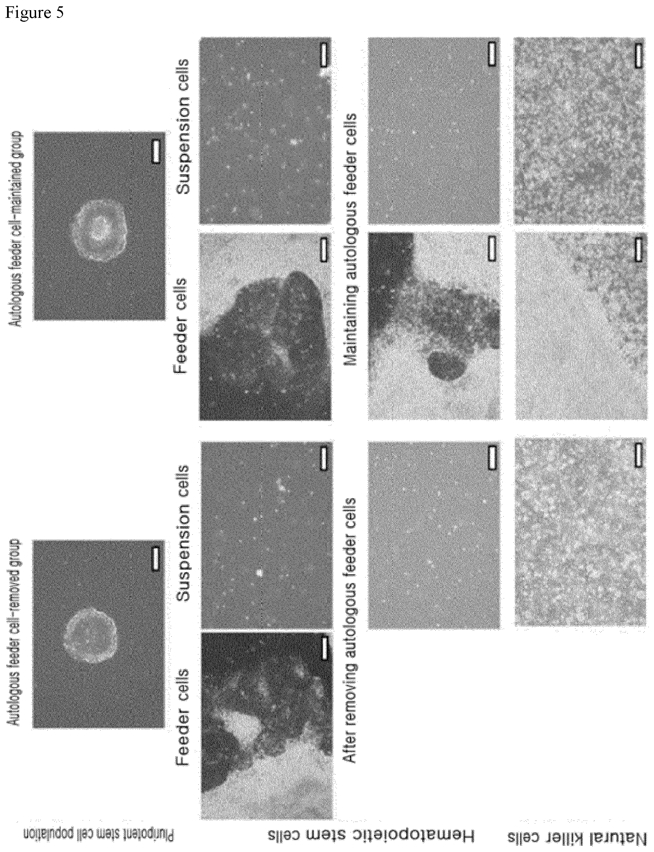

In addition, hematopoietic stem cells and natural killer cells were photographed according to the presence or absence of feeder cells (Figure 5). Specifically, photographs were taken from differentiation day 0 to day 45, the final differentiation day into natural killer cells, and the group in which autologous feeder cells were removed on differentiation day 12 (the stage at which hematopoietic stem cell differentiation is complete) and the group in which autologous feeder cells were maintained were photographed and compared.

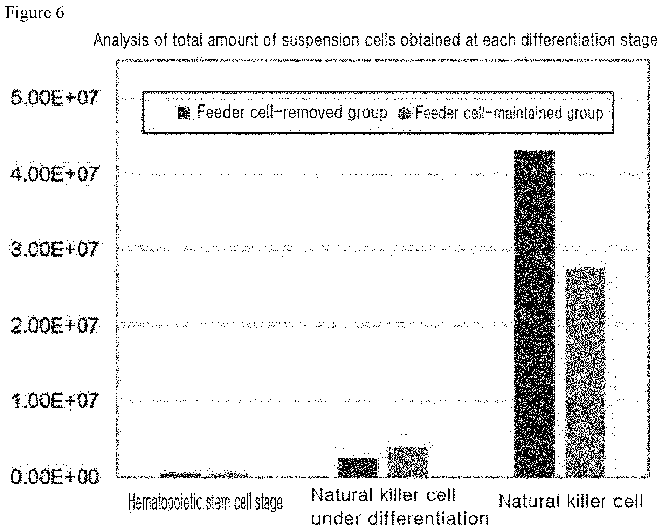

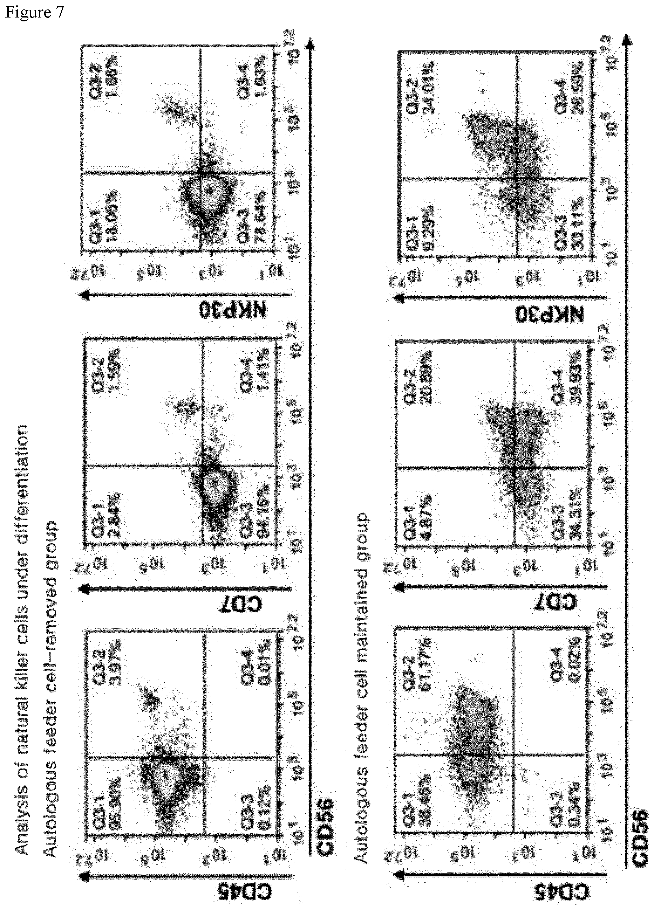

In addition, the total amount of suspension cells obtained and the expression rate of natural killer cell surface marker proteins (markers) at each differentiation stage in the group in which autologous feeder cells were maintained and in the group in which autologous feeder cells were removed were confirmed by flow cytometry (Figures 6 and 7).

### Example 1.7. Analysis of natural killer cell yield according to the number of iPSCs inoculated

In order to confirm the proliferation ability according to the number of iPSCs inoculated, the iPSCs were cultured by the methods of Examples 1.1 to 1.4 above with different numbers of iPSCs in the culture medium. The number of iPSCs at the time of inoculation can be confirmed through the number of colonies of the cultured iPSCs.

Specifically, the number of cells was measured using the following method. First, the medium in the T-150 flask was removed and then washed with 1 mL of DPBS. 10 ml of TrypLE Express was added and reacted in a 37 °C incubator for 15 minutes. The colonies attached to the scraper were collected and obtained in a 50 ml tube. At this time, the colonies were collected after thoroughly checking that there were no remaining colonies. After washing with 15 ml of complete medium so that all cells remaining in the flask could be collected, they were collected in a tube. Step 4 was repeated once more to make a total of 40 ml.

Centrifugation was performed at 300g for 5 minutes. The supernatant was removed, and then the cells were released by tapping, and then the cells were resuspended by pipetting with 1 mL of complete medium. 9 ml of complete medium was added to make a total of 10 ml. Thereafter, the number of cells was counted.

Specifically, iPSCs were proliferated under the conditions described in Table 1 below to produce natural killer cells.

**[Table 1]**

| Condition | Average number of colonies | Culture medium | Colony size (µm) |
|---|---|---|---|
| Condition 1 | 173 | 60 | 700-1200 |
| Condition 2 | 565 | 60 | 700-1200 |
| Condition 3 | 1089 | 60 | 700-1200 |
| Condition 4 | 1637 | 60 | 700-1200 |
| Condition 5 | 1156 | 120 | 700-1200 |
| Condition 6 | 1751 | 180 | 700-1200 |

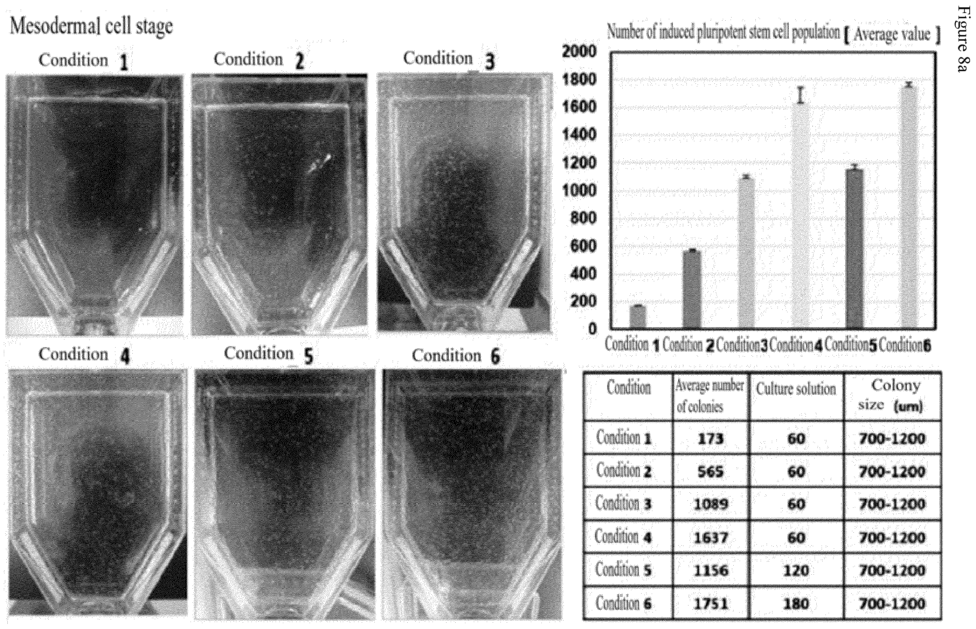

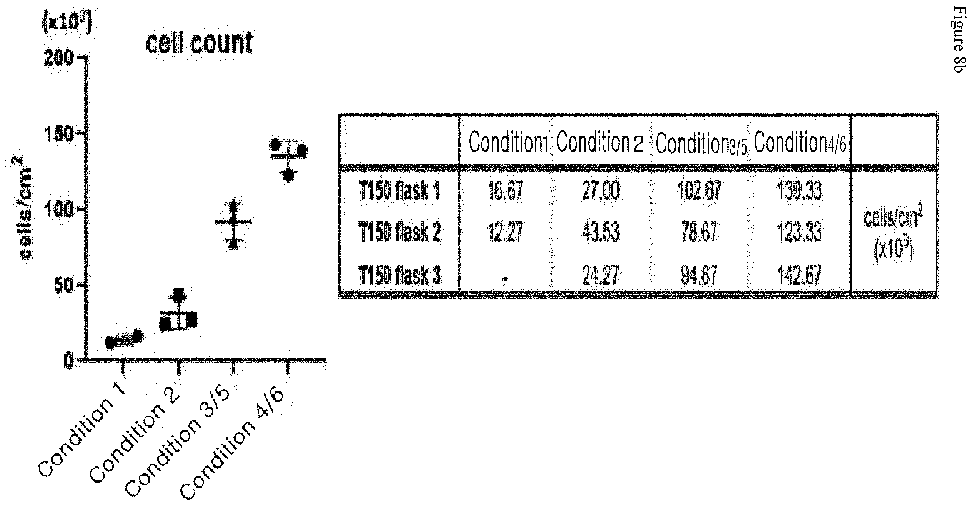

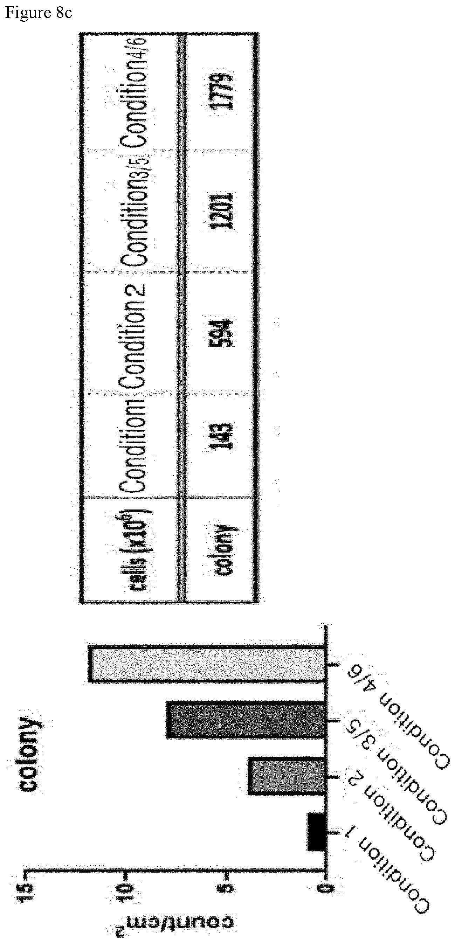

At this time, Figure 8a shows the results obtained by analyzing the number of adhered iPSC colonies in each of the above conditions. Specifically, induced pluripotent stem cell colonies were formed differentially for each condition, and then the entire culture vessel was photographed at the mesodermal cell stage (differentiation day 3 to 4). The number of iPSC colonies and the size of the colonies were measured. As a result, the induced pluripotent stem cell colonies on differentiation day 0 could not be photographed at the corresponding magnification, and there was no difference in the number of colonies on differentiation day 0 and differentiation day 4. However, it was confirmed that the size of the iPSC colonies on day 4 was larger. The measurement results of the above Figure 8a were analyzed and presented as a numerical value for the total number of cells / adhered vessel area (cm²) (Figures 8b to 8c).

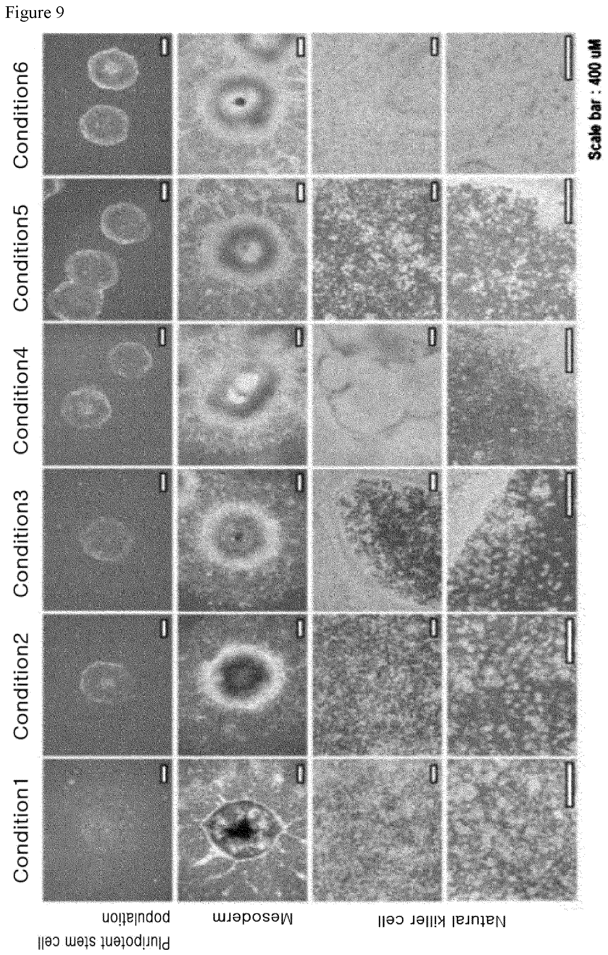

In addition, the morphological changes of the cells differentiated at each stage for each condition 1 to condition 6 were confirmed using an optical microscope (Figure 9).

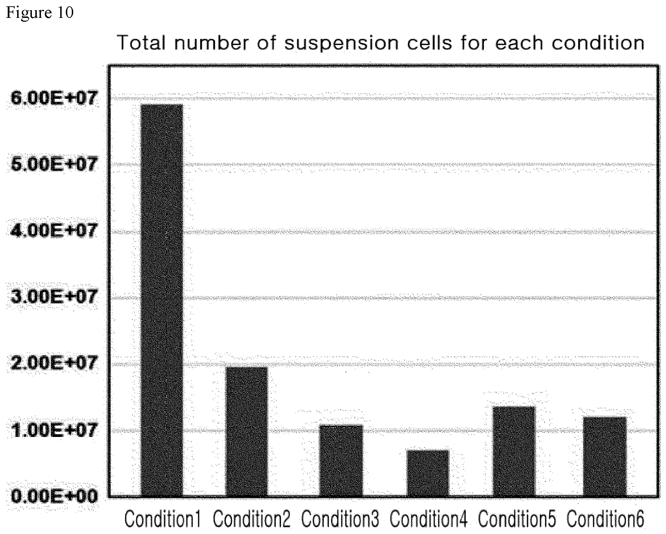

Moreover, the amount of differentiated natural killer cells (suspension cells, Day 35) for each condition was confirmed (Figure 10), and the yield of suspension cells, which are differentiated natural killer cells, was confirmed for each condition based on the suspension cell production amount of condition 2 (Table 2). In addition, the results were analyzed based on the results of condition 2 as the amount of suspension cells for each condition as "Early differentiation pluripotent cell colonies/culture medium volume" (Table 3) and "Early differentiation pluripotent cell colonies/differentiation area" (Table 4).

**[Table 2]**

| | Day 35 | | | | |
|---|---|---|---|---|---|
| Group | Average number of colonies | Colony ratio* | Expected production amount | Actual production amount | Actual expected |
| Condition 1 | 173 | 1/3.26 times | 5.88E+06 | 5.92E+07 | 1000.68% |
| Condition 2 | 565 | 1 | 1.97E+07 | 1.97E+07 | 100% |
| Condition 3 | 1089 | 1.92 times | 3.78E+07 | 1.09E+07 | 28.83% |
| Condition 4 | 1637 | 2.89 times | 5.69E+07 | 7.09E+06 | 12.46% |
| Condition 5 | 1156 | 2.04 times | 4.01E+07 | 1.37E+07 | 34.16% |
| Condition 6 | 1751 | 3.09 times | 6.08E+07 | 1.21E+07 | 19.90% |

**[Table 3]**

| | Culture medium | Day 35 | | | |
|---|---|---|---|---|---|
| Group | Media | Colony | Colony/ml | Production amount | Note |
| Condition 1 | 60 | 173 | 2.8 | 5.92E+07 | Not the production ratio according to the absolute amount of raw material input |
| Condition 2 | 60 | 565 | 9.4 | 1.97E+07 | |
| Condition 3 | 60 | 1089 | 18.15 | 1.09E+07 | |
| Condition 4 | 60 | 1637 | 27.2 | 7.09E+06 | |
| Condition 5 | 120 | 1156 | 9.6 | 1.37E+07 | |
| Condition 6 | 180 | 1751 | 9.7 | 1.21E+07 | |

**[Table 4]**

| | Area | Day 35 | | | |
|---|---|---|---|---|---|
| Group | T-150 | Colony | Colony/cm² | Production amount | Note |
| Condition 1 | 150 cm2 | 173 | 1.15 | 5.92E+07 | Not the production ratio according to the absolute amount of raw material input |
| Condition 2 | 150 cm2 | 565 | 3.76 | 1.97E+07 | |
| Condition 3 | 150 cm2 | 1089 | 7.26 | 1.09E+07 | |
| Condition 4 | 150 cm2 | 1637 | 10.91 | 7.09E+06 | |
| Condition 5 | 150 cm2 | 1156 | 7.7 | 1.37E+07 | |
| Condition 6 | 150 cm2 | 1751 | 11.67 | 1.21E+07 | |

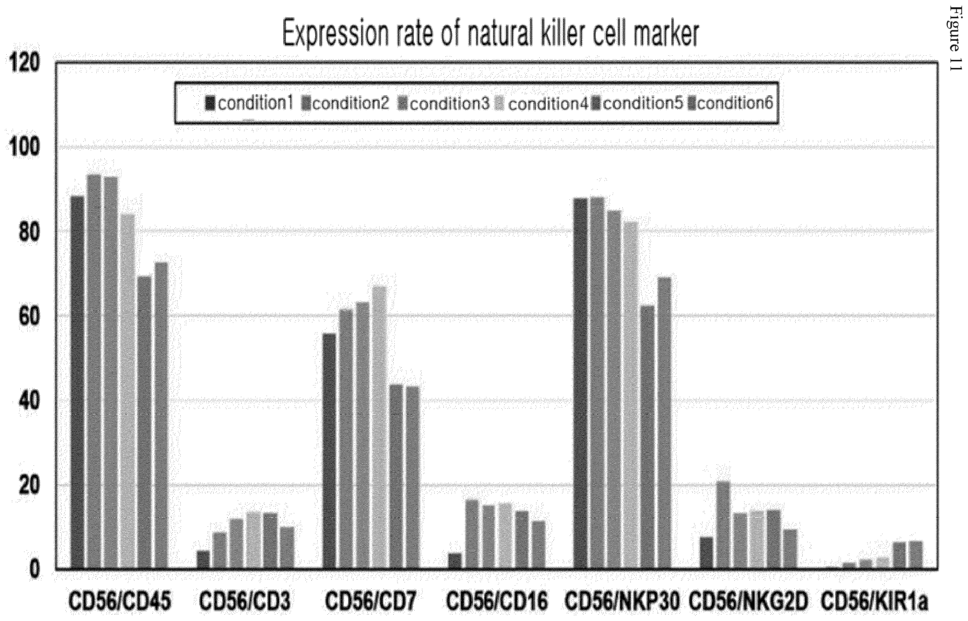

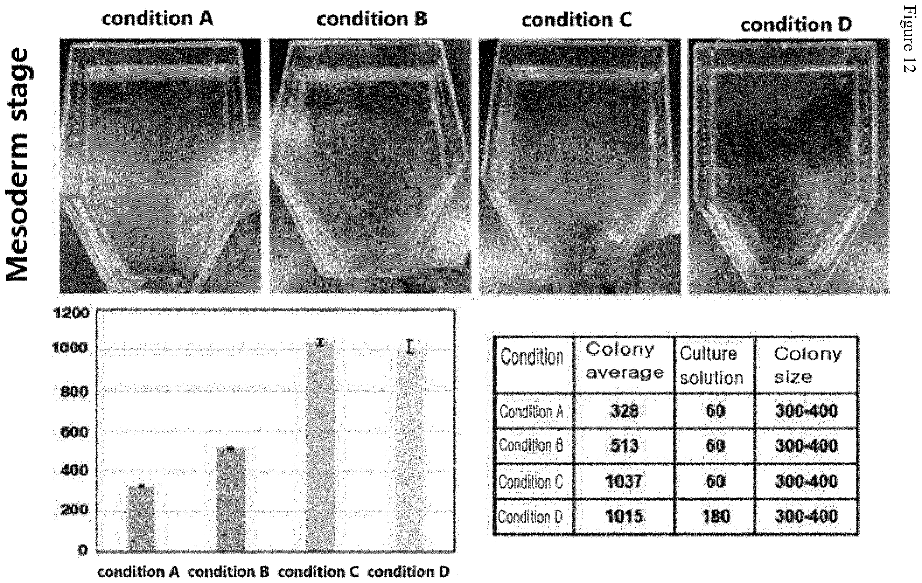

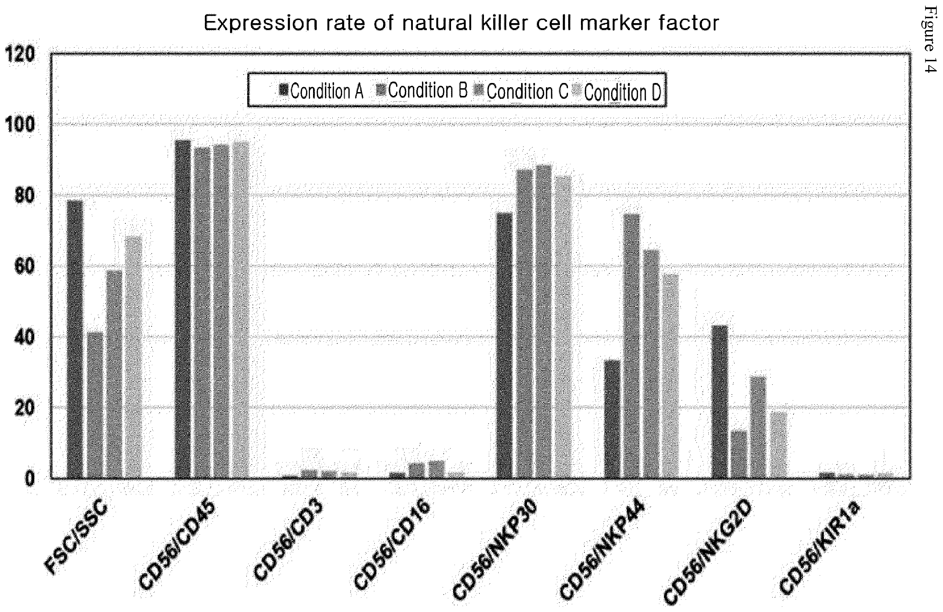

In order to confirm the characteristics of iPSC-derived natural killer cells induced to differentiate under each of the above condition 1 to condition 6, the expression rate of natural killer cell surface marker proteins (markers) was confirmed by flow cytometry, and the results are shown in Figure 11. When inducing differentiation, the total number of natural killer cells (suspension cells) obtained under the above conditions was confirmed. The smaller iPSC colonies were formed compared to the above condition 1 to condition 6, and then differentiation into mesodermal cells was induced. As a result, as shown in Figure 12, Figure 13, and Table 5, it was confirmed that differentiation into mesodermal cells was possible even in iPSC colonies of 300 µm to 400 µm in size (Figures 12 to 14 and Table 5).

**[Table 5]**

| Condition | Average number of colonies | Culture medium | Colony size |
|---|---|---|---|
| condition A | 328 | 60 | 300-400 |
| condition B | 513 | 60 | 300-400 |
| condition C | 1037 | 60 | 300-400 |
| condition D | 1015 | 180 | 300-400 |

### Example 1.8. Reproducibility experiment on production of natural killer cells derived from induced pluripotent stem cell colonies

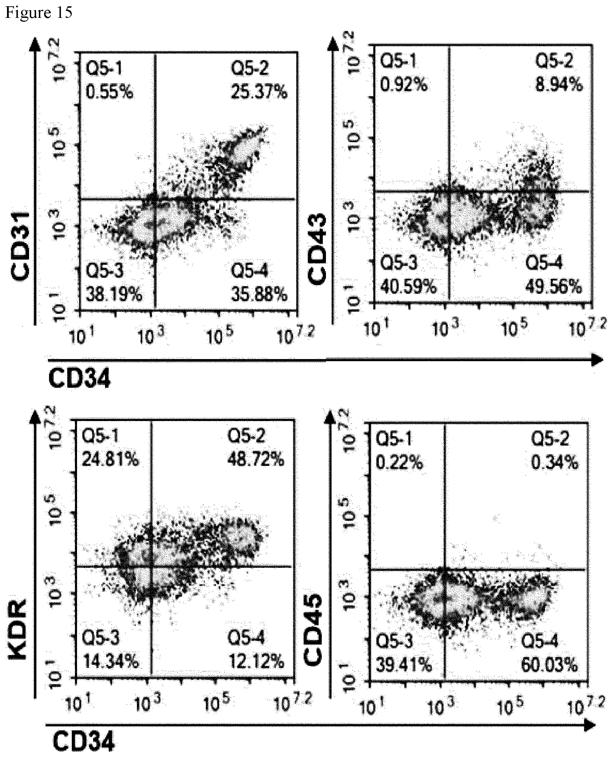

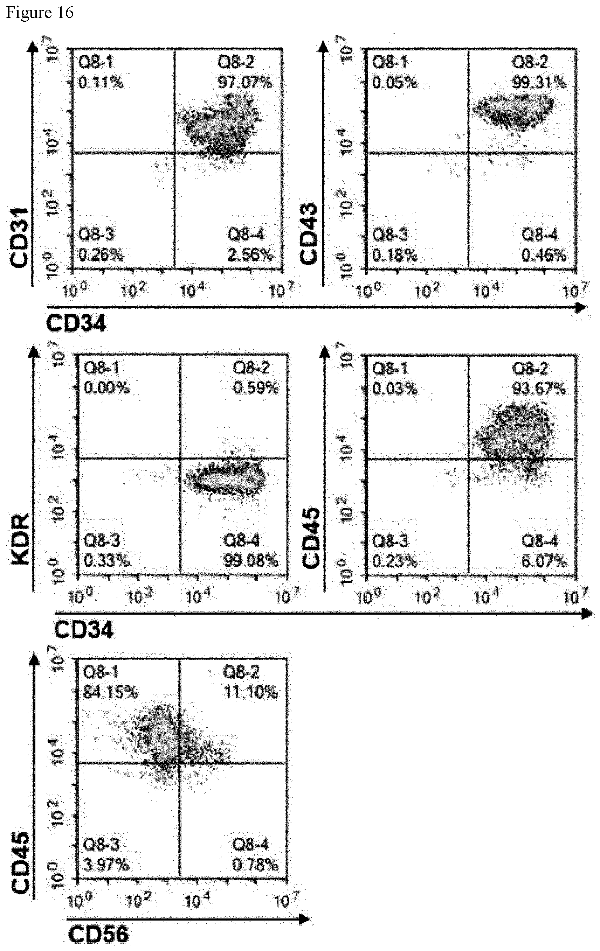

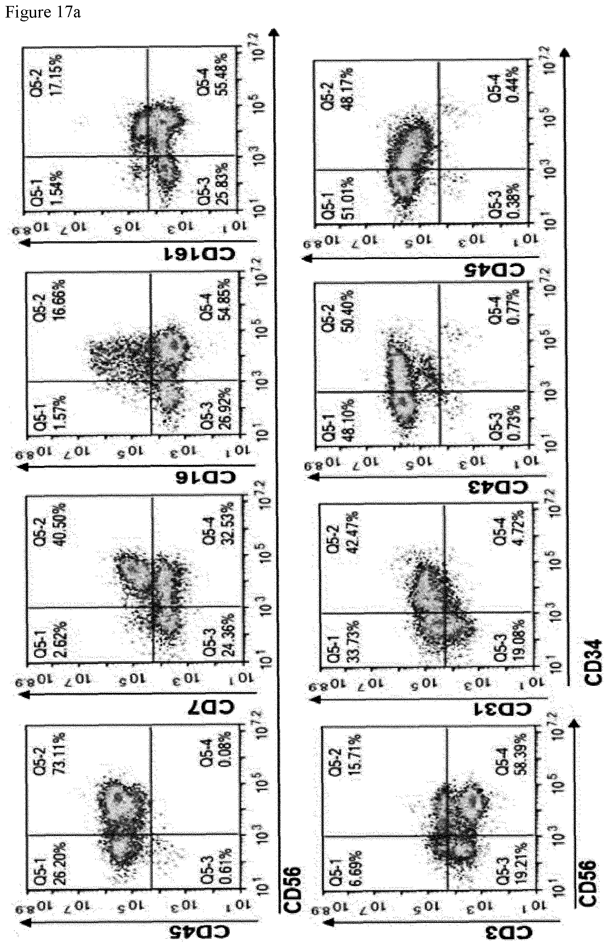

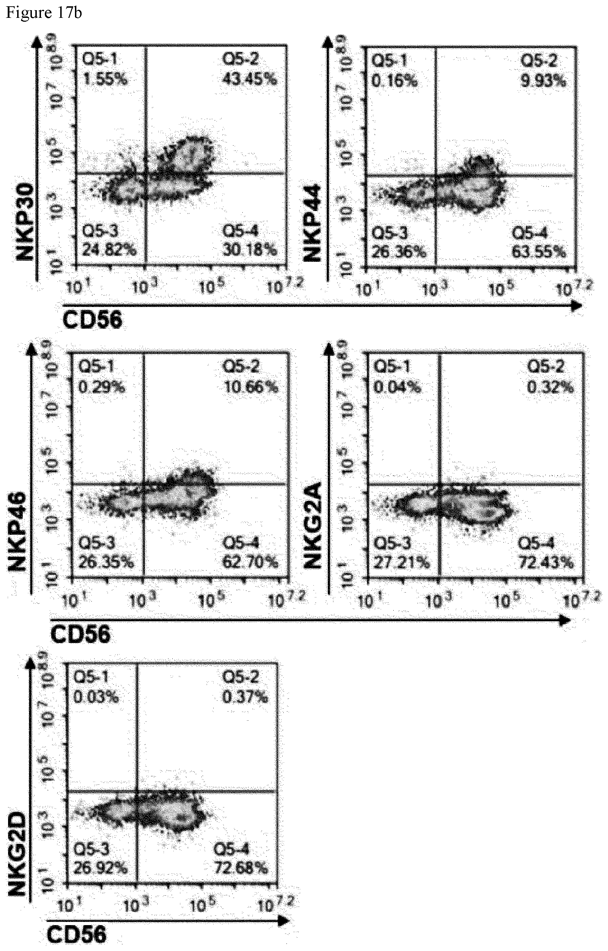

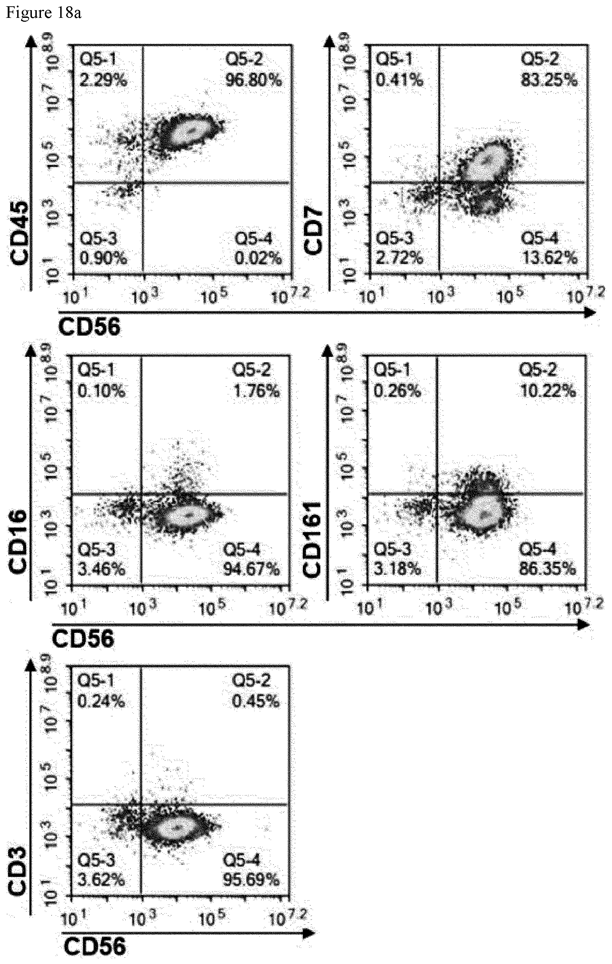

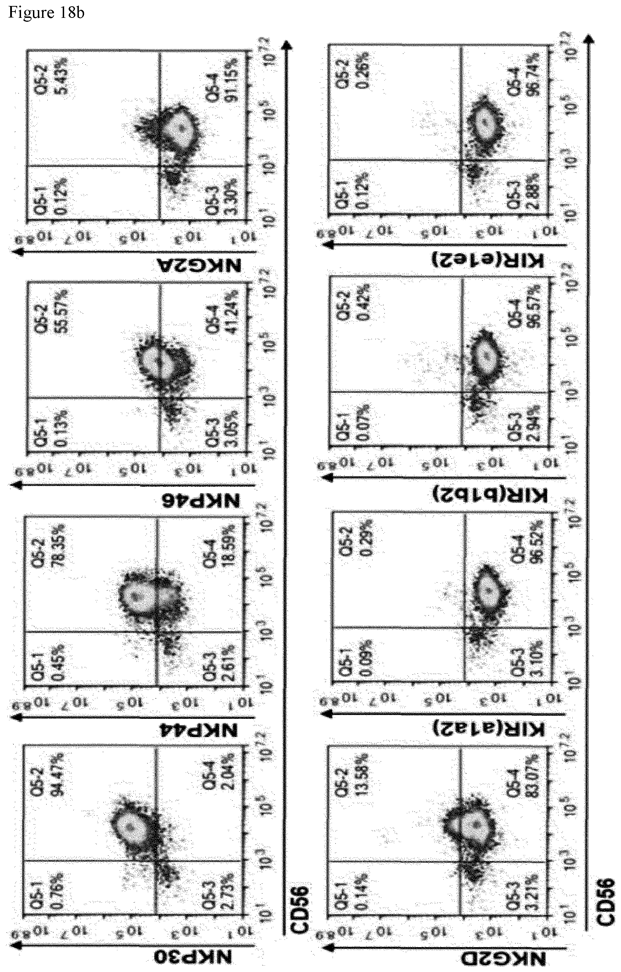

Differentiation from induced pluripotent stem cells into natural killer cells was repeatedly performed under the same conditions as condition 2 above, and the experimental results were summarized (Figures 15 to 18).

### Example 1.9. Production of natural killer cells derived from induced pluripotent stem cell colonies according to culture medium changes

Natural killer cells were produced from induced pluripotent stem cells by the differentiation methods of Examples 1.1 to 1.4 above. At this time, natural killer cells were produced in the same manner, except that Stempro34 media was used (Figure 19).

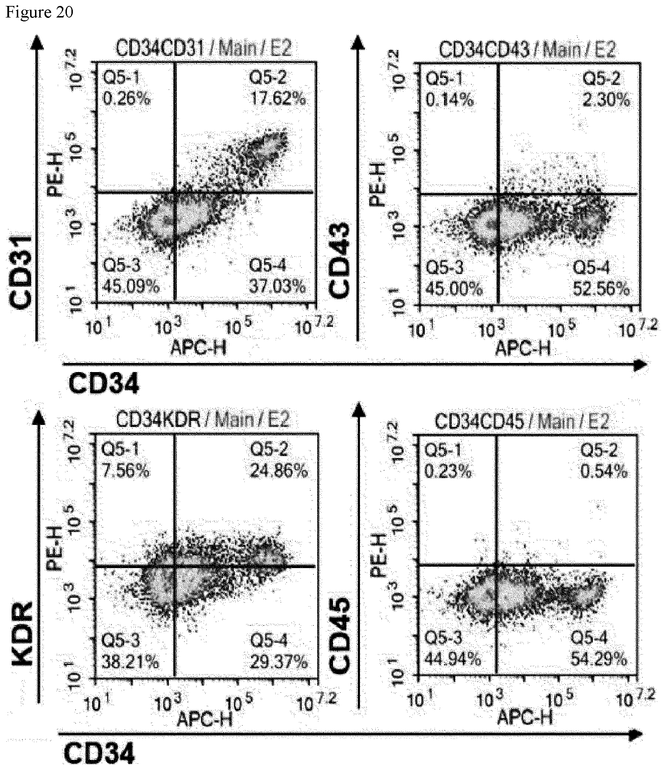

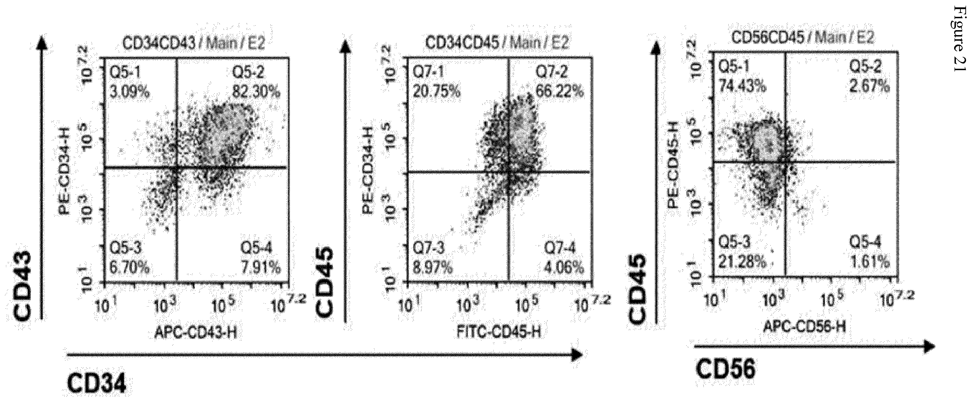

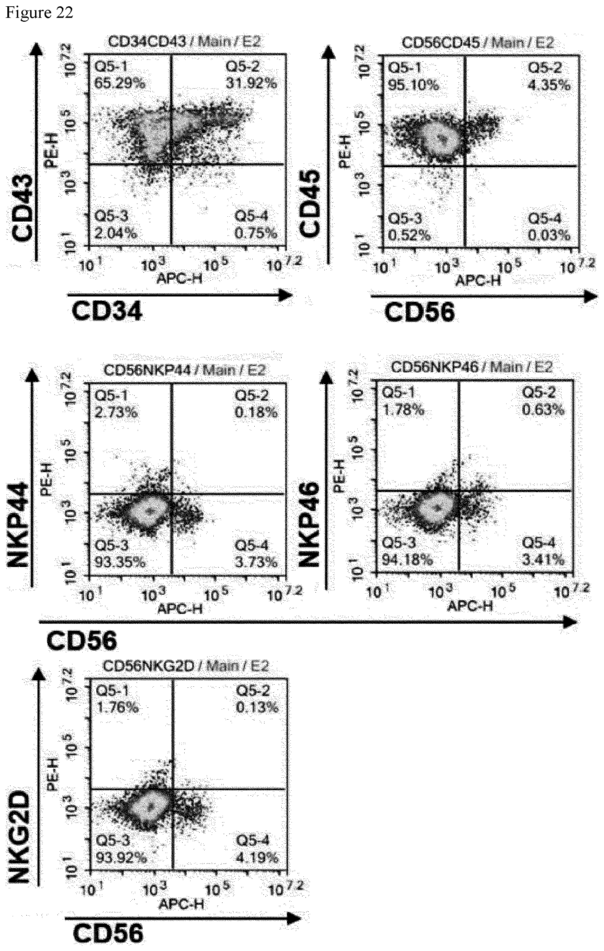

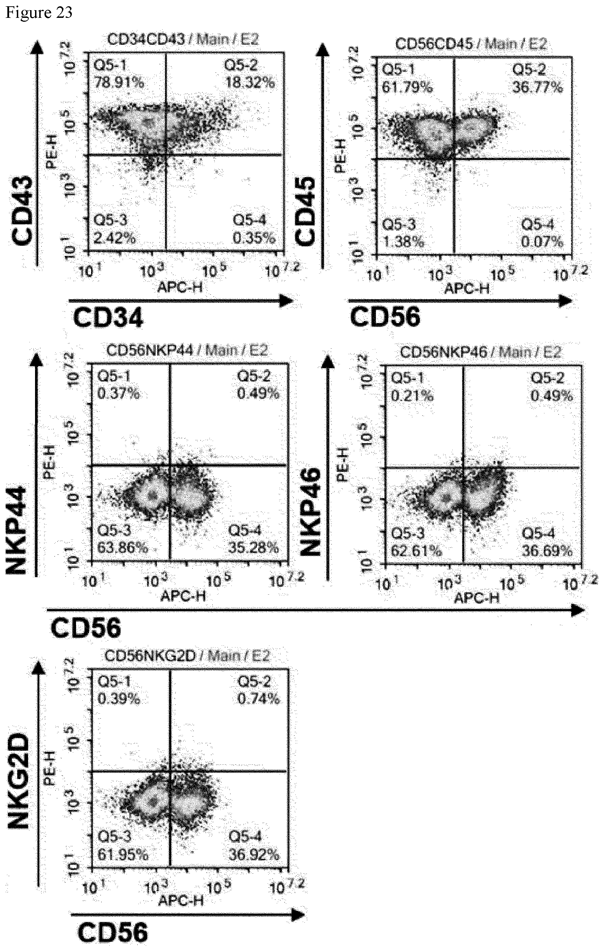

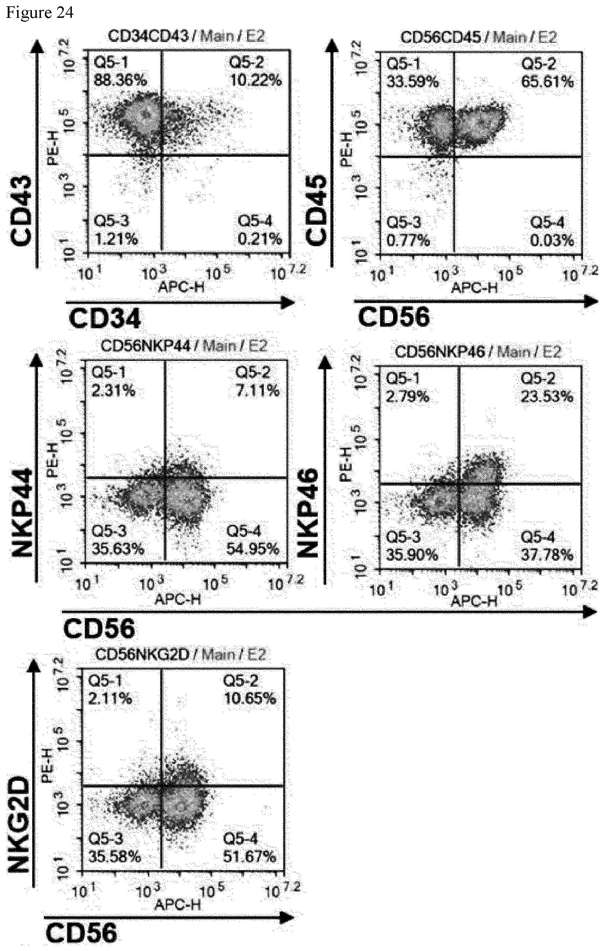

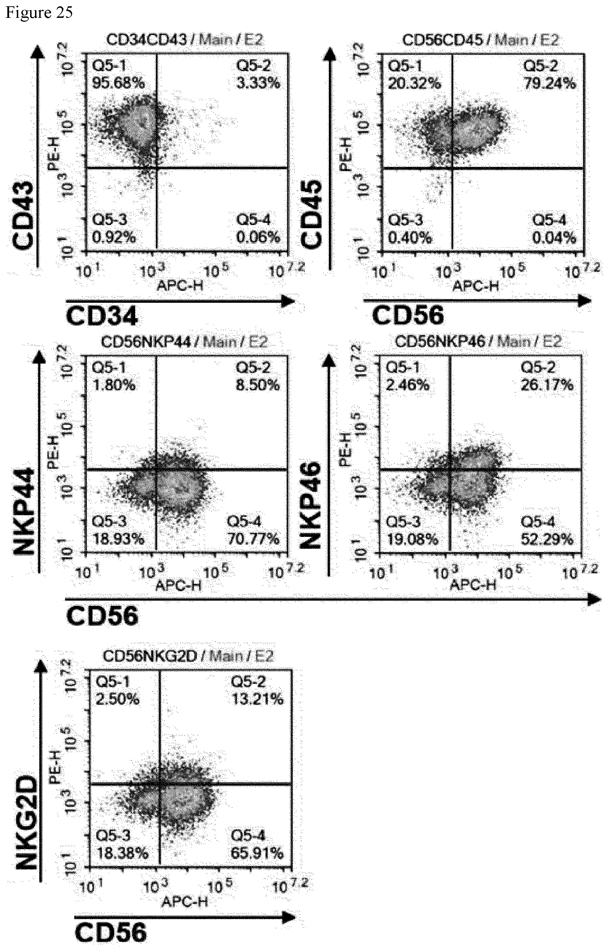

When inducing differentiation from iPSCs into natural killer cells by the above method, the surface marker proteins (markers) of natural killer cells were identified in the cells obtained at each period by flow cytometry, and the results are shown (Figures 20 to 25).

### Example 1.10. Mass proliferation of natural killer cells derived from induced pluripotent stem cells

The natural killer cells obtained in the same manner as in Examples 1.1 to 1.4 above were proliferated by a mass culture process. The above mass culture process was performed once every 2 to 3 days. Specifically, it was performed on days 0, 3, 5, 7, 10, 12, 14, 17, 19, and 21, and the cells were finally harvested on day 21.

The culture medium used in the mass culture process was used by adding additional ingredients to the basic medium as described in Table 6. Specifically, the basic medium prepared in step 1 consists of DMEM, Ham's F 12 Nutrient Mix, human AB serum, Penicillin-Streptomycin, and L-glutamine. Thereafter, cytokines and factors selected from β-mercaptoethanol, sodium selenite, ethanolamine, L-ascorbic acid, SCF, IL-7, IL-15, FLT-3 Ligand, and IL-2 were added to the basic medium and used. Hereinafter, the 'culture medium' was referred to as 'culture solution'.

**[Table 6]**

| Culture medium for mass proliferation of natural killer cells derived from induced pluripotent stem cells | |
|---|---|
| Basic medium | DMEM/(Gibco, 10566-016) |
| | Ham's F12 Nutrient Mix/(Gibco, 31765035) |
| | L-Glutamine (1%)/(Gibco, 25030081) |
| | human AB serum (2 to 20%)/(Gemcell, 100-512) |
| | Penicillin-Streptomycin (1%)/(Gibco, 15140-122) |
| | Sodium selenite (0.1 to 50 ng/ml)/(Sigma, S5261) |
| | Ehtanolamine (0.1 to 250 µm)/(Sigma, E0135) |
| | β-mercaptoethanol (0.1 to 5 µm)/(Gibco, 21985-023) |
| | L-ascorbic acid (1 to 250 ng/ml)/(Sigma, A4544) |
| Additional ingredients | IL-2 (1 to 1000 IU/ml)/(BL&H) |
| | IL-7 (0.2 to 250 ng/ml)/ (R&D systems, 207-IL-200/CF) |
| | SCF (0.2 to 250 ng/ml)/ (R&D systems, 255-SC/CF) |
| | IL-15 (0.1 to 100 ng/ml)/ (R&D systems, 247-ILB-500/CF) |
| | FLT-3 Ligand (0.1 to 100 ng/ml)/ (R&D systems, 308-FK/CF) |

In the mass culture process, first, on the day when differentiation was completed (day 0), the first irradiated feeder cell stimulation was performed on differentiated natural killer cells. Specifically, natural killer cells and irradiated feeder cells were co-cultured in a 6-well plate containing 2 ml of culture medium at a ratio of 1:2. On the third day of culture, half of the culture solution, 1 ml, was replaced. At this time, the cells in the replaced culture solution were collected and cultured again. On the fifth day of culture, half of the culture solution, 1 ml, was replaced, and then 3 ml of new culture solution was added to increase the volume to 4 ml. In addition, when all processes were performed up to the 14th day of culture in the subsequent culture process, half of the culture solution was replaced, the cells were collected, and the volume of culture solution was increased.

When the culture vessel was changed to a T-25 flask, half of the culture solution, 2 ml, was replaced, and then 10 ml of new culture solution was added to increase the volume to 12 ml. At this time, the cells adhered to the bottom of the 6-well plate were also collected using a 24 mm scraper (TPP, 99002), counted, and then transferred to a T-25 flask.

On the 10th day of culture, the culture vessel was changed from a T-25 flask to a T-75 flask. At this time, half of the culture solution, 6 ml, was replaced, and then 24 ml of new culture solution was added to increase the volume of the culture solution to 30 ml. The cells adhered to the bottom of the T-25 flask were also collected and transferred using a 30 mm scraper (TPP, 99003). On the 12th day of culture, the culture vessel was changed from a T-75 flask to a T-150 flask. At this time, half of the culture solution, 15 ml, was replaced, and then 60 ml of new culture solution was added to increase the volume of the culture solution to 75 ml. When changing the culture vessel, the cells adhered to the bottom of the T-75 flask were also collected using a 30 mm scraper (TPP, 99003) and transferred to a T-150 flask.

On the 14th day of culture, the culture vessel was changed to a 350 ml CO₂ permeable bag. At this time, half of the culture solution (38 ml) was replaced, and then 151 mL of new culture solution was added to increase the volume of the culture solution to 188 ml. In addition, the cells adhered to the bottom of the T-150 flask were also collected using a 30 mm scraper (TPP, 99003) in the same manner as above, counted, and then transferred to a 350 ml CO₂ permeable bag. At this time, the above process was performed using a 50 ml Luer Lock syringe (NORM-JECT, 48500003000).

On the 17th day of culture, the volume of the culture solution was increased to 470 ml using a 50 ml Luer Lock syringe. From this time on, half of the culture solution was continuously added without being replaced. On the 19th day of culture, the culture vessel was changed to a 1 L CO₂ permeable bag, and the volume of the culture solution was increased to 1175 ml.

On the 21st day of culture, the cells being cultured in the 1 L CO₂ permeable bag were finally collected. In the present specification, the natural killer cells induced to differentiate from induced pluripotent stem cells were referred to as "EiNK cells".

### II. Confirmation of characteristics of produced natural killer cells

### Experimental Example 1. Verification of the expression of major markers of natural killer cells derived from induced pluripotent stem cells

The iPSC-derived natural killer cells produced by the methods of Examples 1.1 to 1.4 above were obtained by the method of Example 1.7 above, and the total number of suspension cells was confirmed, and then the expression of major markers of natural killer cells was confirmed by flow cytometry.

Specifically, the NK cells produced by the methods of Examples 1.1 to 1.4 above were obtained, and then the cell pellet was resuspended in FACS buffer (Invitrogen, 00-4222-57). The total number of cells was counted by the method of Example 1.7, and then 1×10⁵ cells were placed in a FACS tube, and 1 µl of the FACS antibodies presented in Table 7 were added per 100 µl of FACS buffer and suspended. The tube was wrapped with aluminum foil to block light, and staining was performed at refrigerated temperature for 1 hour.

1 mL of FACS buffer was added to the cells that had completed staining, and centrifugation was performed at 300g for 5 minutes, and then the supernatant was removed. The pellet was resuspended by adding 1 mL of FACS buffer again, and then centrifugation at 300g for 5 minutes was repeated twice again. Finally, the cells were resuspended in 500 µl of FACS buffer, and then the expression of cell surface markers was measured using a NovoCyte^{®} Flow Cytometer (Agilent, NovoCyte 3005) and analyzed using NovoExpress software (Agilent, Ver. 1.5.0).

**[Table 7]**

| Antigen (Clone) | Supplier | Cat No. |
|---|---|---|
| CD34 (8G12) | BD | 345804 |
| CD31 (L133.1) | BD | 340297 |
| KDR (89106) | BD | 560494 |
| CD45 (HI30) | BD | 555483 |
| CD43 (1G10) | BD | 560199 |
| CD56 (B159) | BD | 555518 |
| CD7 (M-T701) | BD | 340581 |
| NKG2A (Z 199) | Beckman Coulter | IM3291U |
| NKG2D (1D11) | BD | 561815 |
| CD16 (3G8) | BD | 560995 |
| NKp30 (p30-15) | BD | 558407 |
| NKp44 (p44-8) | BD | 558563 |
| NKp46 (9E2/NKp46) | BD | 557991 |
| KIRa,ah(EB6B) | Beckman Coulter | A09778 |
| KIRb1,b2 (GL183) | Beckman Coulter | IM2278U |
| KIRe1,e2 (Z27.3.7) | Beckman Coulter | IM3292 |
| CD3 (OKT3) | BD | 566683 |
| IgGK1 (MOPC-21) | BD | 555751 |
| IgGK1 (MOPC-21) | BD | 555749 |
| IgG2a,K (G155-178) | BD | 559319 |
| IgG2b,k (MPC-11) | BD | 559529 |

### Experimental Example 2. Evaluation of cytotoxicity of natural killer cells derived from induced pluripotent stem cell colonies

The cytotoxicity function of the natural killer cells derived from induced pluripotent stem cells produced by the methods of Examples 1.1 to 1.4 above was evaluated using Abcam's Cytotoxicity Assay Kit (Abcam, ab270780). Specifically, 1×10⁵ cells of chronic myeloid leukemia cell line, K562 cells, were suspended in 180 µl of measurement buffer (Abcam, ab270780) + 20 µl of CSFE solution (Abcam, ab270780), and then reacted at room temperature for 15 minutes to perform CSFE staining.

Centrifugation was performed at 1,000 rpm for 3 minutes, and then the supernatant was removed, and K562 cells that had completed CSFE staining and the natural killer cells (EiNK cells) derived from induced pluripotent stem cells produced by the methods of Examples 1.1 to 1.4 above were mixed in 400 µl of RPMI1640 (Hyclone, SH30027.01) culture solution containing 10% FBS (Gibco, 10100147) at a cell ratio of 1:10, 1:5, 2:1, and 1:1, respectively, and cultured in a cell incubator for 4 hours.

After the culture was completed, 20 µl of 7-AAD (Abcam, ab270780) solution was mixed with the cell suspension and reacted at 4 °C for 10 minutes, and then the cytotoxicity level was confirmed using a NovoCyte^{®} Flow Cytometer (Agilent, NovoCyte 3005) and analyzed using NovoExpress software (Aglient, Ver. 1.5.0). The results are shown in Figure 26.

### Experimental Example 3. Confirmation of characteristics of short-term mass proliferated natural killer cells

### Experimental Example 3.1. Morphological verification of produced natural killer cells

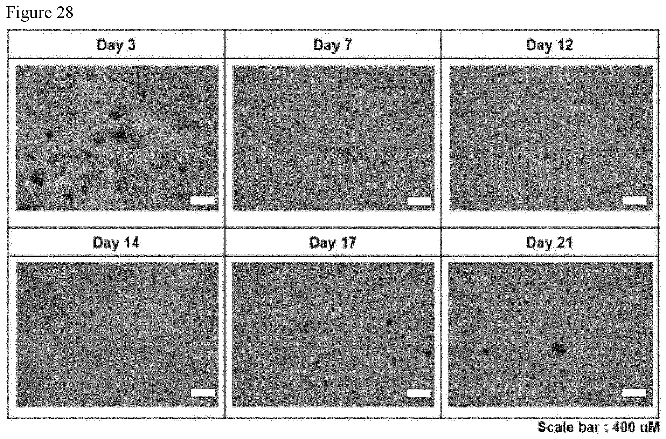

While mass proliferation on the iPSC-derived natural killer cells (EiNK cells) produced by the methods of Examples 1.1 to 1.4 above was performing by the method of Example 1.10, the morphological changes of the cultured natural killer cells were observed using an optical microscope. Specifically, the morphology of natural killer cells was confirmed using an optical microscope (Olympus, Olympus-CKX53) at the time of culture after the first, second, and third co-culture with feeder cells and during the 21-day mass proliferation process according to the process of mass proliferation of natural killer cells derived from induced pluripotent stem cells (Figure 28).

### Experimental Example 3.2. Confirmation of yield through mass proliferation process of natural killer cells derived from induced pluripotent stem cells

The yield of the natural killer cells (EiNK cells) derived from induced pluripotent stem cells produced in the same manner as in Examples 1.1 to 1.4 above was analyzed using the following method during mass proliferation culture by the method of Example 1.10.

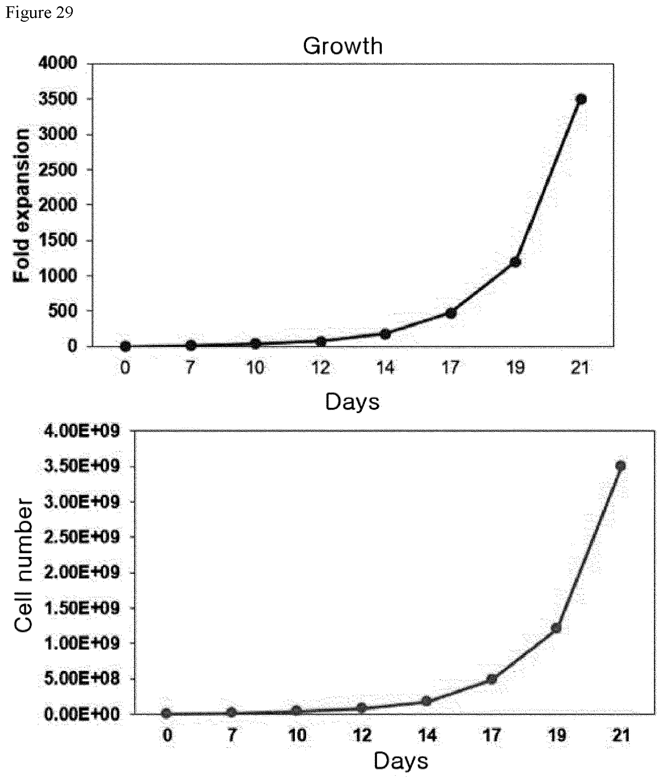

Specifically, during the mass culture process of natural killer cells derived from induced pluripotent stem cells and after the completion of mass culture, all cells suspended in the cell culture vessel were obtained together with the natural killer cell culture solution and centrifuged at 300 g for 5 minutes using a centrifuge (Labogene, 1580R). The cell pellet obtained by centrifugation was resuspended in the natural killer cell culture solution, and then the total number of cells in the suspension was counted using the Incyto c-chip (Incyto, DHC-N01-5), and the results are shown in Figure 29 and Table 8.

**[Table 8]**

| Days | Expansion fold | Cell number |
|---|---|---|
| 0 | 1 | 1.00E+06 |
| 7 | 17 | 1.70E+07 |
| 10 | 40 | 4.00E+07 |
| 12 | 80 | 8.00E+07 |
| 14 | 180 | 1.80E+08 |
| 17 | 480 | 4.80E+08 |
| 19 | 1200 | 1.20E+09 |
| 21 | 3500 | 3.50E+09 |

### Experimental Example 3.3. Verification of expression of cell markers of mass proliferated natural killer cells derived from induced pluripotent stem cells

The natural killer cells (EiNK cells) derived from induced pluripotent stem cells produced in the same manner as in Examples 1.1 to 1.4 above were obtained by the method of Example 1.7 above, and the total number of suspension cells was confirmed, and the expression of major markers of natural killer cells was confirmed by flow cytometry.

Specifically, the mass cultured natural killer cells derived from induced pluripotent stem cells were obtained, and then the cell pellet was resuspended in FACS buffer (Invitrogen, 00-4222-57). 1×10⁵ of the resuspended cells were placed in a FACS tube, and then 1 µl of each antibody that binds to the cell markers (activation marker proteins) was added per 100 µl of staining buffer and suspended. The tube was wrapped with aluminum foil to block light, and then staining was performed at refrigerated temperature for 1 hour.

1 mL of staining buffer was added to the cells that had completed staining, and centrifuged at 300g for 5 minutes to remove the supernatant, and then resuspended by adding 1 mL of staining buffer. The resuspended solution was centrifuged at 300g for 5 minutes. The above resuspension and centrifugation process was repeated twice. Finally, the cells were resuspended in 500 µl of staining buffer, and the expression of cell markers was measured using a NovoCyte^{®} Flow Cytometer (Agilent, NovoCyte 3005), and the measured values were analyzed using NovoExpress software (Aglient, Ver. 1.5.0). At this time, the used antibodies are shown in Tables 7 and 9. In addition, anti-IgG1 antibody (BD, 555751) and anti-IgG2a, κ antibody (BD, 559319) were additionally used.

**[Table 9]**

| Surface marker | Supplier (Cat No.) |
|---|---|
| NKG2D | BD (561815) |
| DNAM1 | BD (559789) |

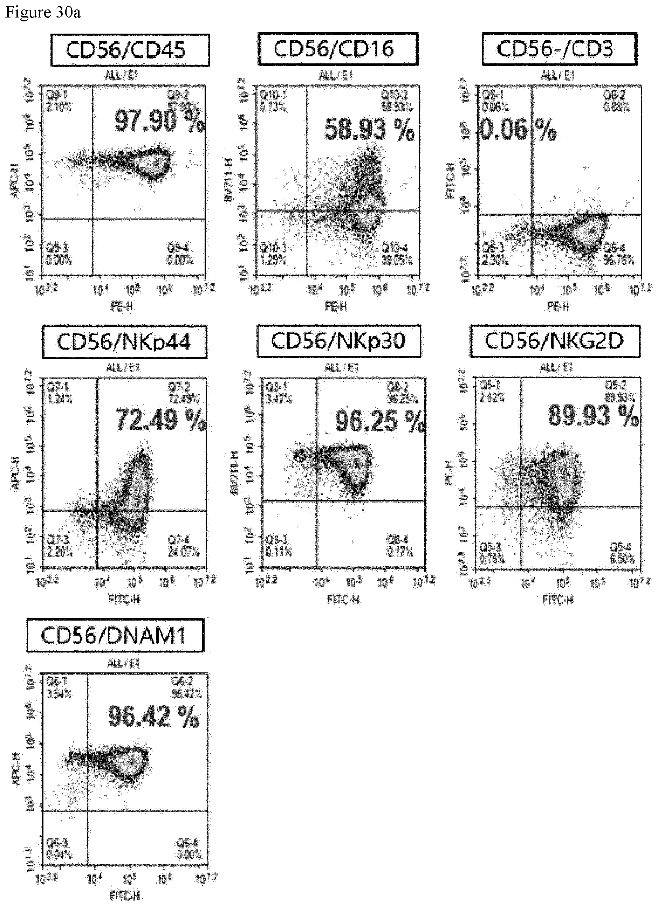

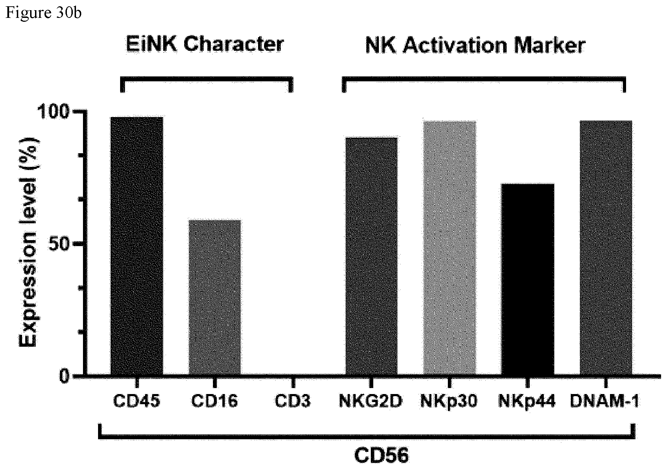

As a result, as shown in Figures 30a and 30b, the expression rate of NKG2D, one of the natural killer cell activation receptors, was about 90% or higher. The NKG2D is an important receptor for recognizing heterogeneous tumor cells, and is also an essential receptor in combination therapy with anticancer agents that increase the expression of NKG2D ligands in tumor cells. In addition, the expression rates of DNAM1 and NKp30 were confirmed to be about 90%. The expression rate of CD16, which is involved in the antibody-dependent cell mediated cytotoxicity (ADCC) of natural killer cells, was about 50%. At this time, the receptor plays a role in mediating other forms of MHC non-restricted killing.

### Experimental Example 3.4. Confirmation of natural killer cell characteristics of mass proliferated natural killer cells derived from induced pluripotent stem cells

In order to confirm the killing ability of the natural killer cells (EiNK cells) derived from induced pluripotent stem cells produced in the same manner as in Examples 1.1 to 1.4 above against tumor cells, EiNK cells were obtained on day 21 and day 28 of mass proliferation by the method of Example 1.7. The obtained natural killer cells were counted and cultured with a blood cancer cell line (K562) stained with a fluorescent substance, CFSE (Abcam, ab270780). At this time, the fluorescently stained blood cancer cell line was prepared by the following method. First, CFSE was diluted 1:250, and K562 cells were treated with CFSE and then reacted at room temperature for 15 minutes.

1 mL of cell culture solution was added to the cells that had completed staining, and centrifugation was performed at 2,000 rpm for 3 minutes, and then the supernatant was removed. 2 ml of cell culture solutionwas added to the cells again, reacted in a 37 °C 5% CO₂ incubator for 30 minutes, and then centrifuged at 2,000 rpm for 3 minutes to obtain stained K562 cells. The K562 cells and natural killer cells prepared by the above method were counted, inoculated into each well according to the E:T ratio, and co-cultured for 4 hours.

After 4 hours of co-culture, all cells in the co-culture were placed in a FACS tube and then centrifuged at 2,000 rpm for 3 minutes. After centrifugation, the cells were resuspended by adding 1 mL of physiological saline and then centrifuged again at 2,000 rpm for 3 minutes to remove the supernatant. The above resuspension and centrifugation were repeated twice. Thereafter, the cells were suspended in 200 µl of staining buffer, treated with 7-AAD (Abcam, ab270780), and then reacted on ice for 10 minutes.

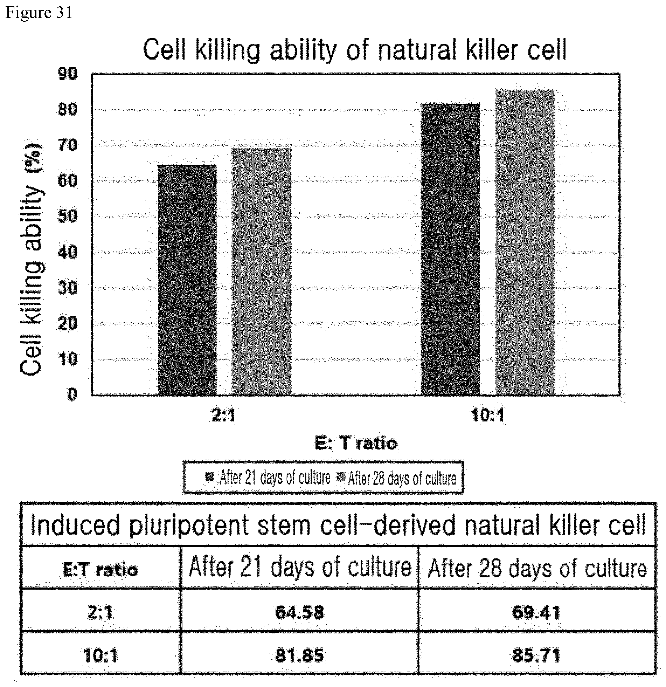

After the reaction was completed, the cells stained with CFSE and 7-AAD at the same time were analyzed using a NovoCyte^{®} Flow Cytometer (Agilent, NovoCyte 3005). The measured values were analyzed using NovoExpress software (Agilent, Ver. 1.5.0), and the results are shown in Figure 31 and Table 10.

**[Table 10]**

| Natural killer cells derived from induced pluripotent stem cells | | |
|---|---|---|
| E:T ratio | After 21 days of culture | After 28 days of culture |
| 2:1 | 64.58 | 69.41 |
| 10:1 | 81.85 | 85.71 |

### Experimental Example 3.5. Confirmation of natural killer cell characteristics of mass proliferated natural killer cells derived from induced pluripotent stem cells

The natural killer cells derived from induced pluripotent stem cells (EiNK cell) mass cultured for 26 days in the same manner as in Example 1.10 above were obtained in the same manner as in Experimental Example 3.4 above, and the total number of suspension cells was confirmed, and the expression of iPSC markers (marker proteins, Table 11) in the obtained cells was confirmed by flow cytometry.

For flow cytometry, specifically, the natural killer cells produced by the method of Examples 1 were obtained, and then the cell pellet was resuspended in FACS buffer (Invitrogen, 00-4222-57). The total number of cells was counted, and then 1×10⁵ cells were placed in a FACS tube. 1 µl of the antibodies shown in Table 5 was added per 100 µl of FACS buffer and suspended. The tube was wrapped with aluminum foil to block light, and then the cells were stained by reacting at a refrigerated temperature for 1 hour. After staining was completed, 1 mL of FACS buffer was added to the cells, and centrifugation was performed at 300 g for 5 minutes. After removing the supernatant, the cell pellet was resuspended in 1 mL of FACS buffer and centrifuged again at 300 g for 5 minutes to wash the cells. The washing process was repeated a total of 2 times. Finally, the cells were resuspended by adding 500 µl of FACS buffer, and then the expression of iPSC markers on the cell surface was measured using a NovoCyte^{®} Flow Cytometer (Agilent, NovoCyte 3005), and the measured values were analyzed using NovoExpress software (Aglient, Ver. 1.5.0).

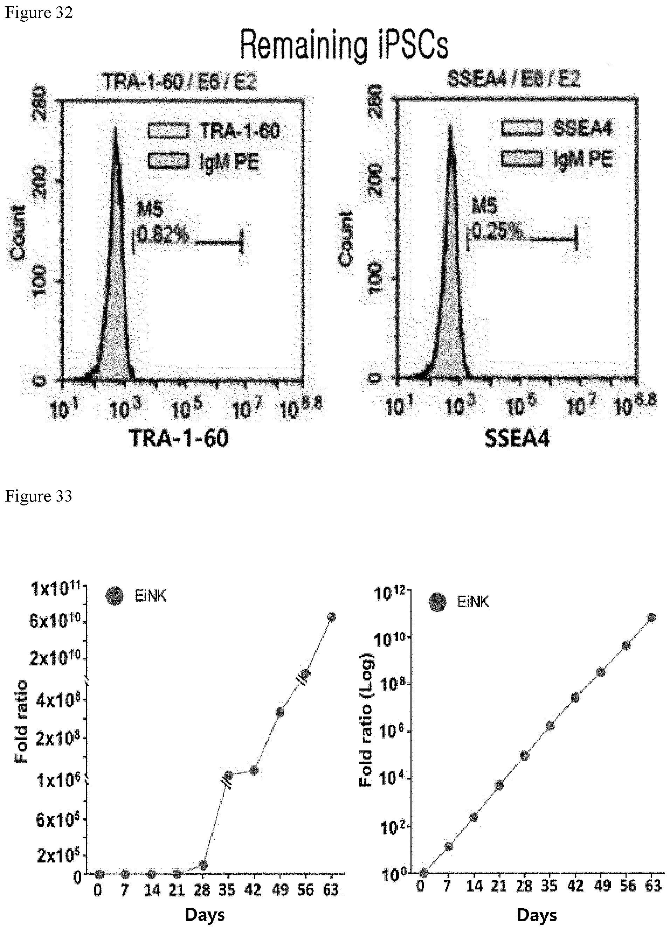

As a result, as shown in Figure 32, it was confirmed that the expression rate of marker factors of induced pluripotent stem cells in the mass cultured natural killer cells derived from induced pluripotent stem cells was 1% or less. Based on the above results, it was confirmed that there were almost no induced pluripotent stem cells remaining in the cells after the differentiation induction.

**[Table 11]**

| Antigen (Clone) | Manufacturer | Catalog number |
|---|---|---|
| Tra-1-60 (TRA-1-60) | eBioscience | 12-8863-82 |
| SSEA4 (MC-813-70) | eBioscience | 12-8843-42 |
| Mouse IgM (G155-228) | BD | 555584 |
| Mouse IgG3 (B10) | eBioscience | 12-4742-42 |

### Experimental Example 4. Confirmation of characteristics of long-term mass proliferated natural killer cells derived from induced pluripotent stem cells

### Experimental Example 4.1. Confirmation of yield of long-term mass proliferated natural killer cells derived from induced pluripotent stem cells

The EiNK cells produced by the methods of Examples 1.1 to 1.4 above were cultured for up to 63 days, and then the proliferation rate of EiNK cells was confirmed. At this time, the EiNK cell medium was used by adding β-mercaptoethanol (Gibco, 21985-023), sodium selenite (Sigma, S5261-100G), L-ascorbic acid (Sigma, A4544-100G), and IL-15 (R&D systems, 247-ILB-500/CF) to RPMI1640 (Cytiva, SH30027.01) containing human AB serum (Gemcell, 100-512) and penicillin-streptomycin (Gibco, 15140-122) (Table 12).

**[Table 12]**

| Culture medium for mass proliferation of natural killer cells derived from induced pluripotent stem cells | |
|---|---|
| Basic medium | DMEM/(Gibco, 10566-016) |
| | Ham's F12 Nutrient Mix/(Gibco, 31765035) |
| | L-Glutamine (1%)/(Gibco, 25030081) |
| | Human AB serum (2 to 20%)/(Gemcell, 100-512) |
| | Penicillin-Streptomycin (1%)/(Gibco, 15140-122) |
| | Sodium selenite (0.1 to 50 ng/ml)/(Sigma, S5261) |
| | Ehtanolamine (0.1 to 250 µm)/(Sigma, E0135) |
| | β-mercaptoethanol (0.1 to 5 µm)/(Gibco, 21985-023) |
| | L-ascorbic acid (1 to 250 ng/ml)/(Sigma, A4544) |
| Additional ingredients | IL-2 (1 to 1000 IU/ml)/(BL&H) |
| | IL-7 (0.2 to 250 ng/ml)/ (R&D systems, 207-IL-200/CF) |
| | SCF (0.2 to 250 ng/ml)/ (R&D systems, 255-SC/CF) |
| | IL-15 (0.1 to 100 ng/ml)/ (R&D systems, 247-ILB-500/CF) |
| | FLT-3 Ligand (0.1 to 100 ng/ml)/ (R&D systems, 308-FK/CF) |

The EiNK cells were stimulated every 7 days with irradiated feeder cells and cultured for up to 63 days. As a result, as shown in Figure 33, the EiNK cells cultured for up to 63 days were proliferated by 6.5×10¹⁰ times that of the initially cultured cells (fold expansion). As a result of confirming the results as log values, it was confirmed that the cell were expanded while maintaining the period-specific fold expansion at a constant rate.

### Experimental Example 4.2. Confirmation of cell killing ability of long-term mass proliferated iPSC-derived natural killer cells

The phenotype of the EiNK cells produced in the same manner as in Experimental Example 4.1 above, and the natural killer cell phenotype and killing ability against K562 cells were confirmed. The phenotype was confirmed in the same manner as in Experimental Example 3.3 and Experimental Example 3.5, and the killing ability was confirmed in the same manner as in Experimental Example 3.4.

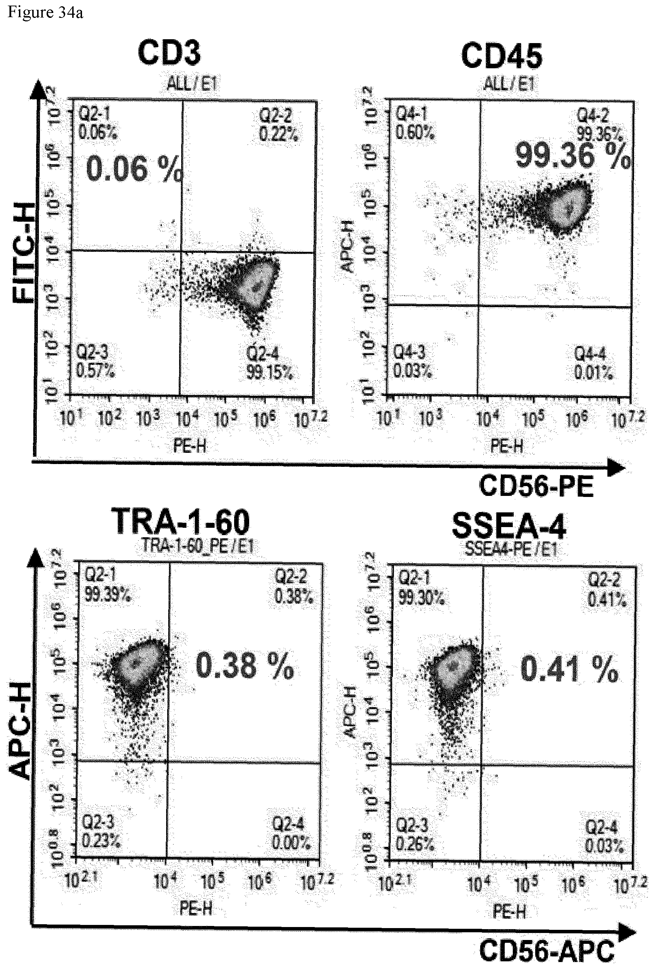

As a result, as shown in Figure 34a, it was confirmed that in EiNK cells cultured for 63 days, the expression rate of CD56+CD45, a natural killer cell phenotype, was 99%, and the expression rate of CD56-CD3+, a T cell phenotype, was 0.06%, indicating that there were almost no T cells. In addition, as a result of confirming the induced pluripotent stem cell phenotype (TRA-1-60+, SSEA-4+) in EiNK cells cultured for 63 days, the expression rate was 1% or less, indicating that almost no induced pluripotent stem cells remained.

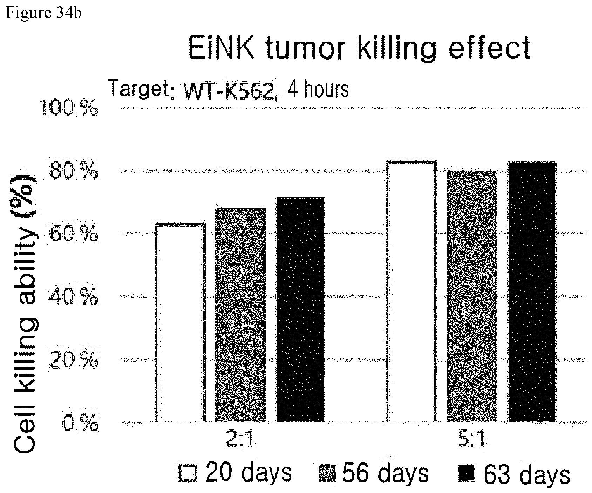

In addition, as a result of confirming the cell killing ability of EiNK cells cultured for 20, 56, and 63 days against cancer cells, as shown in Figure 34b, a high level of cancer cell killing ability was observed regardless of the culture period. Based on the above results, it was confirmed that the EiNK cells of the present invention maintain high cytotoxicity even after long-term culture.

### Experimental Example 5. Confirmation of anticancer activity of iPSC-derived natural killer cells: In vivo

The anticancer activity of the EiNK cells (cultured for 21 days) mass cultured by the method of Example 1.10 above was confirmed at the animal level. At this time, 6-week-old female NSG mice were used.

Specifically, luciferase was overexpressed in OVCAR3 cells, an ovarian cancer cell line, and 2×10⁶ cells/100 µl (PBS, vehicle) were administered intraperitoneally to each mouse. The EiNK cells used as test substances were thawed 2 days before administration, stabilized in a T-75 flask for 2 days, and then administered intraperitoneally to each mouse at 1 × 10⁷ cells/100 µl (PBS, vehicle). At this time, RPMI medium containing 5% to 15% human AB serum, 1% L-glutamine, 50 IU/ml IL-2, and 10 ng/ml IL-15 was used as the culture medium for the thawed EiNK cells. The vehicle treatment group was used as the control.

The test substance (EiNK) was administered 12 times in total on days 2 (day 0), 4 (day 2), 6 (day 4), 9 (day 7), 11 (day 9), 13 (day 11), 16 (day 14), 18 (day 16), 20 (day 18), 23 (day 21), 25 (day 23), and 27 (day 25) after tumor cell administration. At the same time, IL-2 (10,000 IU/mouse) and IL-15 (0.5 ug/mouse) were administered intraperitoneally at intervals of 2 to 3 days for 28 days. After test substance administration, the weight of the mice was measured three times in total, once every 7 days from day 0. In addition, luciferin was administered into the right abdominal cavity of mice on days 0, 3, 7, 10, 14, 17, 21, 24, and 28, and the tumor cells present in the mice were confirmed using IVIS imaging equipment after a reaction for 10 minutes.

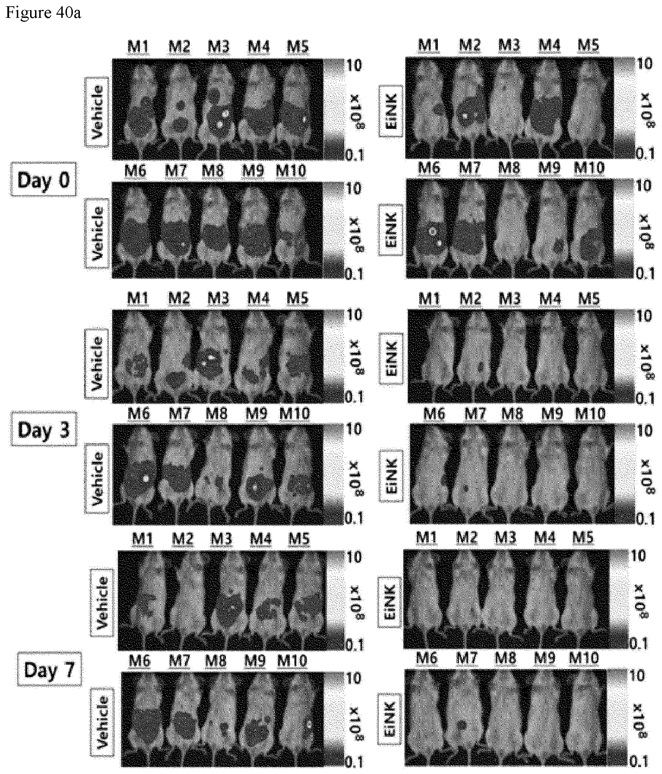

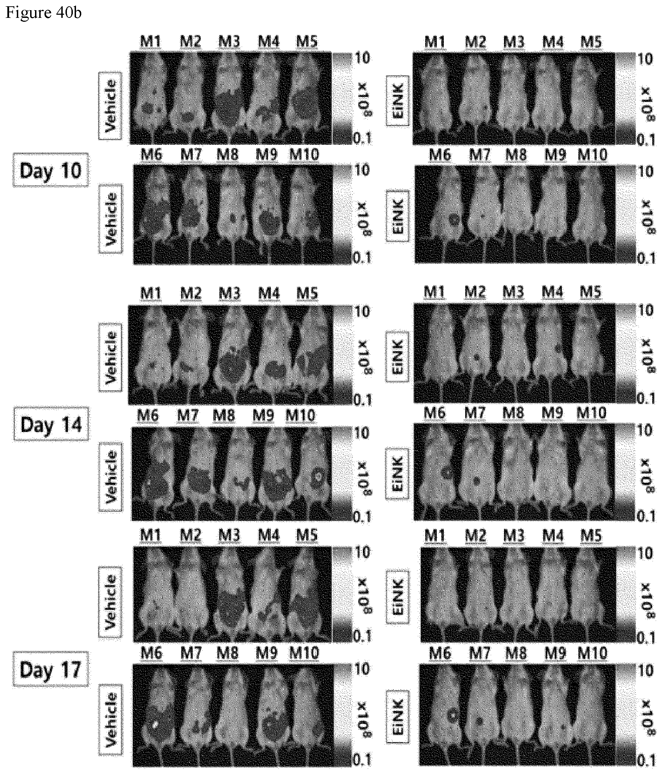

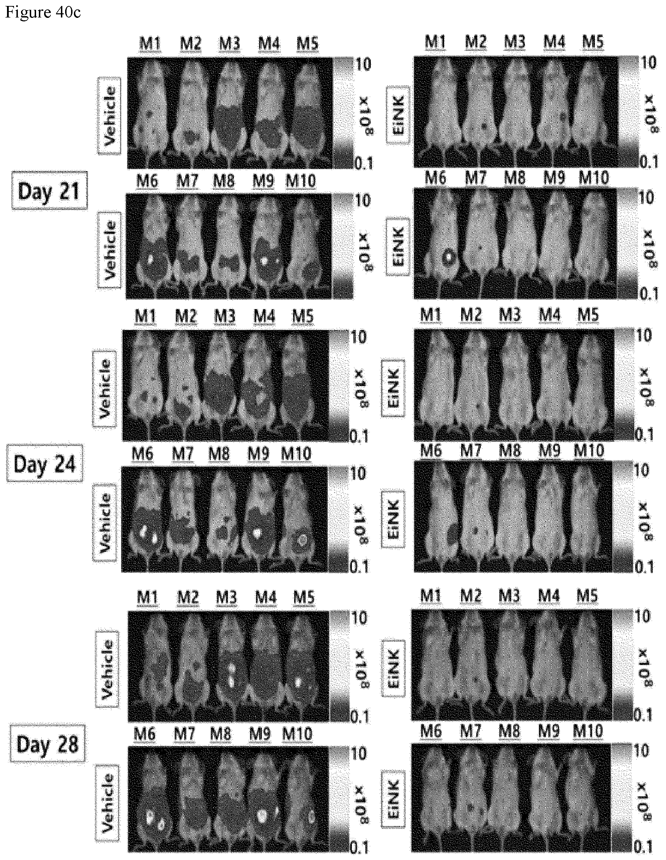

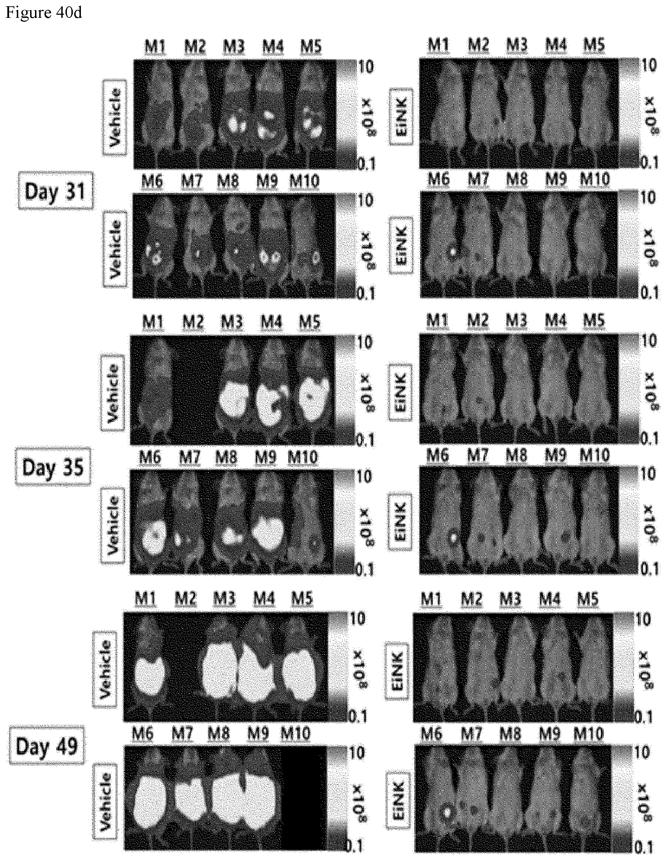

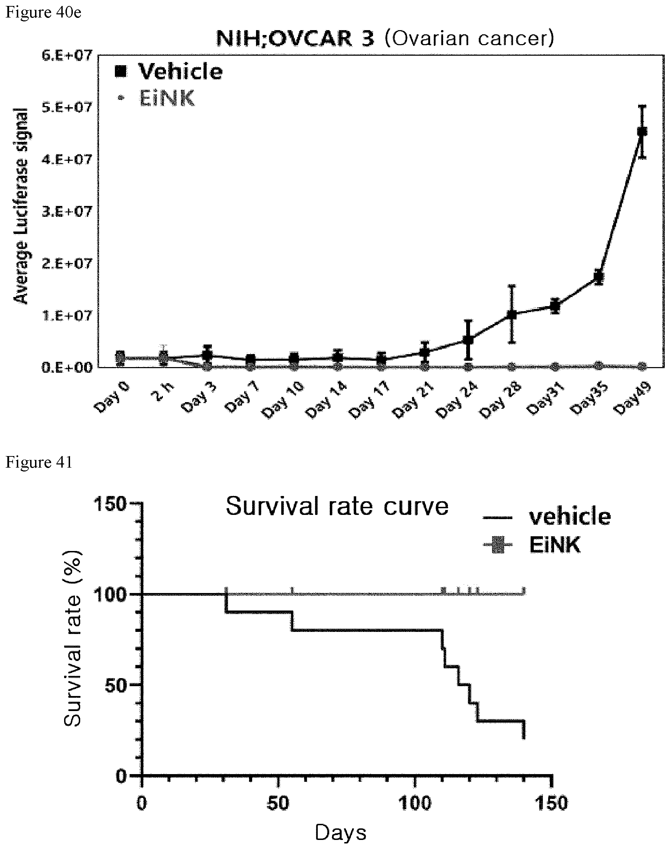

As a result, as shown in Figures 40a to 40e, it was confirmed that in the control (vehicle), luciferase activity was increased rapidly after day 17. The results suggest that the OVCAR3 cells administered into the abdominal cavity were gradually proliferated *in vivo* over time. On the other hand, in the test substance treatment group, the tumor cells continued to decrease from the day after test substance administration (day 0), and after the third day, almost no tumor cells proliferated in the abdominal cavity could be found. Based on the above results, it was confirmed that the EiNK cells have anticancer activity at the animal level.

### III. Comparison of iPSC-derived natural killer cells and blood-derived natural killer cells

### Experimental Example 6. Comparison of cell surface marker expression of mass proliferated iPSC-derived natural killer cells and blood-derived natural killer cells

The EiNK cells (cultured for 21 days) mass cultured by the method of Example 1.10 above; and the natural killer cells (PBNK, cultured for 22 days) isolated from blood and then cultured by the method of Example 1.10 were counted, and the expression of natural killer cell surface marker proteins (markers) was confirmed by flow cytometry.

At this time, the expression of cell surface markers was analyzed using BD Lyoplate human cell surface screening panel (BD, 560747). A panel of 242 antibodies for the human cell surface markers was used to label EiNK cells and PBNK cells.

Specifically, the EiNK cells were harvested and counted on the 21st day of mass culture, and the PBNK cells were harvested and counted on the 22nd day of culture. Thereafter, the cells were resuspended in staining buffer (FACS buffer) at a concentration of 2.5 × 10⁶ cells/ml. The resuspended cells were inoculated into each well of a 96-well plate at a density of 2.5 × 10⁵ cells/well.

Each inoculated cell was labeled with 242 primary antibodies for 30 minutes, and then the cells were washed with staining buffer and centrifuged (at 300 g, for 4 minutes) to remove the supernatant. The primary antibodies were washed, and then Alexa 647-labeled secondary antibodies (mouse or rat) were added to each primary antibody and reacted for 30 minutes. After the secondary antibody reaction, the cells were washed with staining buffer and centrifuged at 300 g for 4 minutes to remove the supernatant. The cells that had completed staining were measured using a NovoCyte^{®} Flow Cytometer (Agilent, NovoCyte 3005), and the measured values were analyzed using NovoExpress software (Aglient, Ver. 1.5.0).

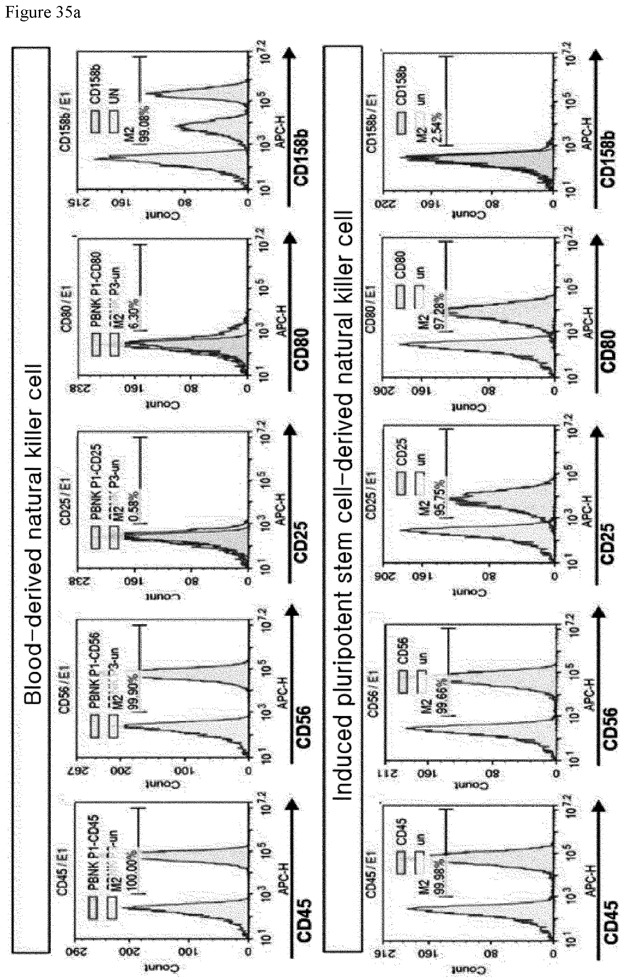

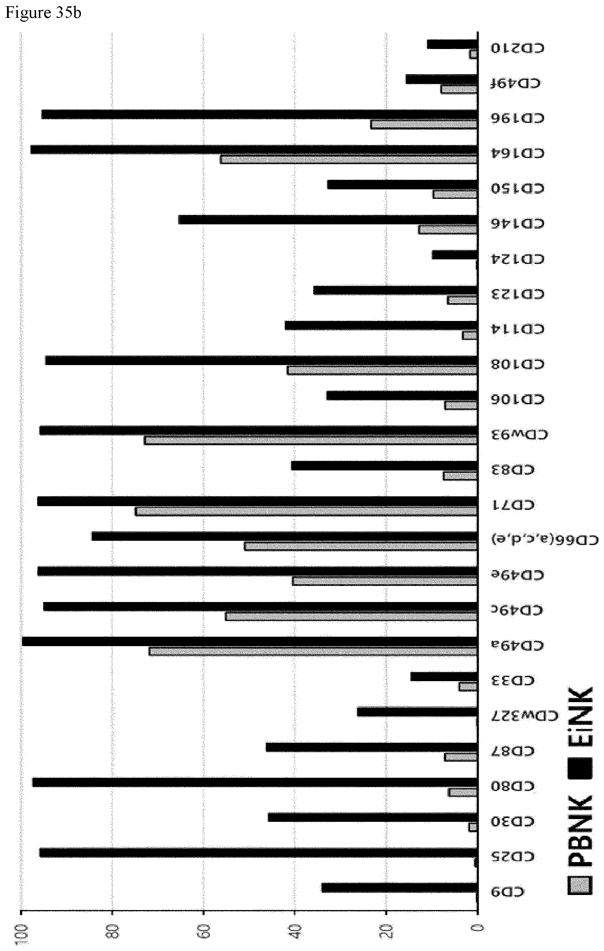

Among the results, the results of cell surface markers that showed differences in EiNK cells mass proliferated for 21 days and PBNK cells cultured for 22 days are shown in Figures 35a and 35b, and Tables 13 and 14.

**[Table 13]**

| Item | Cell surface marker | Expression rate (%) | |
|---|---|---|---|
| | | PBNK | EiNK |
| 1 | CD9 | 0.26 | 34 |
| 2 | CD25 | 0.58 | 95.75 |
| 3 | CD30 | 1.88 | 45.67 |
| 4 | CD80 | 6.3 | 97.28 |
| 5 | CD87 | 7.14 | 46.2 |
| 6 | CDw327 | 0.08 | 26.23 |
| 7 | CD33 | 4.02 | 14.53 |
| 8 | CD49a | 71.9 | 99.5 |
| 9 | CD49c | 55.12 | 94.89 |
| 10 | CD49e | 40.54 | 96.23 |
| 11 | CD66(a,c,d,e) | 50.98 | 84.41 |
| 12 | CD71 | 74.9 | 96.27 |
| 13 | CD83 | 7.44 | 40.66 |
| 14 | CDw93 | 72.91 | 95.72 |
| 15 | CD 106 | 7.06 | 32.87 |
| 16 | CD108 | 41.58 | 94.57 |
| 17 | CD114 | 3.2 | 42.09 |
| 18 | CD 123 | 6.5 | 35.73 |
| 19 | CD124 | 0.22 | 9.88 |
| 20 | CD 146 | 12.84 | 65.32 |
| 21 | CD150 | 9.72 | 32.69 |
| 22 | CD164 | 56.24 | 97.67 |
| 23 | CD196 | 23.3 | 95.32 |
| 24 | CD49f | 7.98 | 15.51 |
| 25 | CD210 | 1.66 | 10.83 |

**[Table 14]**

| Cell surface marker | PBNK | EiNK | Supplier (clone, Cat No.) |
|---|---|---|---|
| CD243 | 63.88 | 0.28 | BD (17F9, 560747) |
| γδTCR | 46.44 | 0.28 | BD (B1, 560747) |
| NKB1 | 56.14 | 0.42 | BD (DX9, 560747) |
| CD205 | 82.8 | 2.02 | BD (MG38, 560747) |
| CD158b | 99.08 | 2.54 | BD (HP-3E4, 560747) |
| CD158a | 34.1 | 0.4 | BD (HP-3E4, 560747) |
| CDw328 | 24.72 | 1.22 | BD (F023-420, 560747) |

As a result, it was confirmed that among a total of 242 surface markers, 26 markers had an increased expression rate in EiNK cells compared to PBNK cells. It was confirmed that markers that were increased more than 20-fold compared to PBNK cells were CD25, CD9, CDw327, and CD30, and markers that were increased more than 10-fold compared to PBNK cells were CD80 and CD114. In addition, it was confirmed that markers that were increased more than 5-fold compared to PBNK cells were CD124, MIC A_B, CD210, CD87, CD123, CD83, and CD146, and markers that were increased more than 1.3-fold compared to PBNK cells were CD106, CD196, CD33, CD150, CD49e, CD108, CD49f, CD164, CD49c, CD66(a,c,d,e), CD49a, CDw93, and CD71. On the other hand, 7 markers were expressed only in blood-derived natural killer cells.

### Experimental Example 7. Comparison of gene expression of natural killer cells derived from induced pluripotent stem cells and blood-derived natural killer cells

For the EiNK cells (cultured for 28 days) mass cultured by the method of Example 1.10 above; and the natural killer cells (PBNK, cultured for 22 days) isolated from blood and then cultured by the method of Example 1.10, RNA was extracted from the natural killer cells and subjected to transcriptome sequencing and data analysis using QuantSeq 3' mRNA-Seq. Analysis of the entire genome RNA sequencing was performed by outsourcing to Ebiogen (Korea). The sequencing results were analyzed for gene expression changes using ExDEGA (Excel based Differentially Expressed Gene Tool).

As a result, as shown in Figure 36, it was confirmed that the expression of factors involved in tumor cell-killing and factors involved in immune activation was increased in EiNK cells compared to PBNK cells.

### Experimental Example 8. Comparative analysis of chemokines and cytokines secreted in culture solution of iPSC-derived natural killer cells and blood-derived natural killer cells

For the EiNK cells (cultured for 28 days) mass cultured by the method of Example 1.10 above; and the natural killer cells (PBNK, cultured for 22 days) isolated from blood and then cultured by the method of Example 1.10, cytokines of natural killer cells in the culture solution were analyzed. Cytokine analysis was performed using the Proteome Filer Human XL Cytokine Array Kit (R&D Systems, Cat. No. ARY022B) according to the manufacturer's protocol. The measurement results of the cytokine array kit were analyzed using GelQuantNet software (BiochemLabSolutions) for relative dot intensity to the reference control dot. The cytokines, chemokines, and growth factors used in the cytokine array are listed in Table 15.

Specifically, the qualitative analysis of 102 cytokines, chemokines, or cell growth factors was performed according to the following process. After preparing all reagents and culture solution samples of the provided product at room temperature, Array Buffer 6 and membrane were added to each well of a pre-designed 4-well plate and reacted at room temperature for 1 hour in a rocking shaker. Thereafter, the solution was discarded, and the culture solution sample was added together with Array Buffer 6 to a final volume of 1.5 ml (the culture solution sample was diluted to 1/10 of the original concentration and used). After reacting for 12 hours or more while maintaining 2 to 8 °C in a rocking shaker, the mixture was washed three times for 10 minutes each with a washing solution. The Detection Antibody Cocktail (60 µl) was added to the mixture (6 ml) of the provided Array Buffer 6 (4 ml) and Array Buffer 4 (2 ml), and each well was treated with 1.5 ml and reacted at room temperature for 1 hour. Thereafter, the wells were washed three times for 10 minutes each with the washing solution, and Streptavidin-HRP was added to each well and reacted for 30 minutes. Thereafter, the wells were washed three times for 10 minutes each with the washing solution, and the Chemi Reagent Mix solution was added and reacted for 1 minute, and the luminescence level was confirmed (Chemi-doc photography).

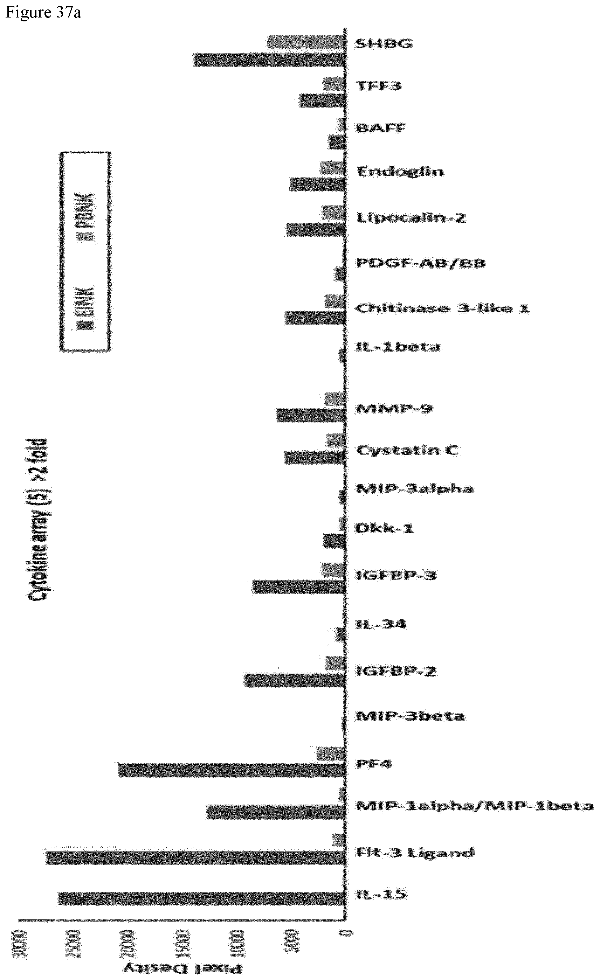

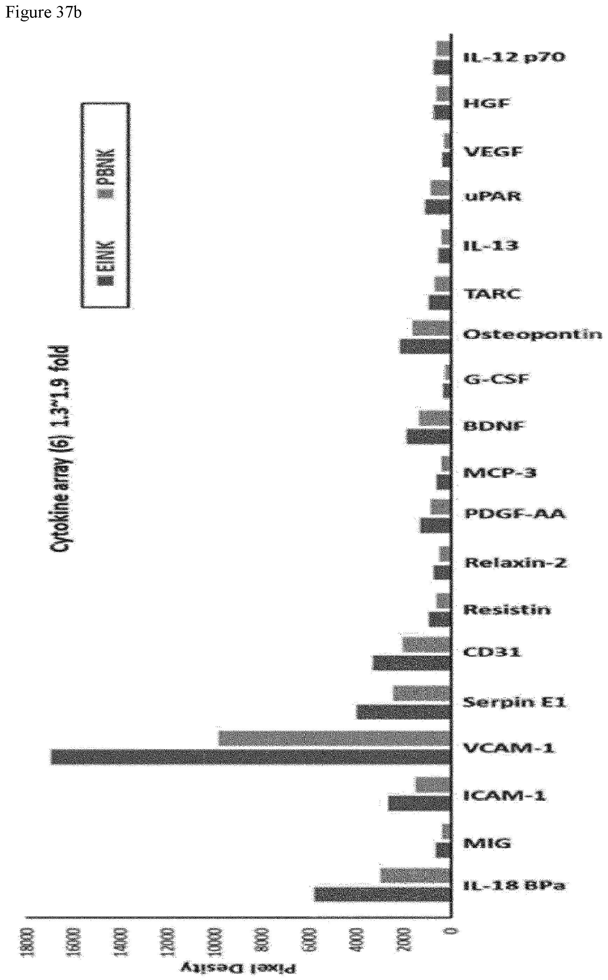

As a result, as shown in Figures 37a and 37b, it was confirmed that the concentration of chemokines and cytokines released by activated natural killer cells in the EiNK cell culture solution was higher compared to PBNK cells.

**[Table 15]**

| Proteome Profiler Human XL Cytokine Array Kit (ARY022B) | | |
|---|---|---|
| Adiponectin/Acrp30 | IFN-gamma | Lipocalin-2/NGAL |
| Aggrecan | IGFBP-2 | CCL2/MCP-1 |
| Angiogenin | IGFBP-3 | CCL7/MCP-3 |
| Angiopoietin-1 | IL-1 alpha/IL-1F1 | M-CSF |
| Angiopoietin-2 | IL-1 beta/IL-1F2 | MIF |
| BAFF/BLyS/TNFSF13B | IL-1ra/IL-1F3 | CXCL9/MIG |
| BDNF | IL-2 | CCL3/CCL4 MIP-1 alpha/beta |
| CD14 | IL-3 | CCL20/MIP-3 alpha |
| CD30 | IL-4 | CCL19/MIP-3 beta |
| CD40 ligand | IL-5 | MMP-9 |
| Chitinase 3-like 1 | IL-6 | Myeloperoxidase |
| Complement Component C5/C5a | IL-8 | Osteopontin (OPN) |
| Complement Factor D | IL-10 | PDGF-AA |
| C-Reactive Protein/CRP | IL-11 | PDGF-ABBB |
| Cripto-1 | IL-12 p70 | Pentraxin-3 |
| Cystatin C | IL-13 | CXCL4/PF4 |
| Dkk-1 | IL-15 | RAGE |
| DPPIV/CD26 | IL-16 | CCL5/RANTES |
| EGF | IL-17A | RBP4 |
| CXCL5/ENA-78 | IL-18 BPa | Relaxin-2 |
| Endoglin/CD105 | IL-19 | Resistin |
| EMMPRIN | IL-22 | CXCL12/SDF-1 alpha |
| Fas Ligand | IL-23 | Serpin E1/PAI-1 |
| FGF basic | IL-24 | SHBG |
| KGF/FGF-7 | IL-27 | ST2/IL-1 R4 |
| FGF-19 | IL-31 | CCL17/TARC |
| Flt-3 Ligand | IL-32 alpha/beta/gamma | TFF3 |
| G-CSF | IL-33 | TfR |
| GDF-15 | IL-34 | TGF-alpha |
| GM-CSF | CXCL10/IP-10 | Thrombospondin-1 |
| CXCL1/GRO alpha | CXCL 1 1/I-TAC | TNF-alpha |
| Growth Hormone (GH) | Kallikrein 3/PSA | uPAR |
| HGF | Leptin | VEGF |
| ICAM-1/CD54 | LIF | Vitamin D BP |

### Experimental Example 9. Comparative analysis of expression of inhibitory receptors and cell surface markers of iPSC-derived natural killer cells and blood-derived natural killer cells

For the EiNK cells (cultured for 21 days) mass cultured by the method of Example 1.10 above; and the natural killer cells (PBNK, 21 days) isolated from blood and then cultured by the method of Example 1.10, the expression of natural killer cell inhibitory receptors and cell surface markers was confirmed by flow cytometry. The above experiment was repeated a total of 4 times.

The cells were obtained and used in the same manner as in Experimental Example 3.4, and FACS analysis was performed by the method of Experimental Example 3.5. At this time, the antibodies used in the analysis are described in Table 7, Table 11, and Table 16.

**[Table 16]**

| Antigen (Clone) | Supplier | Cat. No |
|---|---|---|
| CD56 (CMSSB) | eBioscience | 12-0567-42 |
| CD94 (HP-3D9) | BD | 559876 |
| TIM-3 (F38-2E2) | eBioscience | 12-3109-42 |
| TIGIT (MBSA43) | eBioscience | 12-9500-42 |
| CD57 (NK-1) | BD | 560844 |
| Mouse IgG1 kappa Isotype (P3.6.2.8.1) | eBioscience | 12-4714-82 |

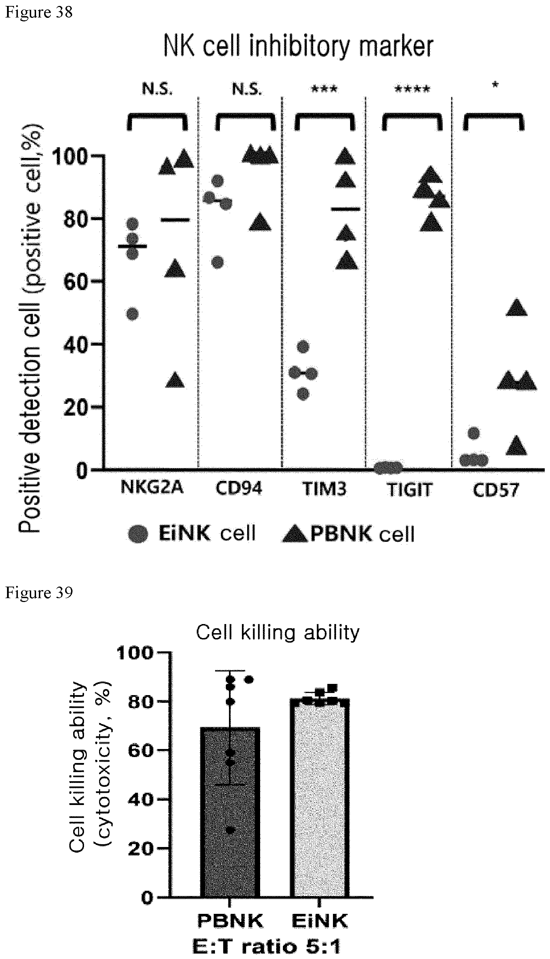

As a result, as shown in Figure 38, NKG2A/CD94PBNK, a marker protein expressed in mature natural killer cells, showed similar expression rates in PBNK cells and EiNK cells. On the other hand, it was confirmed that TIM-3 and TIGIT, which are known to have the function of suppressing immune activity as inhibitory receptors, have a reduced expression rate in EiNK cells compared to PBNK cells. Based on the above results, it is thought that the immune activation ability of EiNK cells is more superior than that of PBNK cells.

### Experimental Example 10. Comparison of tumor cell-killing ability of iPSC-derived natural killer cells and blood-derived natural killer cells

For the EiNK cells (cultured for 21 days or 28 days) mass cultured by the method of Example 1.10 above; and the natural killer cells (PBNK, cultured for 16 days) isolated from blood and then cultured by the method of Example 1.10, the tumor cell-killing ability of natural killer cells was confirmed. Each cell was obtained by the method of Example 3.4, and the tumor cell-killing ability was confirmed by the method of Example 3.4. The above experiment to confirm the killing ability was repeated a total of 7 times.

As a result, as shown in Figure 39, it was confirmed that the killing ability of EiNK cells or PBNK cells against tumor cells was at a similar level. However, while PBNK cells showed differences in cell killing ability depending on blood donor, EiNK cells showed similar levels of tumor cell-killing ability depending on batch culture.

### IV. Comparison of method for differentiating natural killer cells derived from induced pluripotent stem cells of the present invention and conventional method for differentiating natural killer cells derived from induced pluripotent stem cells

### Experimental Example 11. Comparison of cell yield and proliferation rate according to CD34+ sorting

Figure 42a shows the results obtained by confirming the changes in cell yield, cell surface marker expression, and cell proliferation rate by the CD34 positive cell sorting process, which is a hematopoietic stem cell differentiation marker, during the conventional method for differentiating natural killer cells derived from induced pluripotent stem cells.

Specifically, the induction of natural killer cell differentiation from conventional iPSCs was performed as follows.

For the differentiation of induced pluripotent stem cells (iPSCs), iPSCs were inoculated into the Aggrewell plate (aggrewell 400, STEMCELL, 34425), and then embryoid body (EB) formation was induced for 5 days while replacing the EB-A medium (STEMdiff^{™} NK Cell Kit, STEMCELL, 100-0170) every day. At this time, only 50% of the existing medium was removed, and new medium was added in the amount to be removed. In order to collect only EBs larger than a certain size, the medium containing EBs was passed through a strainer (reversible 37 µm, STEMCELL, 27215) to remove single cells or small-sized EBs. After collecting the EBs filtered through the strainer by washing them with the medium, they were inoculated into a non-tissue culture 6-well plate (STEMCELL, 38040) and cultured for 7 days by replacing the medium using EB-B medium (STEMdiff^{™} NK Cell Kit, STEMCELL, 100-0170) once every 2 to 3 days. At this time, the medium replacement method was the same as described above.

The EBs cultured as above were collected and reacted with collagenase (Collagenase Type IV, STEMCELL, 07909) for 20 minutes and with TrypLE Select (Gibco, A12563011) for 20 minutes (total 40 minutes of reaction with the degrading enzyme) to separate them into single cells. The separated cells were sorted only for CD34 expressing cells using Easysep Human CD34 Positive Selection Kit II (STEMCELL, 17856). At this time, sorting was performed by collecting suspension cells, mixing them with anti-CD34 coupled to magnetic beads antibodies (CD34 microbeads kit, Miltenyi Biotec, 130-046-702), loading them onto an LC column (Miltenyi Biotec, 130-042-401) adhered to a MACS sorter, washing the column several times with DPBS (Cytiva, SH3002802), and collecting the cells remaining in the column.

The separated cells were inoculated into a non-tissue culture 24-well plate (STEMCELL, 38042), and the medium was replaced once every 3 to 4 days. The medium replacement method was the same as described above. From day 12 to day 19 after differentiation induction, lymphocyte differentiation medium (STEMdiff^{™} NK Cell Kit, STEMCELL, 100-0170) was used, and from day 19, lymphoid progenitor expansion medium (STEMdiff^{™} NK Cell Kit, STEMCELL, 100-0170) was used for culture. On day 26 of differentiation induction, all cells were collected, and the number of cells was measured, and then the cells were re-inoculated into a non-tissue culture 24-well plate (STEMCELL, 38042) and cultured. The medium was replaced once every 3 to 4 days as described above. On day 41 of differentiation induction, all cells were collected, flow cytometry analysis and cell counting were performed to confirm the results of natural killer cell differentiation.

As described above, when conventionally inducing differentiation from induced pluripotent stem cells to natural killer cells, differentiation from mesodermal cells to hematopoietic stem cells was induced, and then only CD34 positive cells (hematopoietic stem cells) were obtained and induced to differentiate into NK cells. When CD34 positive cells were sorted and used in this way, it was confirmed that the cell yield was significantly reduced, cell death was increased, and the expression rate of the marker protein (CD56) and the activation marker protein (NKp30) of natural killer cells in the sorted CD34 positive cells was reduced (Figures 42a and 42b). In addition, it was confirmed that the cell proliferation rate was also significantly reduced (Figure 42c).

On the other hand, the EiNK production method according to the present invention can induce differentiation into NK cells without performing CD34 sorting because the expression rate of CD34, a hematopoietic stem cell differentiation marker, which is an intermediate stage of NK differentiation, is high at over 95%, thereby increasing cell yield and activity (Figure 42d).

### Experimental Example 12. Comparison of cell survival rates according to third and second cultures of iPSCs

Figures 43 and 44 show the results obtained by confirming cell survival rates when cells are obtained by culturing induced pluripotent stem cells using the third culture method.

Usually, induced pluripotent stem cells were cultured using the third culture method and differentiation was induced using the method described in Experimental Example 11.

As shown in Figures 43 and 44, it was confirmed that when iPSCs were subjected to the third culture using Aggrewell, the size of the embryoid body was larger compared to the second culture. In addition, it was confirmed that there was a difference in the size of the embryoid body by batch, and that the cell survival rate was reduced when obtaining a single cell by treatment with a degrading enzyme. On the other hand, it is considered that the method for differentiating EiNK cells according to the present invention is simpler and more efficient than the third culture since iPSCs are subjected to the second culture and are induced to differentiate.

### Experimental Example 13. Comparison of differentiation induction period of natural killer cells derived from induced pluripotent stem cells

Figures 45 and 46 show the results of comparing the production period of natural killer cells by a conventional EiNK differentiation method (general method) and a differentiation method according to the present invention (method of the present invention) and confirming the phenotypes of EiNK produced according to the present invention. The conventional EiNK differentiation was performed in the same manner as described in Experimental Example 11.

The conventional EiNK differentiation method required a total differentiation induction period of 48 days, while the EiNK differentiation method according to the present invention required a differentiation induction period of 26 days. As a result of confirming the natural killer cell marker proteins (CD56, CD45, NKp44) and activation proteins (CD56, NKp30) in the EiNK cells induced to differentiate for 26 days, the expression rate of NK marker proteins and activation proteins was confirmed to be 66% or higher. On the other hand, the expression rate of iPSC marker proteins (TRA-1-60, SSEA4) was found to be 0%, indicating that the EiNK cell differentiation method according to the present invention is an efficient method capable of producing natural killer cells in a short period of time compared to conventional methods.

## Claims

1. A natural killer cell expressing any one protein selected from the group consisting of CD25, CD9, CD80, and a combination thereof.

2. The natural killer cell according to claim 1, wherein the natural killer cell additionally expresses any one protein selected from the group consisting of CD1d, CD30, CD87, CDw327, CD33, CD49a, CD49c, CD49e, CD49f, CD66a, CD66c, CD66d, CD66e, CD71, CD83, CDw93, CD106, CD108, CD114, CD123, CD124, CD146, CD150, CD164, CD196, CD210, and a combination thereof.

3. The natural killer cell according to claim 1, wherein the natural killer cell expresses any one natural killer cell marker protein selected from the group consisting of CD56, CD45, CD16, NKp30, NKp44, NKG2D, DNAM1, and a combination thereof.

4. The natural killer cell according to claim 1, wherein the natural killer cell is **characterized by** an increased expression of the protein of claim 1 or 2 compared to a natural killer cell isolated from blood.

5. The natural killer cell according to claim 4, wherein the expression of the protein is increased by at least two times compared to a natural killer cell isolated from blood.

6. The natural killer cell according to claim 1, wherein the natural killer cell is **characterized by** an additional increase in the expression of any one gene selected from the group consisting of GZMB, CD226, RASGRP1, CD107a, CCR9, CD40LG, CD300c, and a combination thereof compared to a natural killer cell isolated from blood.

7. The natural killer cell according to claim 1, wherein the natural killer cell is **characterized by** an increase in the secretion of any one protein selected from the group consisting of FLT3L, MIP 1 alpha/beta, PF4, IGFBP2, IGFBP3, MMP9, and a combination thereof compared to a natural killer cell isolated from blood

8. The natural killer cell according to claim 7, wherein the secretion of the protein is increased by at least two times compared to a natural killer cell isolated from blood.

9. The natural killer cell according to claim 1, wherein the natural killer cell does not additionally express or expresses at a low rate any one protein selected from the group consisting of CD205, CD158b, CD243, γδTCR, NKB1, CD158a, CDw328, TIM3, TIGIT, and a combination thereof compared to a natural killer cell isolated from blood.

10. The natural killer cell according to claim 9, wherein the expression of the protein is reduced by at least two times compared to a natural killer cell isolated from blood.

11. The natural killer cell according to claim 1, wherein the natural killer cell does not express CD3, a marker protein of T cells.

12. The natural killer cell according to claim 1, wherein the natural killer cell is differentiated from an induced pluripotent stem cell (iPSC).

13. The natural killer cell according to claim 12, wherein the natural killer cell is produced by a method for producing natural killer cells, comprising:
inoculating induced pluripotent stem cells (iPSCs) as iPSCs or iPSC spheroids into a cell culture vessel so that 0.5 to 20 colonies are formed per 1 cm² of cell culture vessel area and performing adhesion culture to produce iPSC colonies;
differentiating the iPSC colonies into mesodermal cells to produce mesodermal cells;
differentiating the mesodermal cells into hematopoietic stem cells to produce hematopoietic stem cells; and
differentiating the hematopoietic stem cells into natural killer cells to produce natural killer cells.

14. The natural killer cell according to claim 13, wherein the inoculated iPSCs are inoculated at 1 to 8 iPSCs per 1 cm² of cell culture vessel area.

15. The natural killer cell according to claim 13, wherein the inoculated spheroids are inoculated at 1 to 8 spheroids per 1 cm² of cell culture vessel area.

16. The natural killer cell according to claim 13, wherein the total number of stem cells on the start date of differentiation is 1×10³ to 1×10⁶ cells per 1 cm² of cell culture vessel area.

17. The natural killer cell according to claim 13, wherein the cells adhered to the culture vessel are not removed in the differentiating the mesodermal cells into hematopoietic stem cells.

18. The natural killer cell according to claim 13, wherein the cell differentiated from the mesodermal cell into a hematopoietic stem cell has a CD34 protein expression rate of 85% or more without a CD34 positive cell sorting process.

19. The natural killer cell according to claim 13, wherein the natural killer cell is produced by additionally performing a mass culture proliferation on the natural killer cell induced to differentiate.

20. The natural killer cell according to claim 19, wherein the mass culture proliferation is culturing the natural killer cell induced to differentiate, in a medium comprising SCF (stem cell factor), FLT3L (FMS-like tyrosine kinase 3 ligand), IL-7, IL-15, IL-2, or a combination thereof.

21. The natural killer cell according to claim 19, wherein the mass culture proliferation is culturing the natural killer cell induced to differentiate, in a medium comprising β-mercaptoethanol, sodium selenite, ethanolamine, L-ascorbic acid, or a combination thereof.

22. The natural killer cell according to claim 19, wherein the mass culture proliferation of the natural killer cell induced to differentiate is performed for 1 day to 9 weeks.

23. A cell therapeutic agent comprising the natural killer cell according to any one of claims 1 to 22 as an active ingredient.

24. A pharmaceutical composition for preventing and treating a disease, comprising the natural killer cell according to any one of claims 1 to 22 as an active ingredient.

25. The pharmaceutical composition for preventing and treating a disease according to claim 24, wherein the disease is any one selected from the group consisting of an infectious disease, cancer, a neurological disease, and an autoimmune disease

26. The pharmaceutical composition for preventing and treating a disease according to claim 25, wherein the infectious disease is any one selected from the group consisting of hepatitis B, hepatitis C, human papilloma virus (HPV) infection, cytomegalovirus infection, viral respiratory disease, and influenza.

27. The pharmaceutical composition for preventing and treating a disease according to claim 25, wherein the cancer is any one selected from the group consisting of liver cancer, lung cancer, colorectal cancer, breast cancer, prostate cancer, ovarian cancer, pancreatic cancer, colorectal cancer, cervical cancer, thyroid cancer, laryngeal cancer, leukemia, brain tumor, neuroblastoma, retinoblastoma, head and neck cancer, salivary gland cancer, and lymphoma.

28. The pharmaceutical composition for preventing and treating a disease according to claim 25, wherein the neurological disease is a neurological disease caused by nerve damage or abnormal nerve, or a degenerative brain disease.

29. The pharmaceutical composition for preventing and treating a disease according to claim 28, wherein the nerve damage is any one selected from the group consisting of neuropraxia, axonotmesis, and neurotmesis.

30. The pharmaceutical composition for preventing and treating a disease according to claim 28, wherein the neurological disease caused by nerve damage or abnormal nerve is a neurological disease caused by nerve damage or abnormal nerve in the central nervous system, or a neurological disease caused by nerve damage or abnormal nerve in the peripheral nervous system.

31. The pharmaceutical composition for preventing and treating a disease according to claim 30, wherein the neurological disease caused by nerve damage or abnormal nerve in the central nervous system is any one selected from the group consisting of an organic disease and dysfunction in the central nervous system, epilepsy, multiple sclerosis, amyotrophic lateral sclerosis, Alzheimer's disease, Lewy body dementia, Huntington's disease, Parkinson's disease, schizophrenia, traumatic brain injury, stroke, Pick's disease, Creutzfeldt-Jakob disease, progressive supranuclear palsy, multiple system atrophy, corticobasal degeneration, spinocerebellar degeneration, cerebellar atrophy, posttraumatic stress disorder, amnesia, vascular dementia, and cerebral infarction.

32. The pharmaceutical composition for preventing and treating a disease according to claim 30, wherein the neurological disease caused by nerve damage or abnormal nerve in the peripheral nervous system is any one selected from the group consisting of peripheral neuropathy, diabetic neuropathy, peripheral neuropathic pain, peripheral neuropathy due to anticancer chemotherapy, complex regional pain syndrome, optic neuropathy, mononeuropathy, multiple mononeuropathy (mononeuritis multiplex), polyneuropathy, Guillain-Barré syndrome (acute inflammatory demyelinating polyneuropathy), chronic inflammatory demyelinating polyneuropathy, hereditary neuropathy, plexus disorder, glaucoma, macular degeneration, amyotrophic lateral sclerosis, progressive muscular atrophy, progressive bulbar palsy, poliomyelitis, post-polio syndrome, stiff person syndrome, Isaacs' syndrome, myasthenia gravis, neonatal myasthenia, botulism, Eaton-Lambert syndrome, thoracic outlet syndrome, Charcot-Marie-Tooth disease, and spinal muscular atrophy.

33. The pharmaceutical composition for preventing and treating a disease according to claim 25, wherein the neurological disease is a degenerative brain disease.

34. The pharmaceutical composition for preventing and treating a disease according to claim 33, wherein the degenerative brain disease is any one selected from the group consisting of Alzheimer's disease, Parkinson's disease, Huntington's disease, mild cognitive impairment, cerebral amyloid angiopathy, Down syndrome, amyloid stroke, systemic amyloid disease, Dutch type amyloidosis, Niemann-Pick disease, senile dementia, amyotrophic lateral sclerosis, spinocerebellar atrophy, Tourette's syndrome, Friedrich's ataxia, Machado-Joseph's disease, Lewy body dementia, dystonia, progressive supranuclear palsy, and frontotemporal dementia.

35. The pharmaceutical composition for preventing and treating a disease according to claim 25, wherein the autoimmune disease is any one selected from the group consisting of Crohn's disease, erythema, atopy, rheumatoid arthritis, Hashimoto's thyroiditis, pernicious anemia, Addison's disease, type 1 diabetes mellitus, lupus, chronic fatigue syndrome, fibromyalgia, hypothyroidism and hyperthyroidism, scleroderma, Behcet's disease, inflammatory bowel disease, multiple sclerosis, myasthenia gravis, Meniere's syndrome, Guillain-Barré syndrome, Sjogren's syndrome, vitiligo, endometriosis, psoriasis, vitiligo, systemic scleroderma, asthma, and ulcerative colitis.

36. A method for producing natural killer cells, comprising:
inoculating induced pluripotent stem cells (iPSCs) as iPSCs or iPSC spheroids into a cell culture vessel so that 0.5 to 20 colonies are formed per 1 cm² of cell culture vessel area and performing adhesion culture to produce iPSC colonies;
differentiating the iPSC colonies into mesodermal cells to produce mesodermal cells;
differentiating the mesodermal cells into hematopoietic stem cells to produce hematopoietic stem cells; and
differentiating the hematopoietic stem cells into natural killer cells to produce natural killer cells.

37. The method for producing natural killer cells according to claim 36, wherein the production method further comprises a mass culture proliferation on the natural killer cells induced to differentiate.

38. The method for producing natural killer cells according to claim 36, wherein the total number of stem cells on the start date of differentiation is 1×10³ to 1×10⁶ cells per 1 cm² of cell culture vessel area.

39. The method for producing natural killer cells according to claim 36, wherein the cells adhered to the culture vessel are not removed in the differentiating the mesodermal cells into hematopoietic stem cells.

40. A use of the natural killer cell according to claim 1 for the prevention or treatment of a disease.

41. The use for the prevention or treatment according to claim 39, wherein the disease is any one selected from the group consisting of an infectious disease, cancer, a neurological disease, and an autoimmune disease.

42. A method for preventing or treating a disease, comprising: administering the natural killer cell according to claim 1.

43. The method for preventing or treating a disease according to claim 42, wherein the disease is any one selected from the group consisting of an infectious disease, cancer, a neurological disease, and an autoimmune disease.

44. A method for culturing stem cells, comprising: inoculating stem cells as iPSCs or iPSC spheroids into a cell culture vessel so that 0.5 to 20 colonies are formed per 1 cm² of cell culture vessel area.

45. A method for producing natural killer cells, comprising: differentiating hematopoietic stem cells into natural killer cells in the presence of feeder cells.
